# EUROPEAN PATENT SPECIFICATION

(11) **EP 3 003 497 B1**
(45) Date of publication and mention of the grant of the patent: **14.02.2024**
(21) Application number: 14804274.0
(22) Date of filing: 29.05.2014
(51) Int. Cl.: A61Q 7/00, A61P 33/00

(54) **PREVENTING OR MITIGATING CHEMOTHERAPY INDUCED ALOPECIA USING VITAMIN D**
VERHINDERUNG ODER VERRINGERUNG VON CHEMOTHERAPIEINDUZIERTER ALOPEZIE MIT VITAMIN D
PRÉVENTION OU ATTÉNUATION DE L'ALOPÉCIE INDUITE PAR LA CHIMIOTHÉRAPIE AU MOYEN DE VITAMINE D

(30) Priority: 29.05.2013 US 201361828448 P
(43) Date of publication of application: 13.04.2016
(73) Proprietor: BPGbio, Inc., Framingham, MA, 01701 (US)
(72) Inventor: NARAIN, Niven, Rajin, Cambridge, MA 02138 (US); MCCOOK, John, Patrick, Frisco, TX 75034 (US); JIMENEZ, Joaquin, J., Miami, FL 33144 (US)
(74) Representative: Hoffmann Eitle
(86) International application number: PCT/US2014/040084
(87) International publication number: WO 2014/194133

(56) References cited:
- WO-A2-2011/019617
- US-A1- 2002 035 097
- US-A1- 2009 130 216
- US-B2- 6 844 326
- Anonymous: "NCT01588522 on 2012_04_30: ClinicalTrials.gov Archive", , 30 April 2012 (2012-04-30), XP055319564, Retrieved from the Internet: URL:https://clinicaltrials.gov/archive/NCT 01588522/2012_04_30 [retrieved on 2016-11-15] & Thomson Reuters Drug News (formerly DailyDrugNews.com) July 24, 2012 , 24 July 2012 (2012-07-24), Retrieved from the Internet: URL:https://integrity.thomson-pharma.com/i ntegrity/xmlxsl/pk_ref_list.xml_show_ficha _ref?p_ref_id=1869639 [retrieved on 2016-11-15]
- NARAIN N R ET AL: "Protection from chemotherapy-induced alopecia by cytotech lbs API 31543 in a multichemotherapy course model of chloroleukemia", AMERICAN ASSOCIATION FOR CANCER RESEARCH. PROCEEDINGS OF THE ANNUAL MEETING; [CANCER RESEARCH; APRIL 2004, VOLUME 64, ISSUE 7, SUPPLEMENT], AMERICAN ASSOCIATION FOR CANCER RESEARCH, US, vol. 101, 1 January 2010 (2010-01-01), XP009192460, ISSN: 0197-016X
- LI ET AL.: 'Hair shaft elongation, follicle growth, and spontaneous regression in long-term, gelatin sponge-supported histoculture of human scalp skin.' PROCEEDINGS OF THE NATIONAL ACADEMY OF SCIENCES vol. 89, 01 September 1992, pages 8764 - 8768, XP002105341 DOI: 10.1073/PNAS.89.18.8764
- Anonymous: "Vitamin D Spray for Your Vitamin D Deficiency - Mercola.com", , 3 February 2013 (2013-02-03), XP55433198, Retrieved from the Internet: URL:https://web.archive.org/web/2013020310 3517/http://products.mercola.com/vitamin-d -spray/ [retrieved on 2017-12-08]

## Description

### Related Applications

This application claims priority to U.S. Provisional Application Ser. No. 61/828,448, filed May 29, 2013, entitled "Preventing or Mitigating Chemotherapy Induced Alopecia Using Vitamin D".

### Background of the Invention

Alopecia is a common and distressing side effect of many chemotherapeutic agents and for which there is currently few effective preventive measures. In a recent study, thirty-five of forty-six patients receiving chemotherapy ranked alopecia as a more disturbing side effect than vomiting (Tierney et al, B. J. Cancer, 62:527-528, 1990).

Currently, those suffering from alopecia can only attempt to regrow lost hair by repeated applications of topical steroids or can attempt to maintain hair growth by topical application of minoxidil. Moreover, there are currently no approved therapeutic agents with the ability to prevent or mitigate alopecia from occurring as a side effect during chemotherapy treatment, although there have been some promising studies. For example, using a young rat model, it has been demonstrated that ImuVert, a biologic response modifier prepared from the bacterium *Serratia marcescens*, protected the animals from alopecia induced by cytosine arabinoside or adriamycin (Hussein et al., Science 249: 1564-1566, 1990). In subsequent studies, similar protection from ARA-C-induced alopecia was observed from recombinant interleukin-1 (IL-1) beta (Jimenez et al., FASEB J. 1991). Despite these promising results, there remains a need for a safe and effective therapeutic agent that treats alopecia in those suffering from this disorder, and further, prevents chemotherapy-induced alopecia in those receiving cancer treatment. WO 2011/019617 A2 discloses the use of vitamin D3 and analogs thereof for treating alopecia. WO 2006/005845 A1 discloses a spray composition comprising a combination of calcitriol and clobetasol propionate. NCT01588522 is a safety study of topical Calcitriol in patients receiving chemotherapy for breast cancer.

### Summary of the Invention

The present invention relates to the topical use of vitamin D compounds, such as Vitamin D3 or calcitriol and its analogs or a metabolite thereof, dosages and formulations thereof, to prevent or mitigate chemotherapy-induced alopecia (CIA). In particular, the invention provides pharmaceutical compositions for use in a method of preventing or mitigating chemotherapy-induced alopecia (CIA). The pharmaceutical compositions of the invention comprise an effective amount of a vitamin D compound in a topical formulation. The invention has broad applications in chemotherapies that induce alopecia, for example, taxane-based chemotherapy, for the treatment of solid tumor, such as cervical cancer, endometrial cancer, ovarian cancer, fallopian tube cancer, primary peritoneal carcinoma, soft tissue sarcoma, or bone sarcoma. The pharmaceutical compositions for use of the invention can be advantageously administered before and/or concurrent with the chemotherapy.

The present invention includes the following embodiments.
[1] A pharmaceutical composition comprising a therapeutically effective amount of a vitamin D compound for use in a method of preventing or mitigating chemotherapy-induced alopecia in a human subject, the method comprising the steps of:
   (1) selecting a human subject having a cancer and who is scheduled to receive, or is receiving, a chemotherapy; and
   (2) topically administering the pharmaceutical composition to the scalp of the subject using a metered spray unit,
      wherein step (2) is performed prior to and/or concurrently with the chemotherapy,
         wherein the pharmaceutical composition comprises the vitamin D compound at a concentration of 5-20 µg/mL;
      wherein the pharmaceutical composition is administered in a 1.0 mL dose; and
      wherein the vitamin D compound is represented by Formula (1): 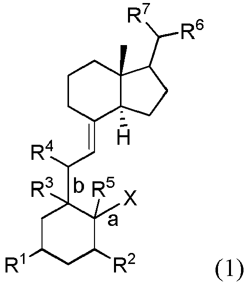
      wherein
      a and b are each independently a single or double bond;
      X is -CH₂ when a is a double bond, or X is hydrogen or a hydroxyl substituted alkyl when a is a single bond;
      R¹ is hydrogen, hydroxyl, alkoxyl, tri-alkyl silyl or alkyl, optionally substituted with one to three halogen, hydroxyl, cyano or -NR'R" moieties;
      R² is hydrogen, hydroxyl, -O-trialkyl silyl, or alkyl, alkoxyl or alkenyl, optionally substituted with one to three halogen, hydroxyl, cyano or -NR'R" moieties;
      R³ is absent when b is a double bond or R³ is hydrogen, hydroxyl or alkyl, or R³ and R¹ together with the carbon atoms to which they are attached may be linked to form 5-7 membered carbocyclic ring when b is a single bond;
      R⁴ is absent when b is a double bond or hydrogen, halogen or hydroxyl when b is a single bond;
      R⁵ is absent when a is a double bond or R⁵ is hydrogen, halogen or hydroxyl when a is a single bond;
      R⁶ is alkyl, alkenyl, alkynyl, cycloalkyl, heterocyclicyl, alkyl-O-alkyl, alkyl-CO2-alkyl optionally substituted with one to five, hydroxyl, oxo, halogen, alkoxyl, aryl, heteroaryl, cyano, nitro or -NR'R" moieties;
      R7 is alkyl optionally substituted with one to three hydroxyl, halogen, alkoxyl, aryl, heteroaryl, cyano, nitro or -NR'R" moieties; and,
      R' and R" are each, independently, hydrogen, hydroxyl, halogen, alkyl or alkoxyl, and pharmaceutically acceptable salts thereof,
      or wherein the vitamin D compound is 1α-hydroxyvitamin D3; 1α,24-dihydroxyvitamin D3, calcitriol, calcipotriol or 1,25-dihydroxy-16-ene-23-yne-cholecalciferol.
[2] The pharmaceutical composition for use according to embodiment [1], wherein step (2) is performed prior to the commencement of the chemotherapy, wherein step (2) is preferably performed at least 3, 4, 5, 6, 7, 8, 9, 10, 11, 12 or 13 days prior to the commencement of the chemotherapy, and most preferably wherein step (2) is performed at least two weeks prior to the commencement of the chemotherapy.
[3] The pharmaceutical composition for use according to any one of embodiments [1]-[2], wherein the pharmaceutical composition is
   (i) administered in a total daily dose of 10-40 µg of the vitamin D compound, and wherein the pharmaceutical composition is preferably administered in a total daily dose of 20 µg or 40 µg of the vitamin D compound; or
   (ii) administered twice daily for a total daily dose of 10-40 µg of the vitamin D compound, wherein each of 2 individual doses per day is 5-20 µg.
[4] The pharmaceutical composition for use according to any one of embodiments [1]-[3],
   wherein 0.25 mL is administered to each of the four quadrants of the scalp.
[5] The pharmaceutical composition for use according to any one of embodiments [1]-[4], wherein step (2) is performed twice daily, wherein the two daily administrations are preferably separated by 10-14 hours.
[6] The pharmaceutical composition for use according to any one of embodiments [1]-[5], wherein the subject has a solid tumor, wherein the subject optionally has an advanced or a recurrent cancer, and/or wherein the subject optionally has cervical cancer, endometrial cancer, ovarian cancer, fallopian tube cancer, primary peritoneal carcinoma, soft tissue sarcoma, bone sarcoma, or breast cancer.
[7] The pharmaceutical composition for use according to any one of embodiments [1]-[6], wherein the subject is selected based on one or more of the following additional criteria:
   the subject is a human of at least 18 years of age;
   the subject has no evidence of alopecia or mild alopecia;
   the subject has hair follicles that are not apoptotic;
   the subject has an Eastern Cooperative Oncology Group (ECOG) performance score of 0 or 1 within 14 days prior to beginning topical administration;
   the subject has a baseline neutrophil count greater than 1500 cells/mm3 within 72 hours prior to beginning topical administration; and
   the subject has a serum calcium level less than or equal to the upper limit of normal (ULN) within 72 hours prior to beginning topical administration,
   or wherein the subject is selected based on one or more of the following additional criteria:
      the subject does not have a history of hypercalcemia or vitamin D toxicity within 30 days of beginning the topical administration;
      the subject does not have a history of hospitalization for treatment for angina, myocardial infarction, or congestive heart failure or psychiatric illness within 30 days of beginning topical administration;
      the subject does not take a vitamin D supplement during topical administration, unless the subject has been taking the vitamin D supplement for 30 days or more prior to beginning topical administration and maintains the same dose throughout topical administration;
      the subject is not receiving a thiazide or furosemide diuretic, with the exception of subjects who have stable doses and have been on therapy for over 6 months;
      the subject does not have hypercalcemia or kidney stones; and
      the subject does not have alopecia grade 2 or greater as per National Cancer Institute Common Terminology Criteria for Adverse Events (NCU-CTCAE) v4.0 or significant hair loss or hair breakage.
[8] The pharmaceutical composition for use according to any one of embodiments [1]-[7], wherein step (2) is performed for at least three months after commencement of the chemotherapy, or wherein step (2) is performed for the duration of the chemotherapy.
[9] The pharmaceutical composition for use according to any one of embodiments [1]-[8], wherein the pharmaceutical composition is formulated such that the vitamin D compound is delivered to epidermis while substantially avoiding dermal delivery.
[10] The pharmaceutical composition for use according to any one of embodiments [1]-[9], wherein the pharmaceutical composition is anhydrous, wherein the pharmaceutical composition optionally comprises a vehicle of 40% (w/w) propylene glycol and 60% (w/w) anhydrous ethanol, or wherein the pharmaceutical composition optionally comprises a vehicle of 30% (w/w) propylene glycol, 10% (w/w) ethoxydiglycol or transcutol, and 60% (w/w) anhydrous absolute ethanol (200 proof, U.S.).
[11] The pharmaceutical composition for use according to embodiments [1]-[10], wherein the vitamin D compound is 1α-hydroxyvitamin D3; 1α,24-dihydroxyvitamin D3, calcipotriol, or calcitriol.
[12] The pharmaceutical composition for use according to embodiments [1]-[11], wherein the vitamin D compound is calcitriol.
[13] A kit for use in a method for preventing or mitigating chemotherapy induced alopecia, the kit comprising a pharmaceutical composition as specified in any one of embodiments [1]-[12] and adapted for topical administration; and instructions for carrying out a method for preventing or mitigating chemotherapy induced alopecia.

It should be noted that all embodiments described herein (above and below) are contemplated to be able to combine with any other embodiment(s) where applicable, including embodiments described only under one of the aspects of the invention, and embodiments described under different aspects of the invention.

### Brief Description of the Drawings

**Figure 1** shows total absorption and mass balance results across the three skin donors, and the distribution of calcitriol from intact human cadaver skin over 48 hours from a single application. Results are shown in log scale as mean ± SE as total mass (ng/cm²).
**Figure 2** shows an exemplary growth curve of HEKa cells over different concentrations of calcitriol present in the growth media. Note the log scale of the calcitriol concentration.
**Figure 3** shows an exemplary growth curve by the pancreatic carcinoma cell line PaCa2, which growth curve is not responsive to the presence of 0.1 µg/mL of calcitriol.
**Figures 4A** and **4B** show the growth of Hep-G2 cells and MCF-7 cells, respectively, in the presence of increasing concentrations of calcitriol.
**Figure 5** shows dosing curves of erlotinib (Tarceva), an EGFR Tyr kinase inhibitor, in the absence (◆) or presence of 0.1 µg/mL calcitriol (×).
**Figure 6** shows dosing curves of gefitinib (Iressa), another EGFR Tyr kinase inhibitor, in the absence (◆) or presence of 0.1 µg/mL calcitriol (×).
**Figure 7** shows dosing curves of sorafinib in the absence (◆) or presence of 0.1 µg/mL calcitriol (×). Sorafenib is known to inhibit several kinases (Raf, VEGF-R2, c-kit, PDGR-R).
**Figure 8** shows dosing curves of dasatinib in the absence (◆) or presence of 0.1 µg/mL calcitriol (×). Dasatinib inhibits BCR/ABL Tyr kinases.
**Figure 9** shows dosing curves of staurosporin in the absence (◆) or presence of 0.1 µg/mL calcitriol (×). Staurosporin is a relatively nonspecific kinase inhibitor.
**Figure 10** shows dosing curves of cisplatin in the absence (◆) or presence of 0.1 µg/mL calcitriol (×). Cisplatin is a DNA alkylating agent.
**Figure 11** shows dosing curves of carboplatin in the absence (◆) or presence of 0.1 µg/mL calcitriol (×). Carboplatin is also a DNA alkylating agent.
**Figure 12** shows dosing curves of irinotecan in the absence (◆) or presence of 0.1 µg/mL calcitriol (×).
**Figure 13** shows dosing curves of paclitaxol in the absence (◆) or presence of 0.1 µg/mL calcitriol (×).

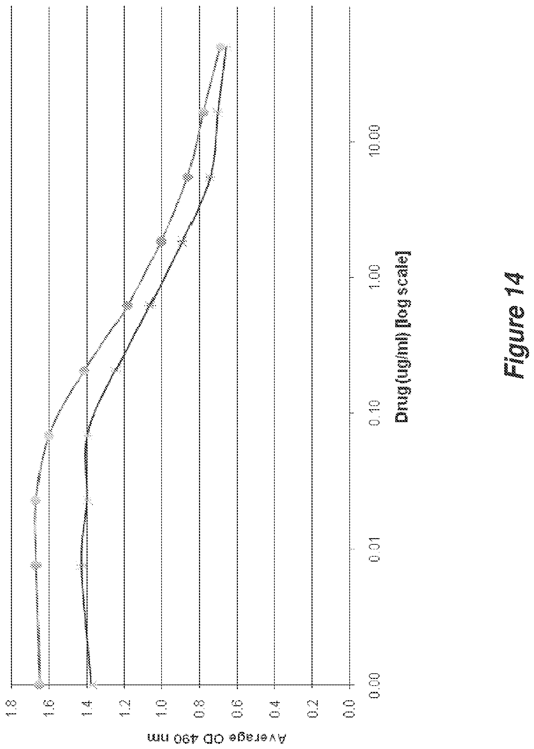

**Figure 14** shows dosing curves of 5-FU in the absence (◆) or presence of 0.1 µg/mL calcitriol (×).
**Figure 15** shows dosing curves of gemcitabine in the absence (◆) or presence of 0.1 µg/mL calcitriol (×).
**Figure 16** shows dosing curves of doxorubicin in the absence (◆) or presence of 0.1 µg/mL calcitriol (×).
**Figure 17** shows dosing curves of tamoxifen in the absence (◆) or presence of 0.1 µg/mL calcitriol (×).
**Figure 18** shows that 0.1 µg/mL calcitriol protects normal keratinocytes HEKa against 5-FU, while does not appreciably affect ED₅₀ values of 5-FU against cancer cells.
**Figure 19** shows that calcitriol does not appreciably alter the cytotoxic effect of Doxorubicin against the cancer cell line SkBr-3.
**Figure 20A** shows that, in Sprague Dawley rats receiving etoposide, a topical formulation of calcitriol protects from chemotherapy-induced alopecia (CIA) in a dose-dependent manner. Left panel: rats receiving etoposide only; middle panel: rats receiving etoposide and topical application of 0.1 µg of calcitriol in a topical formulation; right panel: rats receiving etoposide and topical application of 0.3 mg of calcitriol in a topical formulation. **Figure 20B** shows similar results in the color-coated Long Evans rats.
**Figure 21** shows that a calcitriol topical formulation (0.2 µg total dose) protects Long Evans rats from cyclophosphamide (CTX)-induced alopecia.
**Figure 22A** shows that a calcitriol topical formulation (0.2 µg total dose) protects Long Evans rats from CTX-doxorubicin combination chemotherapy-induced alopecia. **Figure 22B** shows similar protective result by calcitriol topical formulation calcitriol in rats treated by cytarabine-doxorubicin combination chemotherapy-induced alopecia. The protective effect of a calcitriol topical formulation in rats treated by cytarabine alone is shown in **Figure 22C****.**
**Figure 23** shows that a topical calcitriol topical formulation (0.2 µg total dose) protects Long Evans rats injected with MIAC51 (chloroleukemia cells) from CTX-induced alopecia.
**Figure 24** shows that, in *in vivo* experiments conducted on Long Evans rats injected with MIAC51 (chloroleukemia cells), a calcitriol topical formulation does not protect the cancer cells from chemotherapy.
**Figures 25A** and **25B** show the estimated level of recovered calcitriol (ng/mg) from the stratum corneum of the minipig epidermis and the rest of the epidermis. The amount is expressed as mean ± SD of calcitriol recovered. nd = none detected, na = not available.
**Figure 26** shows the near linear correlation between calcitriol doses applied to recovered calcitriol tissue level in epidermis, with a range of calcitriol concentrations from 3 to 100 µg/mL applications.
**Figure 27** illustrates the effect of calcitriol on the first anagen course of chloroleukemic rats receiving cyclophosphamide. **Figure 27A** depicts rats receiving cyclophosphamide alone, **Figure 27B** depicts rats receiving cyclophosphamide and vehicle, while **Figure 27C** depicts rats receiving cyclophosphamide and calcitriol.
**Figure 28** illustrates the effect of calcitriol on the second anagen course of chloroleukemic rats receiving cyclophosphamide. Left to right, rats treated with cyclophosphamide alone, rats treated with cyclophosphamide and vehicle and rats treated with cyclophosphamide and calcitriol.
**Figure 29** illustrates the effect of calcitriolon the first anagen course of chloroleukemic rats receiving cyclophosphamide in combination with doxorubicin. **Figure 29A** depicts rats receiving cyclophosphamide and doxorubicin alone, **Figure 29B** depicts rats receiving cyclophosphamide, doxorubicin and vehicle, while **Figure 29C** depicts rats receiving cyclophosphamide, doxorubicin and calcitriol.
**Figure 30** illustrates the effect of calcitriol on the second anagen course of chloroleukemic rats receiving cyclophosphamide in combination with doxorubicin. Left to right, rats treated with cyclophosphamide and doxorubicin alone, rats treated with cyclophosphamide, doxorubicin and vehicle and rats treated with cyclophosphamide, doxorubicin and calcitriol.
**Figure 31** illustrates the effect of calcitriolon the first anagen course of chloroleukemic rats receiving cyclophosphamide in combination with doxorubicin and cytarabine. **Figure 31A** depicts rats receiving cyclophosphamide, doxorubicin and cytarabine alone, **Figure 31B** depicts rats receiving cyclophosphoramide, doxorubicin, cytarabine and vehicle, while **Figure 31C** depicts rats receiving cyclophosphamide, doxorubicin, cytarabine and calcitriol.
**Figure 32** illustrates the effect of calcitriol on the second anagen course of chloroleukemic rats receiving cyclophosphamide in combination with doxorubicin and cytarabine. Left to right, rats treated with cyclophosphamide, doxorubicin and cytarabine alone, rats treated with cyclophosphamide, doxorubicin, cytarabine and vehicle and rats treated with cyclophosphamide, doxorubicin, cytarabine and calcitriol.
**Figure 33** illustrates the effect of calcitriolon the first anagen course of chloroleukemic rats receiving cyclophosphamide in combination with paclitaxol and etoposide. **Figure 33A** depicts rats receiving cyclophosphamide, paclitaxel and etoposide alone, **Figure 33B** depicts rats receiving cyclophosphoramide, paclitaxel, etoposide and vehicle, while **Figure 33C** depicts rats receiving cyclophosphamide, paclitaxel, etoposide and calcitriol.
**Figure 34** illustrates the effect of calcitriol on the second anagen course of chloroleukemic rats receiving cyclophosphamide in combination with paclitaxel and etoposide. Left to right, rats treated with cyclophosphamide, paclitaxel and etoposide alone, rats treated with cyclophosphamide, paclitaxel, etoposide and vehicle and rats treated with cyclophosphamide, paclitaxel, etoposide and calcitriol.
**Figure 35** illustrates the effect of calcitriolon the first anagen course of chloroleukemic rats receiving doxorubicin in combination with paclitaxel and etoposide. **Figure 35A** depicts rats receiving doxorubicin, paclitaxel and etoposide alone, **Figure 35B** depicts rats receiving doxorubicin, paclitaxel, etoposide and vehicle, while **Figure 35C** depicts rats receiving doxorubicin, paclitaxel, etoposide and calcitriol.
**Figure 36** illustrates the effect of calcitriol on the second anagen course of chloroleukemic rats receiving doxorubicin in combination with paclitaxol and etoposide. Left to right, rats treated with doxorubicin, paclitaxol and etoposide alone, rats treated with doxorubicin, paclitaxol, etoposide and vehicle and rats treated with doxorubicin, paclitaxol, etoposide and calcitriol.

### Detailed Description of the Invention

The invention described herein is partly based on the discovery of topical formulations of vitamin D compounds that can prevent or mitigate chemotherapy-induced alopecia. In some embodiments, the formulations can be selectively delivered to or accumulated in the epidermis layer of the skin while substantially avoiding delivery to and/or accumulation in the deeper dermis layer. This may be advantageous in certain patients undergoing chemotherapy treatment, where deeper accumulation of a vitamin D compound may result in a decrease in the efficacy of the chemotherapy regimen. Such topical formulations may also be advantageous in patients who have medical conditions that may be negatively impacted by the presence of excessive amount of vitamin D compounds, such as patients suffering from kidney stones, and whose condition may worsen upon calcium mobilization by certain vitamin D compounds. Therefore, in such patients, the ideal delivery of the vitamin D compound should be a local delivery of a minimal effective dose to the epidermis layer of the skin, rather than to the dermis layer that is rich in blood vessels.

The invention is also partly based on the discovery that vitamin D compounds exhibit a mild growth stimulatory effect on normal keratinocytes at a relatively low concentration/dosage, while exhibiting a growth inhibitory effect on the same cells at a relatively high concentration/dosage. Thus, the invention provides pharmaceutical compositions for use that exhibit optimal protective effect against alopecia without causing undesirable growth inhibitory effects. Accordingly, the invention includes topically administering the vitamin D compound prior to and/or concurrently with chemotherapy at a dose providing optimal protective effect against alopecia without causing undesirable growth inhibitory effects.

The invention is further based on the discovery that vitamin D compounds activate or inhibit the expression of multiple target genes in normal keratinocytes, therefore providing a basis to select the most suitable vitamin D compounds for specific therapeutic applications, and to identify additional vitamin D analogs with similar biological activity. Accordingly, the invention includes topically administering the vitamin D compound prior to and/or concurrently with chemotherapy.

While not wishing to be bound by any particular theory, the formulations of the invention may be advantageous in terms of minimizing drug interference with chemotherapy reagents. The dermal layer of the skin is rich in blood vessels, and topical drug penetration to this layer might cause drug interference with systemically delivered chemotherapeutic reagents, leading to unfavorable protective effects to cancer cells.

Accordingly, the pharmaceutical composition for use in a method of preventing or mitigating chemotherapy induced alopecia in a subject includes (1) selecting a human subject having cancer who is scheduled to receive, or is receiving, chemotherapy; and (2) topically administering a pharmaceutical composition comprising therapeutically effective amount of a vitamin D compound to the scalp of the subject using a metered spray unit, prior to and/or concurrently with the chemotherapy in accordance with the appended claims.

The term "alopecia" includes the involuntary complete or partial hair loss from the head or body of an individual and includes alopecia areata (AA), alopecia totalis (AT), alopecia universalis (AU), or chemotherapy-induced alopecia (CIA). Alopecia areata may include diffuse alopecia areata, alopecia areata monolocularis, alopecia areata multilocularis, and alopecia areata barbae. In some embodiments, alopecia does not include androgenetic alopecia (alopecia androgenetica, or male baldness) or post-chemotherapy alopecia (PCA).

Alopecia is the medical description of the loss of hair from the head or body, sometimes to the extent of baldness. Unlike the common aesthetic depilation of body hair, alopecia tends to be involuntary and unwelcome, e.g., androgenic alopecia. However, it may also be caused by a psychological compulsion to pull out one's own hair (trichotillomania) or the unforeseen consequences of voluntary hairstyling routines (mechanical "traction alopecia" from excessively tight ponytails or braids, or burns to the scalp from caustic hair relaxer solutions or hot hair irons). In some cases, alopecia is an indication of an underlying medical concern, such as iron deficiency.

When hair loss occurs in only one section, it is known as "alopecia areata." In human alopecia areata, hair is lost from some or all areas of the body, usually from the scalp. Because it causes bald spots on the scalp, especially in the first stages, it is sometimes called spot baldness. In 1%-2% of cases, the condition can spread to the entire scalp (alopecia totalis) or to the entire epidermis (alopecia universalis).

Conditions resembling AA, and having a similar cause, occur also in other species. The most common type of alopecia areata involves hair loss in one or more round spots on the scalp. Hair may also be lost more diffusely over the whole scalp, in which case the condition is called diffuse alopecia areata. Alopecia areata monolocularis describes baldness in only one spot that may occur anywhere on the head. Alopecia areata multilocularis refers to multiple areas of hair loss. The disease may be limited only to the beard, in which case it is called alopecia areata barbae. If the individual loses all the hair on his/her scalp, the disease is then called alopecia areata totalis.

"Alopecia universalis" is when complete hair loss on the body occurs, similar to how hair loss associated with chemotherapy sometimes affects the entire body.

"Androgenic alopecia" (also known as androgenetic alopecia or alopecia androgenetica) is a common form of hair loss in both female and male humans, chimpanzees, and orangutans. In male humans in particular, this condition is also commonly known as male pattern baldness. Hair is lost in a well-defined pattern, beginning above both temples. Over time, the hairline recedes to form a characteristic "M" shape. Hair also thins at the crown of the head. Often a rim of hair around the sides and rear of the head is left, or the condition may progress to complete baldness. The pattern of hair loss in women differs from male pattern baldness. In women, the hair becomes thinner all over the head, and the hairline does not recede. Androgenic alopecia in women rarely leads to total baldness.

The language "preventing alopecia" includes the arresting of or suppression of hair loss associated with alopecia prior to its occurrence.

The language "mitigating alopecia" or "treating alopecia" includes reducing the severity of the hair loss associated with alopecia or reducing the extent of the hair loss associated with of alopecia. In some embodiments, mitigating or treating alopecia includes the amelioration of alopecia.

The term "administering" includes providing one or more doses of the vitamin D compound to the individual in an amount effective to prevent or treat alopecia. Optimal administration rates for a given protocol of administration of the vitamin D compound can ascertained by those skilled in the art using conventional dosage determination tests conducted with regard to the specific compounds being utilized, the particular compositions formulated, the mode of application, the particular site of administration and the like.

The language "topically administering" includes delivering one or more doses of the vitamin D compound to the skin of the individual in an amount effective to treat or prevent alopecia.

The skin contains many specialized cells and structures, and has various important functions, such as serving as a protective barrier that interfaces with the environment, helping to maintain the proper body temperature, gathering sensory information from the environment, and playing an active role in the immune system.

The skin has three layers - the epidermis, dermis, and subcutaneous tissue. The epidermis is the outer layer of skin. Its thickness varies in different types of skin. It is the thinnest on the eyelids at about 0.05 mm and the thickest on the palms and soles at about 1.5 mm. From bottom to top, the epidermis contains five layers: stratum basale, stratum spinosum, stratum granulosum, stratum licidum (optional in some skins), and stratum corneum.

The stratum basale is the bottom layer of keratinocytes in the epidermis and is responsible for constantly renewing epidermal cells. This layer contains just one row of undifferentiated columnar stem cells that divide very frequently. Half of the cells differentiate and move to the next layer to begin the maturation process. The other half stay in the basal layer and divide repeatedly to replenish the basal layer. Cells that move into the spinosum layer (also called prickle cell layer) change from being columnar to polygonal. In this layer, the cells start to synthesize keratin. The cells in the stratum granulosum, or granular layer, have lost their nuclei and are characterized by dark clumps of cytoplasmic material. There is a lot of activity in this layer as keratin proteins and water-proofing lipids are being produced and organized. The stratum lucidum layer is only present in thick skin where it helps reduce friction and shear forces between the stratum corneum and stratum granulosum. The cells in the stratum corneum layer are known as corneocytes. These cells have flattened out and are composed mainly of keratin protein which provides strength to the layer but also allows the absorption of water. The structure of the stratum corneum layer looks simple, but this layer is responsible for maintaining the integrity and hydration of the skin - a very important function.

The dermis also varies in thickness depending on the location of the skin. It is about 0.3 mm on the eyelid and about 3.0 mm on the back. The dermis is composed of three types of tissue that are present throughout - not in layers: collagen, elastic tissue, and reticular fibers. The two layers of the dermis are the papillary and reticular layers. The upper, papillary layer, contains a thin arrangement of collagen fibers. The lower, reticular layer, is thicker and made of thick collagen fibers that are arranged parallel to the surface of the skin. The dermis contains many specialized cells and structures. For example, blood vessels and nerves course through this layer. The hair follicles are also situated in this layer with the erector pili muscle that attaches to each follicle. A portion of the hair follicle also contains stem cells capable of regrowing damaged epidermis. Stem cells may be present at the dermal-epidermal junction (DEJ). Sebaceous (oil) glands and apocrine (scent) glands are associated with the follicle. This layer also contains eccrine (sweat) glands, but they are not associated with hair follicles.
The subcutaneous tissue is a layer of fat and connective tissue that houses larger blood vessels and nerves. This layer is important in the regulation of temperature of the skin itself and the body. The size of this layer varies throughout the body and from person to person.

Accordingly, as used herein, "epidermis" includes all five of its layers (when present), including the junction layer between epidermis and dermis (e.g., dermal-epidermal junction or DEJ), and stem cells that regenerates the epidermal layers (e.g., follicular stem cells and epidermal stem cells).

As used herein, the phrase "selecting a human subject having cancer and who is scheduled to receive, or is receiving, chemotherapy" include selecting a patient who has cancer and who has been prescribed chemotherapy by a physician or who is receiving chemotherapy under the care of a physician, and can further include selecting a patient meeting one or more criteria as described herein.

In some embodiments, selecting the human subject (in the following also referred to as the "subject") includes selecting a subject having the solid tumor. In some embodiments the solid tumor is selected from the group consisting of carcinoma, melanoma, sarcoma, and lymphoma. In certain embodiments, the solid tumor is selected from the group consisting of breast cancer, bladder cancer, colon cancer, rectal cancer, endometrial cancer, ovarian cancer, fallopian tube cancer, primary peritoneal carcinoma, kidney (renal cell) cancer, lung cancer, pancreatic cancer, prostate cancer, thyroid cancer, skin cancer, bone cancer, brain cancer, cervical cancer, liver cancer, stomach cancer, mouth and oral cancers, neuroblastoma, testicular cancer, uterine cancer, soft tissue sarcoma, bone sarcoma, and vulvar cancer. In certain embodiments, the solid tumor is breast cancer, including triple negative breast cancer. In certain embodiments, the solid tumor is a skin cancer selected from the group consisting of melanoma, squamous cell carcinoma, basal cell carcinoma, and cutaneous T-cell lymphoma (CTCL). In one embodiment, the solid tumor is selected from the group consisting of cervical cancer, endometrial cancer, ovarian cancer, fallopian tube cancer, primary peritoneal carcinoma, soft tissue sarcoma, and bone sarcoma.

In some embodiments, selecting the subject includes selecting a subject having breast cancer. In some embodiments, selecting the subject includes selecting a subject having cervical cancer. In some embodiments, selecting the subject includes selecting a subject having endometrial cancer. In some embodiments, selecting the subject includes selecting a subject having ovarian cancer. In some embodiments, selecting the subject includes selecting a subject having fallopian tube cancer. In some embodiments, selecting the subject includes selecting a subject having primary peritoneal carcinoma. In some embodiments, selecting the subject includes selecting a subject having soft tissue sarcoma. In some embodiments, selecting the subject includes selecting a subject having bone sarcoma.

In some embodiments, selecting the subject includes selecting a subject having advanced or recurrent cancer. In some embodiments, the cancer, e.g., advanced cancer, can be metastatic, locally advanced, or unresectable. In some embodiments, the cancer can be selected by stage (*e.g.*, the subject can be selected for a particular cancer at a particular stage, or ranges of stages).

In some embodiments, the cancer can be staged by roman numeral, for example: Stage 0: carcinoma in situ; Stage I: cancers are localized to one part of the body (Stage I cancer can be surgically removed if small enough.); Stage II: cancers are locally advanced (Stage II cancer can be treated by chemo, radiation, or surgery.); Stage III: cancers are also locally advanced (Whether a cancer is designated as Stage II or Stage III can depend on the specific type of cancer; for example, in Hodgkin's Disease, Stage II indicates affected lymph nodes on only one side of the diaphragm, whereas Stage III indicates affected lymph nodes above and below the diaphragm. The specific criteria for Stages II and III therefore differ according to diagnosis. Stage III can be treated by chemo, radiation, or surgery.); Stage IV: cancers have often metastasized, or spread to other organs or throughout the body (Stage IV cancer can be treated by chemo, radiation, surgery, or clinical trials.)

In some embodiments, the cancer can be staged by TNM (Tumor, Node, Metastasis), as accepted by the Union for International Cancer Control (UICC) and the American Joint Committee on Cancer (AJCC). The TNM system is based on the size and/or extent (reach) of the primary tumor (T), the amount of spread to nearby lymph nodes (N), and the presence of metastasis (M) or secondary tumors formed by the spread of cancer cells to other parts of the body. A number is added to each letter to indicate the size and/or extent of the primary tumor and the degree of cancer spread. Primary Tumor (T) - TX: Primary tumor cannot be evaluated; T0: No evidence of primary tumor; Tis: Carcinoma in situ (CIS; abnormal cells are present but have not spread to neighboring tissue; although not cancer, CIS may become cancer and is sometimes called preinvasive cancer); T1, T2, T3, T4: Size and/or extent of the primary tumor. Regional Lymph Nodes (N) - NX: Regional lymph nodes cannot be evaluated; N0: No regional lymph node involvement; N1, N2, N3: Degree of regional lymph node involvement (number and location of lymph nodes). Distant Metastasis (M) - MX: Distant metastasis cannot be evaluated; M0: No distant metastasis; M1: Distant metastasis is present.

In some embodiments, selecting the subject includes one or more of:
selecting a subject who is a human of at least 18 years of age; selecting a subject having no evidence of alopecia or mild alopecia; selecting a subject having hair follicles that are not apoptotic; selecting a subject having an Eastern Cooperative Oncology Group (ECOG) performance score of 0 or 1 within 14 days prior to beginning topical administration; selecting a subject having a baseline neutrophil count greater than 1500 cells/mm³ within 72 hours prior to beginning topical administration; and selecting a subject having a serum calcium level less than or equal to the upper limit of normal (ULN) within 72 hours prior to beginning topical administration.

In some embodiments, selecting the subject includes one or more of: selecting a subject who is not receiving a calcium lowering therapy or a drug that may affect calcium levels within 4 weeks of beginning topical administration, unless the subject is managed with bisphosphonates or calcium lowering therapy for 3 months or greater prior to beginning topical administration and have demonstrated evidence for stability of calcium metabolism; selecting a subject who does not have a history of hypercalcemia or vitamin D toxicity, or hospitalization for treatment for angina, myocardial infarction, or congestive heart failure or psychiatric illness within 30 days of beginning topical administration; selecting a subject who does not take a vitamin D supplement during topical administration, unless the subject has been taking the vitamin D supplement for 30 days or more prior to beginning topical administration and maintains the same dose throughout topical administration; selecting a subject who is not being treated with a medication that is known to affect calcium levels within 4 weeks of beginning topical administration, with the exception of subjects on stable therapy for more than 6 months; selecting a subject who is not receiving a thiazide or furosemide diuretic, with the exception of subjects who have stable doses and have been on therapy for over 6 months; selecting a subject who does not have hypercalcemia or kidney stones; and selecting a subject who does not have alopecia grade 2 or greater as per National Cancer Institute Common Terminology Criteria for Adverse Events (NCU-CTCAE) v4.0 or significant hair loss or hair breakage.

In one embodiment, the subject is a human of at least 18 years of age. In one embodiment, the subject has no evidence of alopecia or mild alopecia. In one embodiment, the subject has hair follicles that are not apoptotic. In one embodiment, the subject has an Eastern Cooperative Oncology Group (ECOG) performance score of 0 or 1 within 14 days prior to beginning topical administration. In one embodiment, the subject has a baseline neutrophil count greater than 1500 cells/mm3 within 72 hours prior to beginning topical administration. In one embodiment, the subject has a serum calcium level less than or equal to the upper limit of normal (ULN) within 72 hours prior to beginning topical administration. In one embodiment, the subject is not receiving a calcium lowering therapy or a drug that may affect calcium levels within 4 weeks of beginning topical administration, unless the subject is managed with bisphosphonates or calcium lowering therapy for 3 months or greater prior to beginning topical administration and has demonstrated evidence for stability of calcium metabolism. In one embodiment, the subject does not have a history of hypercalcemia or vitamin D toxicity within 30 days of beginning the topical administration. In one embodiment, the subject does not have a history of hospitalization for treatment for angina, myocardial infarction, or congestive heart failure or psychiatric illness within 30 days of beginning topical administration. In one embodiment, the subject does not take a vitamin D supplement during topical administration, unless the subject has been taking the vitamin D supplement for 30 days or more prior to beginning topical administration and maintains the same dose throughout topical administration. In one embodiment, the subject is not being treated with a medication that is known to affect calcium levels within 4 weeks of beginning topical administration, with the exception of subjects on stable therapy for more than 6 months. In one embodiment, the subject is not receiving a thiazide or furosemide diuretic, with the exception of subjects who have stable doses and have been on therapy for over 6 months. In one embodiment, the subject does not have hypercalcemia or kidney stones. In one embodiment, the subject does not have alopecia grade 2 or greater as per National Cancer Institute Common Terminology Criteria for Adverse Events (NCU-CTCAE) v4.0 or significant hair loss or hair breakage.

In one embodiment, a subject is selected based on any combination of two, three, four, five, six, seven, eight, nine, ten, eleven, twelve, thirteen, or fourteen criteria listed above.

The term "individual" or "subject" gerneally includes those animals that can exhibit alopecia. Generally, an individual can be a mammal, for example, a cat, dog, primate, mouse, rat, rabbit, cattle, horse, goat, sheep, pig, and the like. The mammal can be a primate, for example, a chimpanzee, human, gorilla, bonobo, orangutan, monkey, and the like or a human. According to the present invention, the individual or subject is a human. An individual or subject can be further categorized by gender and/or age.

As used herein, the term "chemotherapy" includes therapeutic treatment by chemical means. Chemotherapy can include essentially any chemotherapy that can cause alopecia, or a particular class, category, type, sub-type, or variety thereof. In various embodiments, the chemotherapy is cancer chemotherapy.

In some embodiments, the chemotherapy includes taxane based cancer chemotherapy. "Taxane based chemotherapy" can include a taxane therapeutic, a taxane therapeutic using a particular vehicle, a combination of two or more taxane therapeutics, and a combination of a taxane therapeutic and an additional therapeutic, and the like. Likewise, terms such as "paclitaxel based chemotherapy," "nab-paclitaxel based chemotherapy," and "docetaxel based chemotherapy" can be used to denote a paclitaxel/nab-paclitaxel/docetaxel therapeutic, a paclitaxel/nab-paclitaxel/docetaxel therapeutic using a particular vehicle, a combination of two or more paclitaxel/nab-paclitaxel/docetaxel therapeutics, and a combination of a paclitaxel/nab-paclitaxel/docetaxel therapeutic and an additional therapeutic, and the like.

In some embodiments, the taxane based cancer chemotherapy can include paclitaxel, nanoparticle albumin-bound ("nab") paclitaxel, and/or docetaxel cancer chemotherapy. In one embodiment the taxane based cancer chemotherapy is a combination of a taxane therapeutic (e.g., cancer chemotherapy) and an additional therapeutic (e.g., cancer chemotherapy).

In one embodiment, the taxane based chemotherapy does not include paclitaxel. In one embodiment, the taxane based chemotherapy does not include docetaxel. In one embodiment, the taxane based chemotherapy does not include paclitaxel or docetaxel.

In some embodiments, the taxane based cancer chemotherapy includes a taxane therapeutic, where the taxane therapeutic include one or more of: paclitaxel, docetaxel, nanoparticle albumin-bound nab paclitaxel, paclitaxel bonded to a polyglutamate polymer, paclitaxel bonded to docosahexaenoic acid, tumor-activated taxol prodrug, paclitaxel-Angiopep-2 conjugate (ANG1005), paclitaxel polyglumex, co-polymer combination paclitaxel, liposomal-encapsulated paclitaxel, taxol in vitamin E emulsion, and equivalents thereof.

In some embodiments, the taxane based cancer chemotherapy includes paclitaxel. In some embodiments, the taxane based cancer chemotherapy includes docetaxel. In some embodiments, the taxane based cancer chemotherapy includes nanoparticle albumin-bound nab paclitaxel. In some embodiments, the taxane based cancer chemotherapy includes paclitaxel bonded to a polyglutamate polymer. In some embodiments, the taxane based cancer chemotherapy includes paclitaxel bonded to docosahexaenoic acid. In some embodiments, the taxane based cancer chemotherapy includes tumor-activated taxol prodrug. In some embodiments, the taxane based cancer chemotherapy includes paclitaxel-Angiopep-2 conjugate (ANG1005). In some embodiments, the taxane based cancer chemotherapy includes paclitaxel polyglumex. In some embodiments, the taxane based cancer chemotherapy includes co-polymer combination paclitaxel. In some embodiments, the taxane based cancer chemotherapy includes liposomal-encapsulated paclitaxel. In some embodiments, the taxane based cancer chemotherapy includes taxol in vitamin E emulsion, and equivalents thereof.

In some embodiments, the taxane based cancer chemotherapy includes an additional chemotherapeutic. For example, the additional chemotherapeutic can include one or more of: Anthracyclines (Adriamycin/Doxorubicin, Daunorubicin, Epirubicin, Idarubicin, Valrubicin), 5-FU, Tamoxifen, Irinotecan, Carboplatin, Etoposide, Cytoxan/Cyclophosphamide, Cisplatin, Erlotinib (Tarceva), Gemcitabine, Staurosporin, Vincristine, Imatinib (Gleevec), Gefitinib (Iressa), Sorafenib, Dasatinib, Dactinomycin, Hexamethamelamine (HMM, altretamine), Ifosfamide, bleomycin, methotrexate, Vindesine, Vinorelbine, Topotecan, Amsacrine, Cytarabine, Busulphan, Melphalan, Vinblastine, Lomustine(CCNU), Thiotepa, Gemcitabine, Carmustine(BCNU), Mitroxantrone, Mitomycin C, Procarbazine, 6-Mercaptopurine, Sreptozotocin, Fludarabine, Raltitrexate (Tomudex), Capecitabine, and equivalents thereof.

In some embodiments, the cancer includes metastatic breast cancer and the chemotherapy includes paclitaxel based, nab-paclitaxel, or docetaxel based chemotherapy, each optionally in combination with carboplatin.

In some embodiments, the cancer includes ovarian cancer and the chemotherapy includes a paclitaxel and/or docetaxel based chemotherapy, optionally in combination with carboplatin.

In some embodiments, the cancer includes uterine cancer and the chemotherapy includes docetaxel based chemotherapy, optionally in combination with gemcitabine.

In some embodiments, the cancer includes cervical cancer and the chemotherapy includes paclitaxel based chemotherapy, optionally in combination with cisplatin and/or topotecan.

In some embodiments, the pharmaceutical compositions for use of the invention do not substantially reduce the efficacy of chemotherapy, especially systemic chemotherapy. In other embodiments, the pharmaceutical compositions for use of the invention enhances the efficacy of chemotherapy. The language "without interfering with the efficacy of a co-administered chemotherapeutic agent" includes the situation where the vitamin D compound, when administered with one or more chemotherapeutic agents, does not interrupt the biological or therapeutic activity of the one or more chemotherapeutic agents or prevent the one or more chemotherapeutic agents from performing its desired biological or therapeutic activity. The language "without reducing the efficacy of a co-administered chemotherapeutic agent" includes the situation where the vitamin D compound, when administered with one or more chemotherapeutic agents, does not decrease the biological or therapeutic activity of the one or more chemotherapeutic agents.

The pharmaceutical compositions for use of the invention may be used with any chemotherapeutic agent or combination of chemotherapeutic agents that have a cytotoxic effect on the hair follicle or dermal papilla, or is otherwise capable of inducing alopecia. The language "chemotherapeutic agent," "chemotherapy," and "chemotherapeutic regimen" include Anthracyclines (Adriamycin/Doxorubicin, Daunorubicin, Epirubicin, Idarubicin, Valrubicin), 5-FU, Tamoxifen, Irinotecan, Paclitaxel (Taxol), Carboplatin, Etoposide, Cytoxan/Cyclophosphamide, Cisplatin, Erlotinib (Tarceva), bevacizumab, Gemcitabine, Staurosporin, Vincristine, Imatinib (Gleevec), Gefitinib (Iressa), Sorafenib, Dasatinib, Dactinomycin, Hexamethamelamine (HMM, altretamine), Ifosfamide, bleomycin, methotrexate, Docetaxel (Taxotere), Vindesine, Vinorelbine, Topotecan, Amsacrine, Cytarabine, Busulphan, Melphalan, Vinblastine, Lomustine(CCNU), Thiotepa, Gemcitabine, Carmustine(BCNU), Mitroxantrone, Mitomycin C, Procarbazine, 6-Mercaptopurine, Sreptozotocin, Fludarabine, Raltitrexate (Tomudex), Capecitabine, and equivalents thereof.

In some embodiments, the chemotherapy is systemic chemotherapy.

The pharmaceutical compositions for use of the invention preferably do not substantially reduce the efficacy of the chemotherapy, especially systemic chemotherapy. Preferably, the pharmaceutical compositions for use of the invention enhance the efficacy of the chemotherapy.

The pharmaceutical compositions for use of the invention may also be used with any chemotherapeutic hormone therapies or biological therapies that can cause hair thinning.

Topically administering the pharmaceutical composition includes administering the pharmaceutical composition to the subject prior to the commencement of chemotherapy and/or concurrently with chemotherapy.

In some embodiments, the vitamin D compound is co-administered with a chemotherapeutic agent. The language "co-administered with a chemotherapeutic agent" includes administration of the vitamin D compound at substantially the same time as the chemotherapeutic agent. For example, the vitamin D compound may be co-administered with the chemotherapeutic agent; the vitamin D compound may be administered first, and immediately followed by the administration of the chemotherapeutic agent or the chemotherapeutic agent may be administered first, and immediately followed by the administration of the vitamin D compound.

In other embodiments, the individual has not already developed symptoms of alopecia (e.g., alopecia has not commenced). The language "therapeutically effective amount" includes that amount of a vitamin D compound necessary or sufficient to prevent or treat alopecia in an individual.

In some embodiments, topically administering the pharmaceutical composition includes administering the pharmaceutical composition for 4-7 days prior to the beginning (i.e., commencement) of the chemotherapy. Topically administering the pharmaceutical composition can include administering the pharmaceutical composition for 1, 2, 3, 4, 5, 6, 7, 8, 9, 10, 11, 12, 13, 14, 21, 28, 35, 40, or 52 days prior to the commencement of the chemotherapy. Topically administering the pharmaceutical composition can include administering the pharmaceutical composition for at least 1, 2, 3, 4, 5, 6, 7, 8, 9, 10, 11, 12, 13, 14, 21, 28, 35, 40, or 52 days prior to the commencement of the chemotherapy.

In some embodiments, topically administering the pharmaceutical composition includes administering the pharmaceutical composition for 5 ±2 days, i.e., 3-7 days prior to the commencement of the chemotherapy. In some embodiments, topically administering the pharmaceutical composition includes administering the pharmaceutical composition for 7 ±2 days, i.e., 5-9 days prior to the commencement of the chemotherapy. In some embodiments, topically administering the pharmaceutical composition includes administering the pharmaceutical composition for 7-14 days prior to the commencement of the chemotherapy. In some embodiments, topically administering the pharmaceutical composition includes administering the pharmaceutical composition for 8-14 days prior to the commencement of the chemotherapy. In some embodiments, topically administering the pharmaceutical composition includes administering the pharmaceutical composition for 9-14 days prior to the commencement of the chemotherapy. In some embodiments, topically administering the pharmaceutical composition includes administering the pharmaceutical composition for 10-14 days prior to the commencement of the chemotherapy. In some embodiments, topically administering the pharmaceutical composition includes administering the pharmaceutical composition for 11-14 days prior to the commencement of the chemotherapy. In some embodiments, topically administering the pharmaceutical composition includes administering the pharmaceutical composition for 12-14 days prior to the commencement of the chemotherapy. In some embodiments, topically administering the pharmaceutical composition includes administering the pharmaceutical composition for 13-14 days prior to the commencement of the chemotherapy.

In some embodiments, topically administering the pharmaceutical composition includes administering the pharmaceutical composition for at least 3, 4, 5, 6, 7, 8, 9, 10, 11, 12, or 13 days prior to the commencement of the chemotherapy. For example, in one embodiment, topically administering the pharmaceutical composition includes administering the pharmaceutical composition for at least 4 days prior to the commencement of the chemotherapy. In one embodiment, topically administering the pharmaceutical composition includes administering the pharmaceutical composition for at least 5 days prior to the commencement of the chemotherapy. In one embodiment, topically administering the pharmaceutical composition includes administering the pharmaceutical composition for at least 6 days prior to the commencement of the chemotherapy. In one embodiment, topically administering the pharmaceutical composition includes administering the pharmaceutical composition for at least 7 days prior to the commencement of the chemotherapy. In one embodiment, topically administering the pharmaceutical composition includes administering the pharmaceutical composition for at least 8 days prior to the commencement of the chemotherapy. In one embodiment, topically administering the pharmaceutical composition includes administering the pharmaceutical composition for at least 9 days prior to the commencement of the chemotherapy. In one embodiment, topically administering the pharmaceutical composition includes administering the pharmaceutical composition for at least 10 days prior to the commencement of the chemotherapy. In one embodiment, topically administering the pharmaceutical composition includes administering the pharmaceutical composition for at least 11 days prior to the commencement of the chemotherapy. In one embodiment, topically administering the pharmaceutical composition includes administering the pharmaceutical composition for at least 12 days prior to the commencement of the chemotherapy. In one embodiment, topically administering the pharmaceutical composition includes administering the pharmaceutical composition for at least 13 days prior to the commencement of the chemotherapy. In one embodiment, topically administering the pharmaceutical composition includes administering the pharmaceutical composition for at least 14 days prior to the commencement of the chemotherapy.

For example, in one embodiment, topically administering the pharmaceutical composition includes administering the pharmaceutical composition for at least 15 days prior to the commencement of the chemotherapy. In one embodiment, topically administering the pharmaceutical composition includes administering the pharmaceutical composition for at least 16 days prior to the commencement of the chemotherapy. In one embodiment, topically administering the pharmaceutical composition includes administering the pharmaceutical composition for at least 17 days prior to the commencement of the chemotherapy. In one embodiment, topically administering the pharmaceutical composition includes administering the pharmaceutical composition for at least 18 days prior to the commencement of the chemotherapy. In one embodiment, topically administering the pharmaceutical composition includes administering the pharmaceutical composition for at least 19 days prior to the commencement of the chemotherapy. In one embodiment, topically administering the pharmaceutical composition includes administering the pharmaceutical composition for at least 20 days prior to the commencement of the chemotherapy. In one embodiment, topically administering the pharmaceutical composition includes administering the pharmaceutical composition for at least 21 days prior to the commencement of the chemotherapy.

In one embodiment, the pharmaceutical composition is topically administered 3, 4, 5, 6, 7, 8, 9, 10, 11, 12, 13, or 14 days prior to the commencement of the chemotherapy. In one embodiment, the pharmaceutical composition is topically administered 9 days prior to the commencement of the chemotherapy. In one embodiment, the pharmaceutical composition is topically administered 18 days prior to the commencement of the chemotherapy. In one embodiment, the pharmaceutical composition is topically administered 11 days prior to the commencement of the chemotherapy. In one embodiment, the pharmaceutical composition is topically administered 12 days prior to the commencement of the chemotherapy. In one embodiment, the pharmaceutical composition is topically administered 13 days prior to the commencement of the chemotherapy. In one embodiment, the pharmaceutical composition is topically administered 14 days prior to the commencement of the chemotherapy.

While not wishing to be bound by any particular theory, it is believed that application of vitamin D to the scalp or any other area of skin having hair induces differentiation of hair follicles, which is required for the stage conversion of the hair from a growing anagen stage to an involuting catagen stage. It has been discovered by Applicants in experiments carried out in an animal model (e.g., rat) that a minimal treatment duration with calcitriol is required for completing the required differentiation in scalp hair follicles and the conversion to the catagen stage, which subsequently makes them resistant to the cytotoxicity of chemotherapy. In particular, it was found by Applicants using a rat model that application of calcitriol daily at least four days prior to commencement of chemotherapy was required to see any protective effect, with moderate protection at 5 days and increasing protection up to one week of treatment prior to chemotherapy. In humans, a hair follicle of the scalp has a substantially longer anagen phase than that of animals. Indeed, at any time, at least 90% of hair follicles in a human scalp are in the anagen phase. It is believed that a short treatment duration is not sufficient to induce catagen stage in scalp hair follicles, which subsequently makes them more susceptible to cytotoxicity of chemotherapy. To ensure completion of anagen to catagen stage conversion and adequate prevention of chemotherapy-induced alopecia, topical calcitriol is applied at least 4, 5, 6, 7, 8, 9, 10, 11, 12, 13, or 14 days, and preferably at least two weeks or longer, prior to the initiation of chemotherapy, which will induce the catagen phase and thereby provide protection against CIA. Continued application on a daily basis will ensure the maintenance of the catagen stage and extended protection throughout administration of multiple doses of a chemotherapeutic (e.g., taxane-containing)regimen.

Accordingly, in some embodiments, topically administering the pharmaceutical composition includes administering the pharmaceutical composition for at least two weeks prior to the commencement of the chemotherapy. Topically administering the pharmaceutical composition can include administering the pharmaceutical composition for at least 5-7 days prior to the commencement of the chemotherapy. Topically administering the pharmaceutical composition can include administering the pharmaceutical composition for at least 1, 2, 3, 4, 5, 6, 7, or 8 weeks prior to the commencement of the chemotherapy.

In some embodiments, topically administering the pharmaceutical composition includes administering the pharmaceutical composition for the duration of the chemotherapy.

In some embodiments, topically administering the pharmaceutical composition includes administering the pharmaceutical composition for at least three months after beginning of the chemotherapy. Topically administering the pharmaceutical composition can include administering the pharmaceutical composition for at least 1, 2, 3, 4, 5, or 6 months after beginning of the chemotherapy.

In some embodiments, topically administering the pharmaceutical composition includes administering the pharmaceutical composition to the subject after the commencement of chemotherapy, but prior to the commencement of chemotherapy induced alopecia.

In some embodiments, topically administering the pharmaceutical composition includes administering the pharmaceutical composition twice daily. In some embodiments, the two daily administrations are separated by about 10-14 hours. In some embodiments, the two daily administrations are separated by about 8, 9, 10, 11, 12, 13, 14, 15, or 16 hours.

In certain embodiments, the vitamin D compounds of the invention is administered to the individual over a period of about 1 day, about 2 days, about 3 days, about 4 days, about 5 days, about 6 days, about 7 days, about 8 days, about 9 days, about 10 days, about 11 days, about 12 days, about 13 days, about 2 weeks, about 3 weeks, about 4 weeks, about 6 weeks, 8 about weeks, about 3 months, about 4 months, about 5 months, about 6 months, about 7 months, about 8 months, about 9 months, about 10 months, about 11 months or about a year. In some embodiments, the vitamin D compounds of the invention may be administered every day during the treatment period, on alternative days, or every three days.

In certain embodiments, the vitamin D compounds of the invention are administered once daily, twice daily, or three times daily in each treatment day.

In certain embodiments, each administration of the vitamin D compounds of the invention is applied to the same location, or to several different locations on the individual. When applied to different locations, the doses for each location may be the same, or be adjusted based on factors such as skin thickness and differences in drug penetration (if any).

In certain embodiments, the vitamin D compounds of the invention is topically administered to the scalp twice daily each day for two consecutive weeks prior to the commencement of chemotherapy in order to prevent or reduce the severity of any CIA that may occur upon commencement of chemotherapy.

Accoriding to the present invention, the concentration of the vitamin D compound in the topical formulation is 5-20 µg/mL. In one embodiment, the concentration of the vitamin D compound in the topical formulation is 5 µg/mL, In one embodiment, the concentration of the vitamin D compound in the topical formulation is 10 µg/mL, In one embodiment, the concentration of the vitamin D compound in the topical formulation is 20 µg/mL.

According to the present invention, topically administering the pharmaceutical composition includes administering a 1.0 mL dose of the pharmaceutical composition, using a metered spray unit. In some embodiments, topically administering the pharmaceutical composition includes administering a 1.0 mL dose of the pharmaceutical composition, with 0.25 mL to each of the four quadrants of the scalp, using a metered spray unit.

According to the present invention, topically administering the pharmaceutical composition includes administering the vitamin D compound at a concentration of 5, 10, or 20 µg/mL in the pharmaceutical composition. Topically administering the pharmaceutical composition can include administering the vitamin D compound at a concentration of 5, 6, 7, 8, 9, 10, 15, or 20 µg/mL in the pharmaceutical composition.

In some embodiments, topically administering the pharmaceutical composition includes administering a total daily dose of about 10-40 µg of the vitamin D compound to the scalp per day. In one embodiment, topically administering the pharmaceutical composition can include administering a total daily dose of about 5, 10, 20, 30, 40, 50, 60, 75, 80, 100, 10-20, 10-30, 10-50, 20-30, 20-40, 20-50, or 40-50 µg of the vitamin D compound to the scalp per day. In one embodiment, topically administering the pharmaceutical composition can include administering a total daily dose of about 10-90, 20-80, 30-70, 10-20, 10-30, 10-40, 10-50, 10-60, 20-30, 20-40, 20-50, 20-60, 20-70, 30-40, 30-50, 30-60, 40-50, 40-60, 40-70, 40-80, 50-60, 50-70, 50-80, 50-90, 60-70, 60-80, 60-90, 60-100, 70-80, 70-90 or 70-100 µg of the vitamin D compound to the scalp per day. In one embodiment, topically administering the pharmaceutical composition can include administering a total daily dose of about 5, 10, 15, 20, 25, 30, 35, 40, 45, 50, 55, 60, 65, 70, 75, 80, 85, 90, 95, or 100 µg of the vitamin D compound to the scalp per day. In one embodiment, topically administering the pharmaceutical composition includes administering a total daily dose of about 10 µg of the vitamin D compound to the scalp per day. In one embodiment, topically administering the pharmaceutical composition includes administering a total daily dose of about 20 µg of the vitamin D compound to the scalp per day. In one embodiment, topically administering the pharmaceutical composition includes administering a total daily dose of about 40 µg of the vitamin D compound to the scalp per day. In one embodiment, topically administering the pharmaceutical composition includes administering a total daily dose of about 60 µg of the vitamin D compound to the scalp per day. In one embodiment, topically administering the pharmaceutical composition includes administering a total daily dose of about 80 µg of the vitamin D compound to the scalp per day. In one embodiment, topically administering the pharmaceutical composition includes administering a total daily dose of about 100 µg of the vitamin D compound to the scalp per day.

In one embodiment, the total daily dose is administered in a single dose. In one embodiment, the total daily dose is administered in two individual doses. In one embodiment, administering a total daily dose in three individual doses. In one embodiment, the total daily dose is administered in four individual doses.

In one embodiment, the pharmaceutical composition is administered twice daily for a total daily dose of 10-40 µg of the vitamin D compound, wherein each of 2 individual doses per day is 5-20 µg. In one embodiment, the pharmaceutical composition is administered twice daily for a total daily dose of 10 µg of the vitamin D compound, wherein each of 2 individual doses per day is 5 µg. In one embodiment, the pharmaceutical composition is administered twice daily for a total daily dose of 20 µg of the vitamin D compound, wherein each of 2 individual doses per day is 10 µg. In one embodiment, the pharmaceutical composition is administered twice daily for a total daily dose of 40 µg of the vitamin D compound, wherein each of 2 individual doses per day is 20 µg.

The language "effective concentration" includes the concentration of the vitamin D compound in a topical formulation that is necessary or sufficient to prevent or treat alopecia in an individual. In certain embodiments, the concentration of the vitamin D compound in the topical formulation is 5, 6, 7, 8, 9, 10, 15, or 20 µg/mL. According to the present invention, the concentration of the vitamin D compound in the topical formulation is 5-20µg/mL. In one embodiment, the concentration of the vitamin D compound in the topical formulation is 5 µg/mL, In one embodiment, the concentration of vitamin D compound in the topical formulation is 10 µg/mL, In one embodiment, the concentration of vitamin D compound in the topical formulation is 20 µg/mL.

In some embodiments, topically administering the pharmaceutical composition includes substantially avoiding dermal delivery of the vitamin D compound. In some embodiments, the vitamin D compound is topically delivered to and/or accumulated in the epidermis while substantially avoiding delivery and/or accumulation in the dermis.

As used herein, the language "substantially avoiding dermis delivery and/or accumulation" includes the delivery and/or accumulation to the dermis of less than about 25% of the vitamin D compound as compared to the delivery and/or accumulation of the vitamin D compound to the epidermis, for example, less than about 20%, less than about 15%, less than about 10%, less than about 5%, less than about 1% or no delivery and/or accumulation of the vitamin D compound to the dermis when compared to the amount delivered to the epidermis. In some embodiments, between about 1% and 25% of the vitamin D compound is delivered and/or accumulated to the dermis, for example, between about 1% and about 20%, between about 1% and about 15%, between about 1% and about 10% or between about 1% and about 5%, as compared to the delivery and/or accumulation to the epidermis. In some embodiments, the vitamin D compound is not delivered and/or accumulated in the dermis. In some embodiments, the amount of vitamin D compound that is delivered to, or accumulates in, the dermis is less than about 0.3 ng/cm², less than about 0.2 ng/cm² or less than about 0.1 ng/cm².

In some embodiments, the vitamin D compound is formulated to be delivered to/accumulated in human epidermis, especially epidermis of the scalp or neck region, while substantially avoiding delivery to/accumulation in the dermis. One of skill in the art would readily be able to determine the amount of the vitamin D compound, or lack thereof, delivered to/accumulated in the dermis and/or the epidermis using Example 1.

The language "vitamin D compound" includes compounds of Formula I: 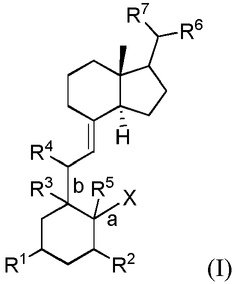 wherein
a and b are each independently a single or double bond;
X is -CH₂ when a is a double bond, or X is hydrogen or a hydroxyl substituted alkyl when a is a single bond;
R¹ is hydrogen, hydroxyl, alkoxyl, tri-alkyl silyl or alkyl, optionally substituted with one to three halogen, hydroxyl, cyano or -NR'R" moieties;
R² is hydrogen, hydroxyl, -O-trialkyl silyl, or alkyl, alkoxyl or alkenyl, optionally substituted with one to three halogen, hydroxyl, cyano or -NR'R" moieties;
R³ is absent when b is a double bond or R³ is hydrogen, hydroxyl or alkyl, or R³ and R¹ together with the carbon atoms to which they are attached may be linked to form 5-7 membered carbocyclic ring when b is a single bond;
R⁴ is absent when b is a double bond or hydrogen, halogen or hydroxyl when b is a single bond;
R⁵ is absent when a is a double bond or R⁵ is hydrogen, halogen or hydroxyl when a is a single bond;
R⁶ is alkyl, alkenyl, alkynyl, cycloalkyl, heterocyclicyl, alkyl-O-alkyl, alkyl-CO₂-alkyl optionally substituted with one to five, hydroxyl, oxo, halogen, alkoxyl, aryl, heteroaryl, cyano, nitro or -NR'R" moieties;
R⁷ is alkyl optionally substituted with one to three hydroxyl, halogen, alkoxyl, aryl, heteroaryl, cyano, nitro or -NR'R" moieties; and,
R' and R" are each, independently, hydrogen, hydroxyl, halogen, alkyl or alkoxyl, and pharmaceutically acceptable salts thereof.

In some embodiments, R¹ is hydroxyl, R² is hydrogen or hydroxyl, a is a double bond, R⁵ is absent, X is -CH₂, b is a double bond, R³ and R⁴ are absent, R⁶ is alkyl (*e.g*., methyl), and R⁷ is alkyl (*e.g*., a substituted or unsubstituted alkyl, for example, a hydroxyl substituted alkyl.

In one embodiment, the vitamin D compound is selected from the following structures, or stereoisomers or pharmaceutically acceptable salts thereof:

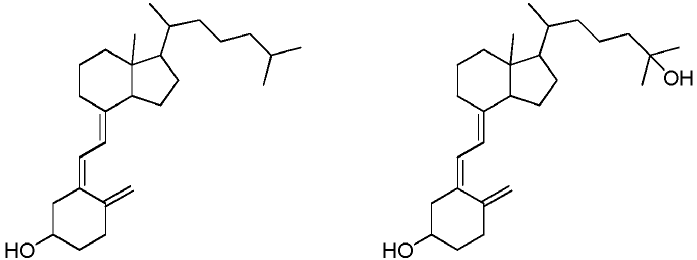

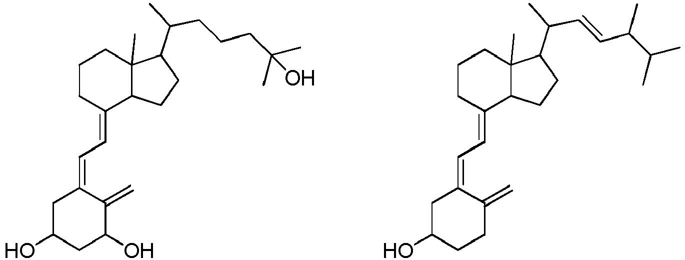

In certain embodiments, the vitamin D compound is 1α-hydroxyvitamin D3; 1α,24-dihydroxyvitamin D3, calcitriol, calcipotriol or 1,25-dihydroxy-16-ene-23-yne-cholecalciferol.

In some embodiments, the vitamin D compound is calcitriol (1,25-dihydroxyvitamin D3).
Other suitable analogs, metabolites, derivatives and/or mimics of vitamin D compounds include, for example, 1,25-dihydroxyvitamin D3 (also known as calcitriol), 1,25-dihydroxy-16-ene-23-yne-cholecalciferol, and other vitamin D analogs, homologs, mimics, and derivatives of vitamin D compounds such as those described in the following patents: U.S. Pat. Nos. 4,391,802 (lα-hydroxyvitamin D derivatives); 4,717,721 (lα-hydroxy derivatives with a 17 side chain greater in length than the cholesterol or ergosterol side chains); 4,851,401 (cyclopentano-vitamin D analogs); 4,866,048 and 5,145,846 (vitamin D3 analogues with alkynyl, alkenyl, and alkanyl side chains); 5,120,722 (trihydroxycalciferol); 5,547,947 (fluoro-cholecalciferol compounds); 5,446,035 (methyl substituted vitamin D); 5,411,949 (23-oxa-derivatives); 5,237,110 (l9-nor-vitamin D compounds); 4,857,518 (hydroxylated 24-homo-vitamin D derivatives). Other suitable examples include ROCALTROL (Roche Laboratories); CALCIJEX injectable calcitriol; investigational drugs from Leo Pharmaceuticals including EB 1089 (24a,26a,27a,trihomo-22,24-diene-la,25-(OH)2-D3, KH 1060 (20-epi-22-oxa-24a,26a,27a-trihomola, 25-(OH)2-D3), MC 1288 (1,25-(OH)2-20-epi-D3) and MC 903 (calcipotriol, la,24s(OH)2-22-ene-26,27-dehydro-D3); Roche Pharmaceuticals drugs that include 1,25-(OH)2-16-ene-D3, 1,25-(OH)2-16-ene-23-yne-D3, and 25-(OH)2-16-ene-23-yne-D3; Chugai Pharmaceuticals 22-oxacalcitriol (22-oxa-1α,25-(OH)2-D3; 1α-(OH)-D5 from the University of Illinois; and drugs from the Institute of Medical Chemistry-Schering AG that include ZK 161422 (20-methyl-1,25-(OH)2-D3) and ZK 157202 (20-methyl-23-ene-1,25-(OH)2-D3); 1α-(OH)-D2; lα-(OH)-D3, 1α-(OH)-D4, 25-(OH)-D2; 25-(OH)-D3; and 25-(OH)-D4. Additional examples include lα,25-(OH)2-26,27-d6-D3; lα,25-(OH)2-22-ene-D3; 1α,25-(OH)2-D3; 1α,25-(OH)2-D2; 1α,25-(OH)2-D4; 1α,24,25-(OH)3-D3; 1α,24,25-(OH)3-D2; 1α,24,25-(OH)3-D4; 1α-(OH)-25-FD3; 1α-(OH)-25-FD4; 1α-(OH)-25-FD2; lα,24-(OH)2-D4; lα,24-(OH)2-D3; lα,24-(OH)2-D2; lα,24-(OH)2-25-FD4; lα,24-(OH)2-25-FD3; 1α,24-(OH)2-25-FD2; 1α,25-(OH)2-26,27-F6-22-ene-D3; 1α,25(OH)2-26,27-F6-D3; lα,25S-(OH)2-26-F3-D3; lα,25-(OH)2-24-F2-D3; lα,25S,26-(OH)2-22-ene-D3; lα,25R,26-(OH)2-22-ene-D3; 1α,25-(OH)2-D2; 1α,25-(OH)2-24-epi-D3; la,25-(OH)2-23-yne-D3; lα,25-(OH)2-24R-F-D3; lα,25S,26-(OH)2-D3; lα,24R-(OH)2-25F-D3; 1α,25-(OH)2-26,27-F6-23-yne-D3; 1α,25R-(OH)2-26-F3-D3; 1α,25,28-(OH)3-D2; 1α,25-(OH)2-16-ene-23-yne-D3; 1α,24R,25-(OH)3-D3; 1α,25-(OH)2-26,27-F6-23-ene-D3; 1α,25R-(OH)2-22-ene-26-F3-D3; lα,25S-(OH)2-22-ene-26-F3-D3; 1α,25R-(OH)2-D3-26,26,26-d3; lα,25S-(OH)2-D3-26,26,26-d3; and 1α,25R-(OH)2-22-ene-D3-26,26,26-d3. Yet additional examples can be found in U.S. Pat. No. 6,521,608. See also, *e.g.*, S.S. Pat. Nos. 6,503,893, 6,482,812, 6,441,207, 6,410,523, 6,399,797, 6,392,071, 6,376,480, 6,372,926, 6,372,731, 6,359,152, 6,329,357, 6,326,503, 6,310,226, 6,288,249, 6,281,249, 6,277,837, 6,218,430, 6,207,656, 6,197,982, 6,127,559, 6,103,709, 6,080,878, 6,075,015, 6,072,062, 6,043,385, 6,017,908, 6,017,907, 6,013,814, 5,994,332, 5,976,784, 5,972,917, 5,945,410, 5,939,406, 5,936,105, 5,932,565, 5,929,056, 5,919,986, 5,905,074, 5,883,271, 5,880,113, 5,877,168, 5,872,140, 5,847,173, 5,843,927, 5,840,938, 5,830,885, 5,824,811, 5,811,562, 5,786,347, 5,767,111, 5,756,733, 5,716,945, 5,710,142, 5,700,791, 5,665,716, 5,663,157, 5,637,742, 5,612,325, 5,589,471, 5,585,368, 5,583,125, 5,565,589, 5,565,442, 5,554,599, 5,545,633, 5,532,228, 5,508,392, 5,508,274, 5,478,955, 5,457,217, 5,447,924, 5,446,034, 5,414,098, 5,403,940, 5,384,313, 5,374,629, 5,373,004, 5,371,249, 5,430,196, 5,260,290, 5,393,749, 5,395,830, 5,250,523, 5,247,104, 5,397,775, 5,194,431, 5,281,731, 5,254,538, 5,232,836, 5,185,150, 5,321,018, 5,086,191, 5,036,061, 5,030,772, 5,246,925, 4,973,584, 5,354,744, 4,927,815, 4,804,502, 4,857,518, 4,851,401, 4,851,400, 4,847,012, 4,755,329, 4,940,700, 4,619,920, 4,594,192, 4,588,716, 4,564,474, 4,552,698, 4,588,528, 4,719,204, 4,719,205, 4,689,180, 4,505,906, 4,769,181, 4,502,991, 4,481,198, 4,448,726, 4,448,721, 4,428,946, 4,411,833, 4,367,177, 4,336,193, 4,360,472, 4,360,471, 4,307,231, 4,307,025, 4,358,406, 4,305,880, 4,279,826, and 4,248,791.

Yet other compounds which may be utilized include vitamin D mimics such as bis-aryl derivatives disclosed by U.S. Pat. No. 6,218,430 and WO publication 2005/037755. Additional examples of non-secosteroidal vitamin D mimic compounds suitable for the present invention can be found in U.S. Pat. Nos. 6,831,106; 6,706,725; 6,689,922; 6,548,715; 6,288,249; 6,184,422, 6,017,907, 6,858,595, and 6,358,939.

Yet other suitable vitamin D3 analogs, metabolites, and/or derivatives which may be utilized include those identified in U.S. Patent Application Publication No. 2006/0177374.

The language "vitamin D analog" includes compounds that are similar to vitamin D in structure and function. In one embodiment, the vitamin D analog is a vitamin D3 analog (*e.g*., a compound that is similar to vitamin D3 in structure and function).

The language "vitamin D metabolite" includes compounds that are intermediates and the products involved in the metabolism of vitamin D. In one embodiment, the vitamin D metabolite is a vitamin D3 metabolite (*e.g.*, a compound that is an intermediate or product involved in the metabolism of vitamin D3).
The language "vitamin D derivative" includes compound that can arise from a parent compound (*e.g.*, vitamin D) by replacement of one atom with another atom or group of atoms. In one embodiment, the vitamin D derivative is a vitamin D3 derivative (*e.g.*, a compound that can arise from vitamin D3 by replacement of one atom with another atom or group of atoms).
The language "vitamin D mimic" includes compounds that can chemically imitate vitamin D in a biological process. In one embodiment, the vitamin D mimic is a vitamin D3 mimic (*e.g.*, a compound that can chemically imitate vitamin D3 in a biological process).

Vitamin D3 is absorbed after ingestion of fish liver oils or irradiated yeast. Plants and animal sources contain only the inactive vitamin D precursors, 7-dehydrocholesterol or ergosterol. 7-Dehydrocholesterol is stored in the skin and can be converted by sunlight into vitamin D3. However, whether ingested or formed by ultraviolet irradiation in the skin, Vitamin D has to be transformed into active metabolites. Vitamin D3 is converted to 25-hydroxycholecalciferol by liver enzymes. Then in the kidneys two compounds 1,25-dihydroxycholecalciferol and 24,25-dihydroxycholecalciferol are formed. The vitamin D active metabolites play an important role in the absorption of calcium from the intestinal tract, bone deposition and bone reabsorption.

The vitamin D compounds of the invention share certain common biological activities, such as the ability to prevent apoptosis in keratinocytes, partly *via* their ability to up- or down-regulate certain target gene expressions in, for example, normal keratinocytes (*e.g*., HEKa). Therefore, in certain embodiments, the vitamin D compounds of the invention may exhibit a similar or identical gene regulation profile as an equivalent amount of calcitriol in, for example, normal keratinocytes (*e.g*., HEKa).

As used herein, "equivalent amount" includes the same molar amount if the vitamin D compounds have substantially the same or equal biological or therapeutic activity in substantially the same molar amount. However, when different vitamin D compounds are not substantially the same or equal in biological or therapeutic activity, the language "equivalent amount" includes that amount of a vitamin D compound that gives rise to substantially the same amount of biological or therapeutic activity compared to a reference vitamin D compound (*e.g*., calcitriol).

The language "gene regulation profile" includes the list or spectrum of genes that are statistically significantly (*e.g*., p < 0.05) modulated (*e.g.*, up- or down-regulated) when comparing to appropriate controls. For example, upon contacting a cell with a vitamin D compound for a pre-determined period of time (*e.g*., 24 hours), a target cell may display a spectrum of genes whose mRNA or protein expression level is modulated (*e.g*., up- or down-regulated) compared to mock / vehicle-treatment control. The list of genes modulated (*e.g.*, up- or down-regulated) at the time of detection constitutes a snapshot of the gene expression profile of the cell at that specific moment.

The language "similar gene regulation profile" includes the situation where more than 50%, 60%, 70%, 80%, 90%, or more of the total number of target genes examined exhibit substantially the same direction of gene expression (*e.g.*, both up-regulated or both down-regulated, although the magnitude or extent of up- or down-regulation in each gene may differ).

The language "identical gene regulation profile" includes the situation where nearly all target genes examined exhibit the same direction of gene expression (*e.g*., both up-regulated or both down-regulated, although the magnitude or extent of up- or down-regulation in each gene may differ).

In one embodiment, a vitamin D compound of the invention promotes the expression of one or more target genes whose expression levels are promoted by an equivalent amount of a reference vitamin D compound (*e.g*., calcitriol). In other embodiments, the vitamin D compound of the invention inhibits the expression of one or more genes whose expression levels are inhibited by an equivalent amount of a reference vitamin D compound (*e.g*., calcitriol).

In certain embodiments, a vitamin D compound of the invention may modulate the expression of proteins in normal keratinocytes. The language "modulate expression of proteins" includes the up-regulation and the down-regulation of proteins in normal keratinocytes. In some embodiments, the vitamin D compound modulates the expression of HSPA2, HSF4 mRNA, HSPB1 or DNAJC6 mRNA. For example, in some embodiments, the vitamin D compound up-regulates the expression of HSPA2 or HSF4 mRNA, and/or down-regulates the expression of HSPB 1 or DNAJC6 mRNA in normal keratinocytes (e.g., HEKa).

In certain embodiments, a vitamin D compound of the invention modulates the expression of SLC1A1, KCNB2, KCNN4 protein or SLC1A3 protein in normal keratinocytes. In some embodiments, the vitamin D compound may up-regulate the expression of SLC1A1, KCNB2, or KCNN4 protein, and/or down-regulate the expression of SLC1A3 protein in normal keratinocytes (*e.g*., HEKa).

In certain embodiments, a vitamin D compound of the invention may modulate one or more proteins in Table 3-1 and Table 3-2. For example, in one embodiment, the vitamin D compound may up-regulate the expression of one or more proteins in Table 3-1 by at least about 2-fold, and/or down-regulate the expression of one or more proteins in Table 3-2 by at least about 2-fold in, for example, normal keratinocytes (*e.g.*, HEKa).

In certain embodiments, a vitamin D compound of the invention may induce over-expression of one or more proteins in any of Tables 3-3, 3-4, 3-5 or 3-6, after about 24-hour exposure of normal keratinocytes (*e.g*., HEKa) to the vitamin D compound.

In certain embodiments, a vitamin D compound of the invention may induce over-expression in normal keratinocytes (*e.g*., HEKa) of one or more of: GST, Keratin 1, Keratin 17, Galectin 1, S 100 A9 (Calprotectin), or S100 A13.

As used herein, the term "alkyl" includes fully saturated branched or unbranched (*e.g*., straight chain or linear) hydrocarbon moiety, comprising 1 to 20 carbon atoms, for example,1 to 7 carbon atoms, or1 to 4 carbon atoms. Representative examples of alkyl moieties include methyl, ethyl, *n*-propyl, *iso*-propyl, *n*-butyl, *sec*-butyl, *iso*-butyl, *tert-*butyl, *n*-pentyl, isopentyl, neopentyl, *n*-hexyl, 3-methylhexyl, 2,2-dimethylpentyl, 2,3-dimethylpentyl, *n*-heptyl.

Moreover, the term "alkyl" includes both "unsubstituted alkyls" and "substituted alkyls." Representative examples of substituents for alkyl moieties are hydroxy, halogen, cyano, nitro, cycloalkyl, alkenyl, alkynyl, alkoxy, alkenyloxy, alkynyloxy, halogen or amino (including alkyl amino, di-alkylamino, arylamino, di-arylamino).

As used herein, the term "alkoxy" includes alkyl-O-, wherein alkyl is defined herein above. Representative examples of alkoxy moieties include, but are not limited to, methoxy, ethoxy, propoxy, 2-propoxy, butoxy, *tert*-butoxy, pentyloxy, hexyloxy, cyclopropyloxy-, cyclohexyloxy- and the like. In some embodiments, the alkoxy groups have about 1-7 carbons, for example 1-4 carbons. The term alkoxy includes substituted alkoxy. Examples of substituted alkoxy groups include halogenated alkoxy groups. Examples of halogen substituted alkoxy groups are fluoromethoxy, difluoromethoxy, trifluoromethoxy, chloromethoxy, dichloromethoxy, and trichloromethoxy.

The term "alkoxyalkyl" includes alkyl groups, as defined above, in which the alkyl group is substituted with alkoxy. Moreover, the term "alkoxyalkyl" includes both "unsubstituted alkoxyalkyl" and "substituted alkoxyalkyl." Representative examples of substituents for alkoxyalkyl moieties include, but are not limited to, hydroxy, halogen, cyano, nitro, alkyl, cycloalkyl, alkenyl, akynyl, alkoxy, alkenyloxy, alkynyloxy, halogen or amino (including alkyl amino, di-alkylamino, arylamino, di-arylamino).

The term "alkenyl" includes branched or unbranched hydrocarbons having at least one carbon-carbon double bond. Representative examples of alkenyl moieties include, but are not limited to, vinyl, prop-1-enyl, allyl, butenyl, isopropenyl or isobutenyl. Moreover, the term "alkenyl" includes both "unsubstituted alkenyls" and "substituted alkenyls." Representative examples of substituents for alkenyl moieties include, but are not limited to, hydroxy, halogen, cyano, nitro, alkyl, cycloalkyl, alkenyl, akynyl, alkoxy, alkenyloxy, alkynyloxy, halogen or amino (including alkyl amino, di-alkylamino, arylamino, di-arylamino).

The term "alkynyl" includes branched or unbranched hydrocarbons having at least one carbon-carbon triple bond. Representative examples of alkynyl moieties include, but are not limited to, ethynyl, prop-1-ynyl (propargyl), butynyl, isopropynyl or isobutynyl. Moreover, the term "alkynyl" includes both "unsubstituted alkynyls" and "substituted alkynyls." Representative examples of substitutents for alkynyl moieties include, but are not limited to, hydroxy, halogen, cyano, nitro, alkyl, cycloalkyl, alkenyl, akynyl, alkoxy, alkenyloxy, alkynyloxy, halogen or amino (including alkyl amino, di-alkylamino, arylamino, di-arylamino).

As used herein, the term "cycloalkyl" includes saturated or unsaturated monocyclic, bicyclic or tricyclic hydrocarbon groups of 3-12 carbon atoms, for example, 3-8, or 3-7 carbon atoms. Exemplary monocyclic hydrocarbon groups include, for example, cyclopropyl, cyclobutyl, cyclopentyl, cyclopentenyl, cyclohexyl and cyclohexenyl. Exemplary bicyclic hydrocarbon groups include, for example, bornyl, indyl, hexahydroindyl, tetrahydronaphthyl, decahydronaphthyl, bicyclo[2.1.1]hexyl, bicyclo[2.2.1]heptyl, bicyclo[2.2.1]heptenyl, 6,6-dimethylbicyclo[3.1.1]heptyl, and 2,6,6-trimethylbicyclo[3.1.1]heptyl, bicyclo[2.2.2]octyl. An example of a tricyclic hydrocarbon group includes, for example, adamantyl.

The term "cycloalkyl" includes both "unsubstituted cycloalkyl" and "substituted cycloalkyl." Representative examples of substitutents for cycloalkyl moieties include, but are not limited to, hydroxy, halogen, cyano, nitro, alkyl, cycloalkyl, alkenyl, akynyl, alkoxy, alkenyloxy, alkynyloxy, halogen or amino (including alkyl amino, di-alkylamino, arylamino, di-arylamino).

The term "aryl" includes monocyclic or bicyclic aromatic hydrocarbon groups having 6-20 carbon atoms in the ring portion. Representative examples of aryl moieties include, but are not limited to, phenyl, naphthyl, anthracyl, phenanthryl or tetrahydronaphthyl. Moreover, the term aryl includes both "unsubstituted aryl" and "substituted aryl." Representative examples of substitutents for aryl moieties include, but are not limited to, hydroxy, halogen, cyano, nitro, alkyl, cycloalkyl, alkenyl, akynyl, alkoxy, alkenyloxy, alkynyloxy, halogen or amino (including alkyl amino, di-alkylamino, arylamino, di-arylamino).

The term "heteroaryl" includes monocyclic or bicyclic heteroaryl moieties, containing from 5-10 ring members selected from carbon atoms and 1 to 5 heteroatoms, selected from O, N or S. Examples of heteroaryl groups include, but are not limited to, thienyl, furyl, pyrrolyl, imidazolyl, pyrazolyl, thiazolyl, isothiazolyl, oxa-2,3-diazolyl, oxa-2,4-diazolyl, oxa-2,5-diazolyl, oxa-3,4-diazolyl, thia-2,3-diazolyl, thia-2,4-diazolyl, thia-2,5-diazolyl, thia-3,4-diazolyl, 3-, 4-, or 5-isothiazolyl, 2-, 4-, or 5-oxazolyl, 3-, 4-, or 5-isoxazolyl, 3- or 5-1,2,4-triazolyl, 4- or 5-1,2, 3-triazolyl, tetrazolyl, 2-, 3-, or 4-pyridyl, 3- or 4-pyridazinyl, 3-, 4-, or 5-pyrazinyl, 2-pyrazinyl, 2-,4-, or 5-pyrimidinyl. A heteroaryl group may be mono-, bi-, tri-, or polycyclic.

The term "heteroaryl" further includes groups in which a heteroaromatic ring is fused to one or more aryl, cycloaliphatic, or heterocyclyl rings, where the radical or point of attachment is on the heteroaromatic ring or on the fused aryl ring. Representative examples of such heteroaryl moieties include, but are not limited to, indolyl, isoindolyl, indazolyl, indolizinyl, purinyl, quinolizinyl, quinolinyl, isoquinolinyl, cinnolinyl, phthalazinyl, naphthyridinyl, quinazolinyl, quinaxalinyl, phenanthridinyl, phenathrolinyl, phenazinyl, phenothiazinyl, phenoxazinyl, benzisoqinolinyl, thieno[2,3-b]furanyl, furo[3,2-b]-pyranyl, 5H-pyrido[2,3-d]-o-oxazinyl, 1H-pyrazolo[4,3-d]-oxazolyl, 4H-imidazo[4,5-d] thiazolyl, pyrazino[2,3-d]pyridazinyl, imidazo[2,1-b] thiazolyl, imidazo[1,2-b][1,2,4]triazinyl, 7-benzo[b]thienyl, benzoxazolyl, benzimidazolyl, benzothiazolyl, benzoxapinyl, benzoxazinyl, 1H-pyrrolo[1,2-b][2]benzazapinyl, benzofuryl, benzothiophenyl, benzotriazolyl, pyrrolo[2,3-b]pyridinyl, pyrrolo[3,2-c]pyridinyl, pyrrolo[3,2-c]pyridinyl, pyrrolo[3,2-b]pyridinyl, imidazo[4,5-b]pyridinyl, imidazo[4,5-c]pyridinyl, pyrazolo[4,3-d]pyridinyl, pyrazolo[4,3-c]pyridinyl, pyrazolo[3,4-c]pyridinyl, pyrazolo[3,4-d]pyridinyl, pyrazolo[3,4-b]pyridinyl, imidazo[1,2-a]pyridinyl, pyrazolo[1,5-a]pyridinyl, pyrrolo[1,2-b]pyridazinyl, imidazo[1,2-c]pyrimidinyl, pyrido[3,2-d]pyrimidinyl, pyrido[4,3-d]pyrimidinyl, pyrido[3,4-d]pyrimidinyl, pyrido[2,3-d]pyrimidinyl, pyrido[2,3-b]pyrazinyl, pyrido[3,4-b]pyrazinyl, pyrimido[5,4-d]pyrimidinyl, pyrazino[2,3-b]pyrazinyl, or pyrimido[4,5-d]pyrimidinyl. Moreover, the term "heteroaryl" includes both "unsubstituted heteroaryl" and "substituted heteroaryl."

The aromatic ring of an "aryl" or "heteroaryl" group can be unsubstituted or substituted at one or more ring positions with substituents including, for example, halogen, hydroxy, cyano, nitro, alkyl, cycloalkyl, alkenyl, akynyl, aryl, heteroaryl, heterocyclyl, alkoxy, cycloalkyloxy, alkenyloxy, alkynyloxy, aryloxy, heteroaryloxy, heterocyclyloxy, arylalkyloxy, heteroarylalkyloxy, heterocyclylalkyloxy, ketones (including alkylcarbonyl, cycloalkylcarbonyl, alkenylcarbonyl, alkynylcarbonyl, aroyl, arylalkylcarbonyl, heteroarylcarbonyl, heterocyclylcarbonyl), esters (including alkoxycarbonyl, cycloalkyloxycarbonyl, aryloxycarbonyl, heteroaryloxycarbonyl, heterocyclyloxycarbonyl, alkylcarbonyloxy, cycloakylcarbonyloxy, arylcarbonyloxy, heteroarylcarbonyloxy, heterocyclylcarbonyloxy), carbonates (including alkoxycarbonyloxy, aryloxycarbonyloxy, heteroaryloxycarbonyloxy), carbamates (including alkoxycarboxylamino, aryloxycarbonylamino, alkenyloxycarbonylamino, alkynyloxycarbonylamino, aryloxycarbonylamino, aminocarbonyloxy, alkylaminocarbonyloxy, di-alkylaminocarbonyloxy, arylaminocarbonyloxy), carbamoyl (including alkylaminoacarbonyl, di-alkylaminocarbonyl, arylaminocarbonyl, arylalkylaminocarbonyl, alkenylaminocarbonyl), amido (including alkylcarbonylamino, alkylcarbonylalkylamino, arylcarbonylamino, heteroarylcarbonylamino), arylalkyl, heteroarylalkyl, heterocycloalkyl, amino (including alkyl amino, di-alkylamino, arylamino, di-arylamino, and alkylarylamino), sulfonyl (including alkylsulfonyl, arylsulfonyl, arylalkylsufonyl, heteroarylsulfonyl, alkoxysulfonyl, aryloxysulfonyl, heteroaryloxysulfonyl, cycloalkylsulfonyl, heterocyclylsulfonyl), sulfamoyl, sulfonamido, phosphate, phosphonato, phosphinato, thioether (including alkylthio, arylthio, heteroarylthio), ureido, imino, amidino, thiocarboxyl (including alkylthiocarbonyl, arylthiocarbonyl), sulfinyl (including alkylsulfinyl, arylsulfinyl), carboxyl, wherein each of the afore-mentioned hydrocarbon groups may be optionally substituted with one or more alkyl, alkenyl, alkynyl, cycloalkyl, halogen, hydroxy or alkoxy groups.

As used herein, the term "heterocyclyl" or "heterocyclo" includes unsubstituted or substituted, saturated or unsaturated non-aromatic ring or ring systems, e.g., which is a 4-, 5-, 6-, or 7-membered monocyclic, 7-, 8-, 9-, 10-, 11-, or 12-membered bicyclic or 10-, 11-, 12-, 13-, 14- or 15-membered tricyclic ring system and contains at least one heteroatom selected from O, S and N, where the N and S can also optionally be oxidized to various oxidation states. In one embodiment, heterocyclyl moiety represents a saturated monocyclic ring containing from 5-7 ring atoms and optionally containing a further heteroatom, selected from O, S or N. The heterocyclic group can be attached at a heteroatom or a carbon atom. The heterocyclyl can include fused or bridged rings as well as spirocyclic rings. Examples of heterocyclyl moieties include, for example, dihydrofuranyl, dioxolanyl, dioxanyl, dithianyl, piperazinyl, pyrrolidine, dihydropyranyl, oxathiolanyl, dithiolane, oxathianyl, thiomorpholino, oxiranyl, aziridinyl, oxetanyl, oxepanyl, azetidinyl, tetrahydrofuranyl, tetrahydrothiophenyl, pyrrolidinyl, tetrahydropyranyl, piperidinyl, morpholino, piperazinyl, azepinyl, oxapinyl, oxaazepanyl, oxathianyl, thiepanyl, azepanyl, dioxepanyl, and diazepanyl.

The term "heterocyclyl" includes heterocyclic groups as defined herein that may be substituted with 1, 2 or 3 substituents such as =O, =S, halogen, hydroxy, cyano, nitro, alkyl, cycloalkyl, alkenyl, akynyl, aryl, heteroaryl, heterocyclyl, alkoxy, cycloalkyloxy, alkenyloxy, alkynyloxy, aryloxy, heteroaryloxy, heterocyclyloxy, arylalkyloxy, heteroarylalkyloxy, heterocyclylalkyloxy, ketones (including alkylcarbonyl, cycloalkylcarbonyl, alkenylcarbonyl, alkynylcarbonyl, aroyl, arylalkylcarbonyl, heteroarylcarbonyl, heterocyclylcarbonyl), esters (including alkoxycarbonyl, cycloalkyloxycarbonyl, aryloxycarbonyl, heteroaryloxycarbonyl, heterocyclyloxycarbonyl, alkylcarbonyloxy, cycloakylcarbonyloxy, arylcarbonyloxy, heteroarylcarbonyloxy, heterocyclylcarbonyloxy), carbonates (including alkoxycarbonyloxy, aryloxycarbonyloxy, heteroaryloxycarbonyloxy), carbamates (including alkoxycarboxylamino, aryloxycarbonylamino, alkenyloxycarbonylamino, alkynyloxycarbonylamino, aryloxycarbonylamino, aminocarbonyloxy, alkylaminocarbonyloxy, dialkylaminocarbonyloxy, arylaminocarbonyloxy), carbamoyl (including alkylaminoacarbonyl, dialkylaminocarbonyl, arylaminocarbonyl, arylakylaminocarbonyl, alkenylaminocarbonyl), amido (including alkylcarbonylamino, alkylcarbonylalkylamino, arylcarbonylamino, heteroarylcarbonylamino), arylalkyl, heteroarylalkyl, heterocyclylalkyl, amino (including alkyl amino, dialkylamino, arylamino, diarylamino, and alkylarylamino),sulfonyl (including alkylsulfonyl, arylsulfonyl, arylalkylsufonyl, heteroarylsulfonyl, alkoxysulfonyl, aryloxysulfonyl, heteroaryloxysulfonyl, cycloakylsulfonyl, heterocyclylsulfonyl), sulfamoyl, sulfonamido, phosphate, phosphonato, phosphinato, thioether (including alkylthio, arylthio, heteroarylthio), ureido, imino, amidino, thiocarboxyl (including alkylthiocarbonyl, arylthiocarbonyl), sulfinyl (including alkylsulfinyl, arylsulfinyl), carboxyl wherein each of the afore-mentioned hydrocarbon groups may be optionally substituted with one or more alkyl, alkenyl, alkynyl, cycloalkyl, halogen, hydroxy or alkoxy groups.

The term "heterocyclylalkyl" is an alkyl substituted with heterocyclyl. The term includes unsubstituted and substituted heterocyclylalkyl moieties which may be substituted with one or more alkyl, alkenyl, alkynyl, cycloalkyl, halogen, hydroxy or alkoxy groups.

The term "carbonyl" or "carboxy" includes compounds and moieties which contain a carbon connected with a double bond to an oxygen atom (C=O). The carbonyl can be further substituted with any moiety which allows the compounds of the invention to perform its intended function. For example, carbonyl moieties may be substituted with alkyls, alkenyls, alkynyls, aryls, alkoxy, aminos, *etc.* Examples of moieties which contain a carbonyl include aldehydes, ketones, carboxylic acids, amides, esters, urea, anhydrides, *etc.*

The term "hydroxy" or "hydroxyl" includes groups with an -OH or -O⁻.

The term "halogen" includes fluorine, bromine, chlorine, iodine, *etc.*

The term "perhalogenated" includes moieties in which all hydrogens are replaced by halogen atoms.

The vitamin D compounds of the invention, or their pharmaceutically acceptable salts, solvates or prodrugs thereof, may contain one or more asymmetric centers and may thus give rise to enantiomers, diastereomers, and other stereoisomeric forms that may be defined, in terms of absolute stereochemistry, as (*R*)- or (*S*)- or, as (D)- or (L)- for amino acids. The present invention is meant to include all such possible isomers, as well as their racemic and optically pure forms. Optically active (+) and (-), (*R*)- and (*S*)-, or (D)- and (L)- isomers may be prepared using chiral synthons or chiral reagents, or resolved using conventional techniques, such as HPLC using a chiral column. When the compounds described herein contain olefinic double bonds or other centers of geometric asymmetry, and unless specified otherwise, it is intended that the compounds include both *E* and *Z* geometric isomers. Likewise, all tautomeric forms are also intended to be included.

The term "stereoisomer" includes compounds made up of the same atoms bonded by the same bonds but having different three-dimensional structures, which are not interchangeable. The present invention contemplates various stereoisomers and mixtures thereof and includes enantiomers, which refers to two stereoisomers whose molecules are nonsuperimposeable mirror images of one another.

The present invention includes all pharmaceutically acceptable isotopically-labeled vitamin D compounds in which one or more atoms are replaced by atoms having the same atomic number, but an atomic mass or mass number different from the atomic mass or mass number usually found in nature.

Examples of isotopes suitable for inclusion in the compounds of the invention comprises isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulphur, such as ³⁵S. Substitution with heavier isotopes such as deuterium, *i.e.*, ²H, may afford certain therapeutic advantages resulting from greater metabolic stability, for example, increased *in vivo* half-life or reduced dosage requirements. Isotopically-labeled vitamin D compounds can generally be prepared by conventional techniques known to those skilled in the art using an appropriate isotopically-labeled reagent in place of the non-labeled reagent previously employed.

The term "prodrugs" includes compounds that may be converted under physiological conditions or by solvolysis to a biologically active compound of the invention. Thus, the term "prodrug" refers to a metabolic precursor of a compound of the invention that is pharmaceutically acceptable. A prodrug may be inactive when administered to a subject in need thereof, but is converted in vivo to an active compound of the invention. Prodrugs are typically rapidly transformed in vivo to yield the parent compound of the invention, for example, by hydrolysis in blood or conversion in the gut or liver. The prodrug compound often offers advantages of solubility, tissue compatibility or delayed release in a mammalian organism (see, Bundgard, H., Design of Prodrugs (1985), pp. 7-9, 21-24 (Elsevier, Amsterdam)).

A discussion of prodrugs is provided in Higuchi, T., et al., "Pro-drugs as Novel Delivery Systems," A.C.S. Symposium Series, Vol. 14, and in Bioreversible Carriers in Drug Design, ed. Edward B. Roche, Anglican Pharmaceutical Association arid Pergamon Press, 1987.

"Pharmaceutically acceptable salt" includes both acid and base addition salts. "Pharmaceutically acceptable acid addition salt" refers to those salts which retain the biological effectiveness and properties of the free bases, which are not biologically or otherwise undesirable, and which are formed with inorganic acids such as, but not limited to, hydrochloric acid, hydrobromic acid, sulfuric acid, nitric acid, phosphoric acid and the like, and organic acids such as, but not limited to, acetic acid, 2,2-dichloroacetic acid, adipic acid, alginic acid, ascorbic acid, aspartic acid, benzenesulfonic acid, benzoic acid, 4-acetamidobenzoic acid, camphoric acid, camphor-10-sulfonic acid, capric acid, caproic acid, caprylic acid, carbonic acid, cinnamic acid, citric acid, cyclamic acid, dodecylsulfuric acid, ethane-1,2-disulfonic acid, ethanesulfonic acid, 2-hydroxyethanesulfonic acid, formic acid, fumaric acid, galactaric acid, gentisic acid, glucoheptonic acid, gluconic acid, glucuronic acid, glutamic acid, glutaric acid, 2-oxo-glutaric acid, glycerophosphorirc acid, glycolic acid, hippuric acid, isobutyric acid, lactic acid, lactobionic acid, lauric acid, maleic acid, malic acid, malonic acid, mandelic acid, methanesulfonic acid, mucic acid, naphthalene-1,5-disulfonic acid, naphthalene-2-sulfonic acid, 1-hydroxy-2-naphthoic acid, nicotinic acid, oleic acid, orotic acid, oxalic acid, palmitic acid, pamoic acid, propionic acid, pyroglutamic acid, pyruvic acid, salicylic acid, 4-aminosalicylic acid, sebacic acid, stearic acid, succinic acid, tartaric acid, thiocyanic acid, p-toluenesulfonic acid, trifluoroacetic acid, undecylenic acid, and the like.

"Pharmaceutically acceptable base addition salt" refers to those salts which retain the biological effectiveness and properties of the free acids, which are not biologically or otherwise undesirable. These salts are prepared from addition of an inorganic base or an organic base to the free acid. Salts derived from inorganic bases include, but are not limited to, the sodium, potassium, lithium, ammonium, calcium, magnesium, iron, zinc, copper, manganese, aluminum salts and the like. Preferred inorganic salts are the ammonium, sodium, potassium, calcium, and magnesium salts. Salts derived from organic bases include, but are not limited to, salts of primary, secondary, and tertiary amines, substituted amines including naturally occurring substituted amines, cyclic amines and basic ion exchange resins, such as ammonia, isopropylamine, trimethylamine, diethylamine, triethylamine, tripropylamine, diethanolamine, ethanolamine, deanol, 2-dimethylaminoethanol, 2-diethylaminoethanol, dicyclohexylamine, lysine, arginine, histidine, caffeine, procaine, hydrabamine, choline, betaine, benethamine, benzathine, ethylenediamine, glucosamine, methylglucamine, theobromine, triethanolamine, tromethamine, purines, piperazine, piperidine, N-ethylpiperidine, polyamine resins and the like.

Often crystallizations produce a solvate of the compound of the invention. As used herein, the term "solvate" refers to an aggregate that comprises one or more molecules of a compound of the invention with one or more molecules of solvent. The solvent may be water, in which case the solvate may be a hydrate. Alternatively, the solvent may be an organic solvent. Thus, the compounds of the present invention may exist as a hydrate, including a monohydrate, dihydrate, hemihydrate, sesquihydrate, trihydrate, tetrahydrate and the like, as well as the corresponding solvated forms. The compound of the invention may be true solvates, while in other cases, the compound of the invention may merely retain adventitious water or be a mixture of water plus some adventitious solvent.

The invention provides a pharmaceutical composition comprising a therapeutically effective amount of a vitamin D compound for use in a method of preventing or mitigating chemotherapy induced alopecia.

The language "pharmaceutical composition" includes formulations of a compound of the invention (*e.g*., a vitamin D compound) and a medium generally accepted in the art, for delivery of the vitamin D compound to an individual. Such a medium includes all pharmaceutically acceptable carriers, diluents or excipients thereof.

In some embodiments, the compositions of the invention can be topically administered to any epithelial surface. An "epithelial surface" include an area of tissue that covers external surfaces of a body, or which lines hollow structures including, but not limited to, cutaneous and mucosal surfaces. Such epithelial surfaces include oral, pharyngeal, esophageal, pulmonary, ocular, aural, nasal, buccal, lingual, vaginal, cervical, genitourinary, alimentary, and anorectal surfaces.

Compositions can be formulated in a variety of conventional forms employed for topical administration. These include, for example, semi-solid and liquid dosage forms, such as liquid solutions or suspensions, gels, creams, emulsions, lotions, slurries, powders, sprays, foams, pastes, ointments, salves, balms, or drops. According to the present invention, the pharmaceutical composition for use is formulated such that it can be applied by a metered spray.

Conventionally used carriers for topical applications include pectin, gelatin and derivatives thereof, polylactic acid or polyglycolic acid polymers or copolymers thereof, cellulose derivatives such as methyl cellulose, carboxymethyl cellulose, or oxidized cellulose, guar gum, acacia gum, karaya gum, tragacanth gum, bentonite, agar, carbomer, bladderwrack, ceratonia, dextran and derivatives thereof, ghatti gum, hectorite, ispaghula husk, polyvinypyrrolidone, silica and derivatives thereof, xanthan gum, kaolin, talc, starch and derivatives thereof, paraffin, water, vegetable and animal oils, polyethylene, polyethylene oxide, polyethylene glycol, polypropylene glycol, glycerol, ethanol, propanol, propylene glycol (glycols, alcohols), fixed oils, sodium, potassium, aluminum, magnesium or calcium salts (such as chloride, carbonate, bicarbonate, citrate, gluconate, lactate, acetate, gluceptate or tartrate).

Standard composition strategies for topical agents can be applied to the vitamin D compounds in order to enhance the persistence and residence time of the drug, and to improve the prophylactic efficacy achieved.

The spray can contain, in addition to the vitamin D compounds, carriers such as lactose, talc, aluminum hydroxide, calcium silicates and polyamide powder, or mixtures of these substances. The Spray can additionally contain customary propellants, such as chlorofluorohydrocarbons and volatile unsubstituted hydrocarbons, such as butane and propane. Sprays can be stored, sold, and/or administered through use of a metered spray bottle.

Ordinarily, an aqueous aerosol is made by formulating an aqueous solution or suspension of the vitamin D compounds together with conventional pharmaceutically acceptable carriers and stabilizers. The carriers and stabilizers vary with the requirements of the particular compound, but typically include nonionic surfactants (e.g., Tweens, Pluronics, polyethylene glycol and the like), proteins like serum albumin, sorbitan esters, oleic acid, lecithin, amino acids such as glycine, buffers, salts, sugars or sugar alcohols. Aerosols generally are prepared from isotonic solutions. Generation of the aerosol or any other means of delivery of the present invention may be accomplished by any of the methods known in the art. For example, in the case of aerosol delivery, the compound is supplied in a finely divided form along with any suitable carrier with a propellant.

Liquefied propellants are typically gases at ambient conditions and are condensed under pressure. The propellant may be any acceptable and known in the art including propane and butane, or other lower alkanes, such as those of up to 5 carbons. The composition is held within a container with an appropriate propellant and valve, and maintained at elevated pressure until released by action of the valve.

In one embodiment, the vitamin D compound may be administered prophylactically. For prophylactic applications, the vitamin D compound can be applied prior to potential alopecia. The timing of application can be optimized to maximize the prophylactic effectiveness of the vitamin D compound. The timing of application will vary depending on the mode of administration, doses, the stability and effectiveness of composition, the frequency of the dosage, *e.g*., single application or multiple dosage. One skilled in the art will be able to determine the most appropriate time interval required to maximize prophylactic effectiveness of the vitamin D compound.

The vitamin D compound when present in a composition will generally be present in an amount from about 0.000001% to about 100%, more preferably from about 0.001% to about 50%, and most preferably from about 0.01% to about 25% of total weight.

For compositions of the present invention comprising a carrier, the composition comprises, for example, from about 1% to about 99%, preferably from about 50% to about 99%, and most preferably from about 75% to about 99% by weight of at least one carrier.

Also, the separate components of the compositions of the invention may be preblended or each component may be added separately to the same environment according to a predetermined dosage for the purpose of achieving the desired concentration level of the treatment components and so long as the components eventually come into intimate admixture with each other. Further, the present invention may be administered or delivered on a continuous or intermittent basis.

In some embodiments, the pharmaceutical composition is not in a water based formulation.

In some embodiments, the pharmaceutical composition includes the vitamin D compound in a vehicle of about 40% (w/w) propylene glycol and about 60% (w/w) anhydrous ethanol.

In some embodiments, the pharmaceutical composition includes the vitamin D compound in a vehicle of about 40% (w/w) propylene glycol and about 60% (w/w) anhydrous absolute ethanol (200 proof, U.S.); or about 30% (w/w) propylene glycol, about 10% (w/w) ethoxydiglycol or transcutol, and about 60% (w/w) anhydrous absolute ethanol (200 proof, U.S.).

In one embodiment, the formulation includes the vitamin D active ingredient, formulated in about 40% (w/w) propylene glycol and about 60% (w/w) anhydrous absolute ethanol (200 proof, US), optionally with other minor pharmaceutically acceptable excipients, carriers, or diluents, such as about 0.4% (w/v) of Phospholipon 90G. In another embodiment, the formulation includes the vitamin D active ingredient, formulated in about 30% (w/w) propylene glycol, about 10% (w/w) Ethoxydiglycol or Transcutol, and about 60% (w/w) anhydrous absolute ethanol (200 proof, US), optionally with other minor pharmaceutically acceptable excipients, carriers, or diluents, such as about 0.4% (w/v) of Phospholipon 90G. In some embodiments, the ethanol is anhydrous absolute 200 proof (U.S.) undenatured ethanol (USP). The formulation described herein provides a level of dermal penetration and delivery of the active vitamin D compounds, and provides an effective means to prevent alopecia, or to reduce the severity of alopecia, especially chemotherapy-induced alopecia (CIA).

In certain embodiments, the pharmaceutical composition comprises about 40% (w/w) propylene glycol (USP grade) and about 60% (w/w) anhydrous absolute ethanol (200 proof, US), undenatured USP.

In some embodiments, the pharmaceutical composition comprises about 40% (w/w) propylene glycol (*e.g.*, USP grade or better), and about 60% (w/w) anhydrous absolute ethanol (200 proof, US), undenatured (*e.g*., USP grade or better).

In other embodiments, the pharmaceutical composition comprises about 30% (w/w) propylene glycol, about 10% (w/w) Ethoxydiglycol or Transcutol, and about 60% (w/w) anhydrous absolute ethanol (200 proof, U.S.).

In yet other embodiments, the pharmaceutical composition comprises about 0.1, 0.2, 0.3, 0.4, 0.5, 0.6, 0.7, 0.8, 0.9, or 1% of Phospholipon, such as Phospholipon 90G.

In other embodiments, the precise percentage (w/w) of propylene glycol and/or anhydrous absolute ethanol may be varied based on the 40%:60% ratio. For example, the % ratio of propylene glycol to anhydrous absolute ethanol may be 20: 80; 25: 75; 30:70; 35:65; 36:64; 37:63; 38:62; 39:61; 41:59; 42:58; 43:57; 44:56; 45:55, etc. The effectiveness of such other formulations may be verified using and art recognized the techniques, such as the procedure described in Example I.

In certain embodiments, the anhydrous absolute ethanol in the formulation may be replaced with 95% ethanol, 96% ethanol, 97% ethanol, 98% ethanol, or 99% ethanol.

In certain embodiments, the pharmaceutical composition may also include trace amount of other inactive ingredients, excipients, or components. The presence of such ingredients does not substantially affect the effectiveness of the vitamin D compounds or its dermal penetration/accumulation behavior.

The vitamin D compounds of the invention are formulated for delivering to epidermis while having substantially no penetration of the dermis layer. A previous different formulation developed by Roche Dermatology was ineffective in protecting against CIA when used at a dose of about 500 - 1000 µg per application, and caused dermatitis in the majority of the human subjects in Phase I study. The same Roche formulation also failed to work in the rat chloroleukemic model (infra).

One of the exemplary formulations of the invention can be prepared according to the following (non-limiting) procedure:

### Formula II: Calcitriol at 5, 10, and 20 µg/g

Formula II is prepared as per the protocol set forth in Example 17, section *4.0 Therapeutic*/*Diagnostic Agents*, below.

### Formula III: Calcitriol at 1.2 µg/g (1.2 ppm)

| **Ingredient** | **% w/w** |
|---|---|
| 100 ppm Calcitriol concentrate | 1.2 |
| 200 Proof Ethanol | 58.8 |
| Propylene Glycol | 40 |
| Total | 100 |

Formula III is prepared as follows: the calcitriol is dissolved in the ethanol; the propylene glycol is then added and mixed until the resulting solution is clear and uniform in appearance. The specific gravity of the above formulation is approximately 0.875 g/mL. The target concentration of the above formula expressed in w/v is 1.05 µg/mL.

### Formula IV: Calcitriol at 3.6 µg/g (3.6 ppm)

| **Ingredient** | **% w/w** |
|---|---|
| 100 ppm Calcitriol concentrate | 3.6 |
| 200 Proof Ethanol | 56.4 |
| Propylene Glycol | 40 |
| Total | 100 |

Formula IV is prepared as follows: the calcitriol is dissolved in the ethanol; the propylene glycol is then added and mixed until the resulting solution is clear and uniform in appearance. The specific gravity of the above formulation is approximately 0.875 g/mL. The target concentration of the above formula expressed in w/v is 3.15 µg/mL.

The reagents used are all USP Grade reagents (meeting the requirements of the U.S. Pharmacopeia).

Using the formulation of the invention, a dosage of about 0.2 µg (administered as 100 µL of 2 µg/mL topical solution) is protective against CIA in neonatal rat. Based on this information, one of skill in the art can readily adjust the proper dosage level based on the average body weight of the mammal to be treated. For example, in human subjects, a total dose of calcitriol (or other equivalent amount of vitamin D compounds) of about 5 µg, 10 µg, 20 µg, 25 µg, 40 µg, 50 µg, 60 µg, 70 µg, 75 µg, 80 µg, 90 µg, or 100 µg may be used. In one embodiment, the total dose of calcitriol is about 11-24 µg, 26-49 µg, 51-74 µg, or 76-99 µg. In an alternative embodiment, the total dose of calcitriol is about 15-25, 16-24 µg, 17-23 µg, 18-22 µg, 19-21 µg, 31-49 µg, 32-48 µg, 33-47 µg, 34-46 µg, 35-45 µg, 36-44 µg, 37-43 µg, 38-42 µg, 39-41 µg, 51-69 µg, 52-68 µg, 53-67 µg, 54-66 µg, 55-65 µg, 56-64 µg, 57-63 µg, 58-62 µg, 59-61 µg, 65-74 µg, 66-73 µg, 67-72 µg, 68-71 µg, 69-70 µg, 76-85 µg, 77-84 µg, 78-83 µg, 79-82 µg, 80-81 µg, 81-99 µg, 82-98 µg, 83-97 µg, 84-96 µg, 85-95 µg, 86-94 µg, 87-93 µg, 88-92 µg, or 89-91 µg. Preliminary animal toxicology study shows that a dose of about 100 µg caused no dermal irritation, and exhibited excellent epidermal penetration without substantial dermal penetration (*e.g.*, extremely low penetration to dermis). See description above for additional dosage information.

In one aspect, the invention provides a kit for use in a method for preventing or mitigating chemotherapy induced alopecia, the kit comprising the pharmaceutical composition for use of the present invention and adapted for topical administration; and instructions for carrying out a method for preventing or mitigating chemotherapy induced alopecia. Instructions can be provided in written or oral format, for example by a health care provider or commercial entity on paper or through electronic media. Instructions can include approved (*e.g*., FDA or other regulatory agency approve) reference or prescribing information, and highlights or summaries thereof. Instructions can also include monographs, textbooks and textbook chapters /sections, pamphlets, drug labels and label supplements, clinical study protocols, including those pertaining to both on and off label use.

### Examples

The following examples illustrate certain aspects of the invention and are not limiting in any respect. Just as various aspects of the invention can include any one or more of the embodiments and features provided above, they can also incorporate any one or more of the embodiments and features provided in the examples below.

### Example 1. Evaluation of the Percutaneous Absorption of Calcitriol, in vitro, Using the Franz Human Skin Finite Dose Model

This example was designed to evaluate the percutaneous absorption pharmacokinetics of various calcitriol formulations. Absorption was measured in human cadaver skin, *in vitro*, using the finite dose technique and Franz Diffusion Cells.
The *in vitro* human cadaver skin model has proven to be a valuable tool for the study of percutaneous absorption and the determination of the pharmacokinetics of topically applied drugs. The model used human cadaver skin mounted in specially designed diffusion cells that allowed the skin to be maintained at a temperature and humidity that match typical *in vivo* conditions. A finite dose (*e.g.*, 4-7 mg/cm²) of formulation was applied to the outer surface of the skin and drug absorption was measured by monitoring its rate of appearance in the receptor solution bathing the inner surface of the skin. Data defining total absorption, rate of absorption, as well as skin content was then accurately determined in this model. The method has historic precedent for accurately predicting in vivo percutaneous absorption kinetics. Thus, the *in vitro* finite dose model on human skin permitted the characterization of the percutaneous absorption pharmacokinetics of vitamin D compounds, such as calcitriol.

In this experiment, six formulations containing calcitriol were tested on three replicate skin sections per formulation on each of three different cadaver skin donors, for the percutaneous absorption of calcitriol over a 48 hour dose period. At pre-selected times after dose application, the dermal receptor solution was removed in its entirety, replaced with fresh receptor solution, and an aliquot saved for subsequent analysis. In addition, the stratum corneum, epidermis, and dermis were recovered and evaluated for drug content. The samples were analyzed for calcitriol content by High Performance Liquid Chromatography (HPLC). A brief description of the protocol used herein is provided below.

Human cadaver trunk skin without obvious signs of skin disease, obtained within 24 - 48 hours of death, was used in this study. The skin was dermatomed, cryopreserved, and sealed in a water-impermeable plastic bag, and stored at < -70°C until the day of the experiment. Prior to use, the skin was thawed in ~37°C water, then rinsed in tap water to remove any adherent blood or other material from the surface. Skin from a single donor was cut into multiple smaller sections large enough to fit on static 2.0 cm² Franz diffusion cells. Three replicates per donor were tested for each formulation. The dermal chamber was filled to capacity with a reservoir solution of phosphate-buffered isotonic saline (PBS), pH 7.4 ± 0.1, and the epidermal chamber was left open to ambient laboratory environment. Volpo (Oleth-20), a non-ionic surfactant known to increase the aqueous solubility of poorly water soluble compounds, may be added to PBS. Volpo in the reservoir solution insures diffusion sink conditions during percutaneous absorption, and is known not to affect the barrier properties of the test skin. The cells were then placed in a diffusion apparatus in which the dermal reservoir solution was stirred magnetically at ~600 RPM and its temperature maintained to achieve a skin surface temperature of 32.0 ± 1.0°C.

To assure the integrity of each skin section, its permeability to tritiated water was determined before application of the test products. Following a brief (0.5-1 hour) equilibrium period, ³H₂O (NEN, Boston, MA, sp. Act. - 0.5 µCi/mL) was layered across the top of the skin by dropper so that the entire exposed surface was covered (approximately 250 - 500 µL). After 5 minutes, the ³H₂O aqueous layer was removed. At 30 minutes, the reservoir solution was collected and analyzed for radioactive content by liquid scintillation counting. Skin specimens in which absorption of ³H₂O is less than 1.56 µL-equ/cm² are considered acceptable. All skin samples used had ³H₂O absorption of less than about 0.50 µL-equ/cm² (results not shown).

*Dose Administration and Sample Collection:* Just prior to dosing, a pre-dose sample was taken and the reservoir solution was replaced with a fresh solution of 0.1 × PBS with 0.2% Volpo (also known as Oleth-20, a non-ionic surfactant used to ensure miscibility of the drug in an aqueous solution). The chimney was removed from the Franz Cell to allow full access to the epidermal surface of the skin. All formulations were then applied to the skin sections using a positive displacement pipette set to deliver 10 µL formulation/cm². The dose was spread across the surface with the Teflon tip of the pipette. Five to ten minutes after application, the chimney portion of the Franz Cell was replaced. At pre-selected times after dosing, (6, 12, 24, and 48 hours) the reservoir solution was removed in its entirety, replaced with fresh reservoir solution, and a predetermined volume aliquot saved for subsequent analysis.

A single skin section from each donor was mounted onto cells which were not dosed but used to evaluate for the appearance of substances diffusing out of the skin, which may represent endogenous calcitriol. After the last sample was collected, the skin surfaces were washed twice (1.0 mL volume each) with 80:20 Ethanol:Water to collect un-absorbed formulation from the surface of the skin. Following the wash, the skin was removed from the chamber and split into epidermis and dermis. Each layer was extracted overnight in 80:20 Ethanol:Water.

Quantification of calcitriol was by High Performance Liquid Chromatography (HPLC). Briefly, HPLC was conducted on a Hewlett-Packard 1100 Series HPLC system with an Agilent 1100 Series LC/MSD. A solvent system consisting of A) 0.1% Ammonium Acetate in Water and B) 0.1% Ammonium Acetate in Methanol was run through a Phenomenex Luna C18 (2) column (100A, 3µ 100 × 4.6 mm) at a flow rate of 0.550 mL/min. Peak areas were quantified to concentration using an external standard curve prepared daily from the neat standard. Samples not assayed on the day of collection were stored at or below -20°C.

In the pilot study, a single formulation from the group was dosed to six chambers at about 5 µL/cm² dose on a single donor. Receptor solutions were collected at 0, 2, 4, 8, 12, 24, 32, and 48 hours. Following the last receptor solution sample, the surface was washed and the skin collected for analysis as previously described. All samples were processed and analyzed for calcitriol content.

The final design of the pivotal study was based on the results observed in the pilot study, in particular, applied dose, receptor solution sampling schedule, and sample processing methods. These modifications were made to optimize the detection and quantification of calcitriol in the pivotal study samples. For example, although the pilot protocol states that reservoir samples were taken at 2, 4, 8, 12, 24, 32, and 48 hours, it was determined after the pilot study, that reservoir samples would be taken at 6, 12, 24, and 48 hours to facilitate better detection levels of Calcitriol in the reservoir samples. In addition, following a pilot study, it was determined that dosing to 2 cm² with 20 µL (dosing amount was then 10 µL/cm²) would improve detection of calcitriol in the reservoir solution samples. However, the non-dosed chambers were retained at 1 cm². The following parameters were calculated: a) total absorption (sum of all reservoir solutions); b) rate and extent of penetration across the study period; and c) mass balance of the applied dose. For data evaluation, a) if any sample was <LLQ (Lower Limit of Quantification), then that sample may be treated as a non-data value. For radioactive samples (*e.g.,* the water integrity test), LLQ was defined as the predetermined mean background of blank samples. At the discretion of the investigator, all values <LLQ were declared as zero values or actual value measured for the purpose of calculating key parameters; b) a suspected outlier were confirmed if it is greater than the mean ± 3SD range of the same values from the set of remaining replicate chambers, or as determined by the Dean and Dixon Outlier test. At the discretion of the investigator, values declared as outliers were removed from the overall summation of the data (but are noted as such in the text or data tables); c) within a chamber, if a given time-point value has been declared a non-data value, or is missing due to other reasons, the time-point value was replaced with an interpolated value to calculate the relevant parameters. The interpolated value is calculated on a line that connects the adjacent values as follows:
▪ Given 3 points: (T1,A), (T2,B) and (T3,C) with (B) missing,
▪ Where T = Time and A-C = measured data values
▪ Estimated B = A - [((A-C)/|T1-T3|) × (|T1-T2|)]

For statistical evaluation, replicates within donors were averaged and standard deviation calculated for each key parameter. Within donor averages were then collated and the across donor population mean with standard error was calculated. Differences between test articles were evaluated using the Student's t-test.

Using this protocol, the following test formulations were evaluated:
▪ **A:** (1 ppm):dissolve 0.2 mL (1% (w/v)) of 100 ppm calcitriol concentrate (lot number **H,** below) into 19.8 mL (99% (w/v)) of 200 proof ethanol (1 µg/mL).
▪ **B** (1 ppm): first, dissolve 0.2 mL (1% (w/v)) of 100 ppm calcitriol concentrate (lot number **H,** below) into 11.8 mL (59% (w/v)) of 200 proof ethanol; then add 8 mL (40% (w/v)) of propylene glycol, and mix until clear and uniform (1 µg/mL).
▪ **C** (1 ppm): first, dissolve 0.2 mL (1% (w/v)) of 100 ppm calcitriol concentrate (lot number **H,** below) into 11.8 mL (59% (w/v)) of 200 proof ethanol; then add 6 mL (30% (w/v)) of propylene glycol and 2 mL (10% (w/v)) of ethoxydiglycol, and mix until clear and uniform (1 µg/mL).
▪ **D** (3 ppm): first, dissolve 0.6 mL (3% (w/v)) of 100 ppm calcitriol concentrate (lot number **H,** below) into 11.4 mL (57% (w/v)) of 200 proof ethanol; then add 6 mL (30% (w/v)) of propylene glycol and mix until clear and uniform; finally add 2 mL (10% (w/v)) of ethoxydiglycol and mix until clear and uniform (3 µg/mL).
▪ **E** (1 ppm): first, dissolve 0.2 mL (1% (w/v)) of 100 ppm calcitriol concentrate (lot number **H,** below) into 11.72 mL (58.6% (w/v)) of 200 proof ethanol (DP-04-099); then add 6 mL (30% (w/v)) of propylene glycol and mix until clear and uniform; then add 2 mL (10% (w/v)) of Transcutol P and mix until clear and uniform; finally, add 0.08 mL (0.4% (w/v)) of Phospholipon 90G concentrate (lot number **G,** below) and disperse into solution, mix until clear and uniform (1 µg/mL).
▪ **F** (3 ppm): first, dissolve 0.6 mL (3% (w/v)) of 100 ppm calcitriol concentrate (lot number **H,** below) into 11.32 mL (56.6% (w/v)) of 200 proof ethanol ; then add 6 mL (30% (w/v)) of propylene glycol and mix until clear and uniform; then add 2 mL (10% (w/v)) of Transcutol P and mix until clear and uniform; finally, add 0.08 mL (0.4% (w/v)) of Phospholipon 90G concentrate (lot number **G,** below) and disperse into solution, mix until clear and uniform (31 µg/mL).
▪ **G:** mix 50 g (50% (w/v)) of 200 proof ethanol with 50 g (50% (w/v)) of Phospholipon 90G, and mix until clear and uniform.
▪ **H:** completely dissolve 0.01 mg (0.01% (w/v)) of calcitriol in 100 mL (99.99% (w/v)) of 200 proof ethanol.

All reagents used in this study were analytical reagent grade or better. Source of unique reagents will be noted after the first mention of each chemical within the text of the final report.

The results of this study are summarized in the Summary table below:

**Summary Table: Average Results Across Donors for Calcitriol Content in Epidermis, Dermis, and Total Absorption Percutaneous Absorption of Calcitriol using Human Cadaver Skin over 48 hours from a Single Application. Mean ± SE as Total Mass (ng)**

| **Test Article** | **Epidermis (ng/cm²)** | **Dermis (ng/cm²)** | **Total Absorption (ng/cm²)** |
|---|---|---|---|
| Lot **A** | 0.98 ± 0.19 | 0.11 ± 0.11 | 9.85 ± 0.62 |
| **Lot B** | 1.63 ± 0.44 | 0.19 ± 0.19 | 9.84 ± 0.67 |
| Lot **C** | 1.89 ± 0.54 | 0.00 ± 0.00* | 9.74 ± 0.43 |
| Lot **D** | 6.44 ± 0.74 | 0.00 ± 0.00 | 10.51 ± 0.10 |
| Lot **E** | 2.19 ± 0.14 | 0.00 ± 0.00 | 9.96 ± 0.32 |
| Lot **F** | 4.83 ± 0.42 | 0.00 ± 0.00 | 8.80 ± 0.25 |
| Non-Dosed Blank Cells | 0.37 ± 0.37 | 0.00 ± 0.00 | 13.75 ± 0.59** |

| | | | |
|---|---|---|---|
| * Zero values indicated results below the lower limit of detection. ** Presumed to be endogenous calcitriol being released from the skin. | | | |

The data indicate that calcitriol did penetrate into, but not necessarily through, human cadaver skin, *in vitro,* from the test formulations evaluated. Blank, non-dosed, skin sections from each donor demonstrated an HPLC/MS coeluting peak consistent with endogenous calcitriol. The amount present in the reservoir solution, being essentially identical across all test formulations, and similar to the non-dosed skin sections, was most likely the diffusion of endogenous calcitriol being released from the skin sections. As little difference was seen across the test formulations and the non-dosed chambers, it is unlikely that the amount seen in the reservoir solution represents calcitriol coming from the topically applied test formulations.

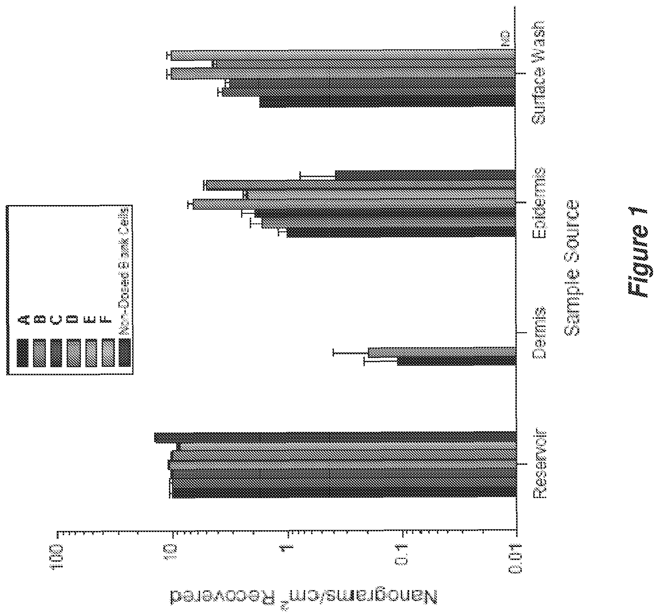

Evidence of calcitriol absorption was observed, as dermal contents, in those skin sections that were dosed with two formulations **(A** and **B),** is seen in Figure 1. As no measurable levels in the dermal skin layer was seen from the non-dosed skin sections, the measurable dermal levels from these two test formulations are interpreted, therefore, to represent absorption from the applied dose. In addition, all epidermal samples dosed with test formulations demonstrated calcitriol levels greater (~3× to ~17×) than the non-dosed skin sections. Rank ordering based upon epidermal calcitriol content arranges the test formulations as:

**D > F > E > C > B > A**>>>Non-Dosed Skin

Consistent with this rank order is that the test formulations demonstrating the greater epidermal contents where those that contain the higher concentrations (3 µg/mL vs. 1 µg/mL) of calcitriol **(D** and **F).** A very similar rank order is observed in the surface wash results (recovery of residual test article from the surface of the skin). No calcitriol was found in the surface wash of the non-dosed blank skin sections.

### Example 2. Identification of Key Proteins involved in Epidermal Cell Culture Response to Calcitriol - Real Time PCR (RTPCR)

This and the following several examples provide additional information regarding the identity of proteins or genes in the activation pathways for Calcitriol. These experiments allow the identification of the mechanism of action and key proteins/genes involved in the cellular response of epidermal cells to vitamin D compounds.

Specifically, it was found that exposing the keratinocyte cell line HEKa to calcitriol caused a significant impact on cellular processes. The experiments described herein focus on the identification of key proteins / genes that were involved in calcitriol induced changes in calcium channel transport and changes in regulation of heat shock proteins. Real-time polymerase chain reaction (RTPCR) methods were employed in this example to identify changes in the level of mRNA's for genes involved in ion channels, transport proteins, and heat shock proteins.

Using PCR arrays as a screening tool, a spectrum of molecular targets that would potentially offer an insight to the mode of biological action of calcitriol within the cells were evaluated. Changes in mRNA levels were evaluated using real-time PCR quantification to assess mRNA levels in preselected subsets containing 80 pathway specific targets (see Appendix). The PCR array analysis utilized two groups of genes - those related to Heat Shock Proteins (SABiosciences), and those related to Neuroscience Ion Channels and Transporters (SABioscience).

*Cell culture:* Primary human epidermal keratinocytes (HEKa) were maintained in Epilife Medium (Cascade Biologics, Inc., Portland OR) along with Human Keratinocyte Growth Supplement (Cascade Biologics, Inc., Portland OR). Cells were grown at 37°C with 5% CO₂.

*D3 treatment of HEKa cells:* HEKa cells were treated with 0.1 µg/mL of calcitriol or the control vehicle. To give a 0.1 µg/mL final concentration of calcitriol, 1 mg of Calcitriol was dissolved in 2 mL of ethanol, and 1 µL of the resulting stock was added to 5 mL of media. Vehicle control group of cells were treated with 5 mL media containing 1 µL of ethanol. Cells were harvested 3, 6, 16, 24, 48, or 72 hours after the start of the treatment.

*RNA isolation:* Cells were lysed for RNA isolation at different treatment times using the RNeasy Mini kit (Qiagen, Inc., Valencia CA) following the manufacturer's instructions. RNA was quantified by measuring optical density at 260 nm.
First Strand Synthesis: First strand cDNA was synthesized from 1 µg of total RNA using the RT2 First Strand Synthesis kit (SABiosciences., Frederick MD) as per manufacturer's recommendations.

*Real-time PCR:* Products from the first strand synthesis were diluted with water, mixed with the SYBR green master mix (SABiosciences., Frederick MD) and loaded onto PCR arrays. Real time PCR was run on the PCR Arrays (Heat Shock Protein Arrays, and Neuroscience and Ion Channel Arrays) (SABiosciences, Frederick MD) on a Biorad CFX96. Data analyses were performed using the PCR array data analysis software available on the SABiosciences website.

Table 2-1 below shows the genes on the Heat Shock Protein Gene Array that are regulated in HEKa cells after calcitriol treatment. Results show only those genes that were regulated in two independent experiments.

**Table 2-1 Genes in the Heat Shock Protein Array Regulated by VitaminD3 Treatment.**

| **Gene symbol** | **Protein** | **Regulation Pattern** |
|---|---|---|
| HSPB1 | Heat shock 27 kDa protein 1 | Down regulated at 48 hours |
| DNAJC6 | DnaJ (Hsp40) homolog, subfamily C, member 6 | Downregulated |
| HSPA2 | Heat shock 70kDa protein 2 | Upregulated at 48 hours |
| HSF4 | Heat shock transcription factor 4 | Upregulated at 48 hours |

Two of the genes that were regulated at the mRNA level by calcitriol treatment in HEKa cells were HSPB1 and HSPA2. HSPB1 is a 27 kDa protein that is expressed not only in the cell membrane, but also in the cytosol, mitochondria, and the golgi bodies. HSPA2 is a 70 kDa protein present in the cell membrane and nucleus, and is regulated by HSF1. Both HSPB1 and HSPA2 have been implicated in apoptosis. HSF4 is regulated by retinoic acid, and is involved in cell differentiation. DNAJC6 belongs to the HSP40 group of proteins. It is present in clathrin coated vesicles and in the cytoplasm.

Similarly, results obtained from the Neuroscience and Ion Channels Array consistent from three independent experiments are summarized below in Table 2-2.

**Table 2-2 Genes in the Neuroscience and Ion Channels Array Regulated by Vitamin D3 Treatment**

| **Gene Symbols** | **Gene** | **Regulation Pattern** |
|---|---|---|
| SLC1A1 | Solute Carrier family 1 (neuronal/epithelial high affinity glutamate transporter, system Xag), member 1) | upregulated at 16 hrs |
| KCNB2 | Potassium voltage-gated channel, Shab-related subfamily, member 2 | upregulated until 24 hours |
| KCNN4 | Potassium intermediate/small conductance calcium-activated channel, subfamily N, member 4 | upregulated at 48 hours |
| SLC1A3 | Solute carrier family 1 (glial high affinity glutamate transporter), member 3 | downregulated at 48 hours |

Changes in glutamate transporters and in potassium channels was consistently observed. SLC1A1 (also known as EAAC1 or EAAT3) is known to be primarily responsible for transport of the excitatory neurotransmitter glutamate across the membrane. This solute carrier protein has been found outside of the nervous system in tissues such as the heart and skin. In rat keratinocytes, there is evidence showing the involvement of glutamate signaling and SLC1A1 in wound healing (Genever *et al.,* 1999). Inhibition of SLC1A1 by Riluzole, a drug currently in clinical trials for melanoma (Clinical Trials.gov, Mosby's Drug Consult, 13th Edition) is indicative of a biological role of SLC1A1 in skin cells. Given that SLC1A1 has been implicated in anti-apoptotic mechanisms in injured motor neurons (Kiryu-Seo *et al.,* 2006), the observation in this experiment that SLC1A1 is upregulated by D3 treatment in HEKa cells suggests a potential protective mechanism pathway link.

SLC1A3 (also known as EAAT1 or EA6) is another solute carrier which allows a sodium-dependent glutamate and aspartate transport. Typically found in glial cells in the brain, this transporter is involved in cleaning up the synaptic space of glutamate, thereby preventing prolonged depolarization of post synaptic neurons. SLC1A3 is known to interact with glial derived neurotropic factor (GDNF) and phosphodiesterase 6B (PDE6B). It is possible that SCL1A3 is involved in reducing cytotoxicity.

KCNN4 is a potassium intermediate / small conductance calcium-activated channel, subfamily N, member 4. Following its activation, the cell membrane is hyperpolarized and there is increased calcium influx into the cell. This potassium channel is localized in many tissues outside of the nervous system.

KCNB2, potassium voltage-gated channel, Shab-related subfamily, member 2, is upregulated at about 24 hours post calcitriol treatment. This potassium channel is important in regulating neurotransmitter release, insulin secretion and smooth muscle contraction.

Although calcitriol was used in these experiments, one of skill in the art will readily understand that other vitamin D compounds of the invention (such as those described herein above) may also exhibit similar activities in regulating target gene expression. It is contemplated that, in certain embodiments, the vitamin D compounds of the invention exhibit a similar or identical gene modulation profile as that of calcitriol in these experiments, *e.g.,* up-regulating the expression (mRNA and/or protein) of one or more target genes similarly up-regulated by calcitriol, or down-regulating the expression (mRNA and/or protein) of one or more target genes similarly down-regulated by calcitriol.

### Example 3. Identification of Key Proteins involved in Epidermal Cell Culture Response to Calcitriol - Antibody Array

Evaluation of protein changes upon calcitriol stimulation was also evaluated through utilization of antibody microarrays, which are capable of screening for changes in over 700 potential target proteins.

In this experiment, an antibody microarray (Panorama XP725 Antibody Array, Sigma) encompassing antibodies against over 700 target proteins was utilized to assess changes in protein concentration/level in HEKa cells treated with calcitriol for about 3, 6, or 24 hours, respectively. Briefly, the treated HEKa cells were first harvested and then extracted to obtain a soluble protein supernatant. Two portions of the extracted protein sample (~1 mg total) from each sample (at 1 mg/mL) were each labeled with fluorescent dye (Cy3 and Cy5, respectively). The excess dye was removed from the protein sample, and the resulting labeled protein samples were used for microarray incubation.

To determine the expression level of a particular target protein at a later time point (*e.g.,* at hour 6 or 24) relative to that at an earlier time point (*e.g.,* at hour 3), the samples were labeled by different labels (*e.g.,* 3-hour extract labeled with Cy3, 6-hour or 24-hour extract labeled with Cy5). Then the two labeled samples containing equal amounts of total protein were mixed (*e.g.,* Cy3-labeled 3-hour sample is mixed with Cy5-labeled 6-hour or 24-hour samples, respectively). After incubation with the microarray chip (according to manufactures recommended protocols), the chips were washed and dried. The microarrays were then scanned with a fluorescent laser scanner to measure the relative fluorescence intensity of the Cy3 and Cy5 dyes.

If the amount of a particular type of target protein increased (or decreased) over time, more (or less) of the dye associated with the later time point (*e.g.,* Cy5) will be retained by the microarray. For example, in this experiment, the earliest time point (*e.g.,* 3-hour) was used as a baseline to determine the relative protein expression level at two later time points (*e.g.,* 6-hour vs. 24-hour). If more Cy5 is retained by the array between 6-24 hours, the expression level of the target protein increased over the time period. Conversely, if there is a decrease in retained Cy5 between hour 6 and 24, the target protein expression level is decreased.

Initial analysis using this method focused on those target proteins exhibiting relative expression level changes >2-fold (increase or decrease). Overall, the antibody array experiments using the calcitriol-treated (24 hour) HEKa cells identified the following target proteins (in Tables 3-1 and 3-2) with significantly altered expression level in response to vitamin calcitriol:

**Table 3-1. Target Proteins with Increased (>2-fold) Protein Levels Following Calcitriol Treatment**

| | |
|---|---|
| Amyloid Precursor Protein | HDAC2 |
| ARTS | HDAC6 |
| ASAP 1 Centaurin b4 | ILK |
| BACH1 | MAP Kinase Activated Protein Kinase2 MAPKAPK2 |
| Bclx | MAP Kinase ERK1 |
| BclxL | Melanocortin3 Receptor |
| BID | Myosin IX Myr5 |
| Bmf | Neurofilament 200 |
| CENPE | Nitric Oxide Synthase bNOS |
| cMyc | p120ctn |
| Cofilin | PAD14 |
| Connexin 32 | Par4 Prostate Apoptosis Response 4 |
| Csk | Presenilin1 |
| CtBP1 | Proliferating Cell Protein Ki67 |
| DcR2 | Protein Kinase Ba |
| Dimethyl Histone H3 diMeLys4 | PUMA bbc3 |
| Dimethyl Histone H3 diMeLys9 | ROCK1 |
| Dystrophin | S100 |
| ERK5 BIG MAPKBMK1 | SHPTP2 |
| Estrogen Receptor | Sin3A |
| FKHRL1 FOXO3a | Substance P Receptor |
| Focal Adhesion Kinase pp 125FAK | Synaptopodin |
| FOXP2 | Tumor Necrosis Factor a |
| Glutamic Acid Decarboxylase 65 | Ubiquitin Cterminal Hydrolase L1 |
| Glutamic Acid Decarboxylase GAD65 67 | Uvomorulin ECadherin |
| gTubulin | Vitronectin |

**Table 3-2. Target Proteins with Decreased (>2-fold) Protein Levels Following Calcitriol Treatment**

| |
|---|
| Crk II |
| Growth Factor Independence1 |
| Serine Threonine Protein Phosphatase 1b |
| Cathepsin D |
| Transforming Growth Factorb pan |
| WAVE |
| Protein Tyrosine Phosphatase PEST |
| CD40 |

Evaluation of calcitriol treated HEKa cells at 24-hour with the same protein antibody array method identified about fifty proteins that were significantly upregulated. These proteins generally fall within four categories: (i) transcriptional and cell cycle control (Table 3-3); (ii) structural, cytosckeletal and adhesion proteins (Table 3-4); (iii) apoptosis regulation proteins (Table 3-5); and (iv) nerve cell differentiation and Alzheimer's disease (Table 3-6).

**Table 3-3. Over-expressed Proteins relating to Cell Cycle and Transcriptional Control after 24 Hours of Calcitriol treatment**

| **Protein** | **Function** |
|---|---|
| BACH1 | transcription factor (Alzheimer's) |
| CENPE | Centromere protein that accumulates in the G2 phase of the cell cycle |
| cMyc | transcription factor (Cancer oncogene) |
| C-src tryosine kinase (Csk) | cell growth (Cancer) |
| CtBP1 | transcriptional repressor |
| Dimethyl Histone H3 diMeLys4 | transcription regulation |
| Dimethyl Histone H3 diMeLys9 | transcription regulation |
| Estrogen Receptor | ligand dependent nuclear receptor |
| FKHRL1 FOXO3a | transcription factor, linked to ROCK kinase and NO signaling |
| FOXP2 | transcription regulator, in development of brain, lung, gut |
| HDAC2 | regulates gene expression |
| MAP Kinase Activated Protein Kinase2 MAPKAPK2 | A kinase involved in many cellular processes (stress and inflammatory responses, nuclear export, gene expression regulation and cell proliferation). Heat shock protein HSP27 was shown to be one of the substrates. |
| MAP Kinase ERK 1 | acts in a signaling cascade that regulates various cellular processes such as proliferation, differentiation, and cell cycle progression in response to a variety of extracellular signals, phosphorylates nuclear proteins |
| Melanocortin3 Receptor | hormone receptor |
| Proliferating Cell Protein Ki67 | proliferation marker |
| S100 | calmodulin-like calcium binding protein involved in regulation of multiple cell processes |
| SHPTP2 | a kinase that plays a regulatory role in various cell signaling events |
| Sin3A | transcriptional regulatory protein |

**Table 3-4. Over-expressed Proteins relating to Structural, cytosckeletal and adhesion (after 24 Hours of Calcitriol treatment)**

| **Protein** | **Function** |
|---|---|
| ARTS | Regulates cytoskeletal organization |
| ASAP1 Centaurin b4 | reculate actin cytoskeleton |
| Cofilin | dissembles actin filaments |
| Connexin 32 | major component of peripheral myelin |
| Dystrophin | large protein for cytoskelton connection |
| Focal Adhesion Kinase pp125FAK | Phosphorylation of focal adhesion kinase is increased in keratinocytes induced to migrate |
| gTubulin | microtubial, spindle pole |
| Myosin IX Myr5 | motor proteins |
| Neurofilament 200 | nerve cell related structural protein |
| p120ctn | adhesion and signal transduction |
| PAD14 | converts arginine residues to citrulline residues; may regulate intermediate filament proteins and intermediate filament-associated proteins in cells undergoing degenerative processes |
| ROCK1 | kinase, contributes to actin stability |
| Uvomorulin ECadherin | Ca-dependent cell adhesion molecule, transmembrane glycoprotein that functions to regulate epithelial cell recognition and adhesion |
| Vitronectin | promotes cell adhesion and spreading |

**Table 3-5. Over-expressed Proteins relating to Apoptosis control (after 24 Hours of Calcitriol Treatment**

| **Protein** | **Function** |
|---|---|
| Bclx | Apotosis regulation |
| BclxL | Apotosis regulation |
| BID | Apotosis regulation |
| Bmf | Apotosis regulation |
| DcR2 | Receptor contains an extracellular TRAIL-binding domain, a transmembrane domain, and a truncated cytoplamic death domain. This receptor does not induce apoptosis, and has been shown to play an inhibitory role in TRAIL-induced cell apoptosis. |
| ERK5 BIG MAPKBMK1 | Protects Endothelial Cells From Apoptosis by phosphorylation of Bad |
| Integrin-linked kinase (ILK) | regulating integrin-mediated signal transduction, may prevent apoptosis in association with PKB/Akt pathways |
| Protein Kinase Ba | (Akt) involved in cell survival and inhibition of apoptosis |
| PUMA bbc3 | apoptosis regulator |

**Table 3-6. Over-expressed Proteins Associated with Nerve Cell Differentiation and Alzheimer's Disease (after 24 Hours of Calcitriol treatment)**

| **Protein** | **Function** |
|---|---|
| Amyloid Precursor Protein | Amyloid precursor protein (APP) is an integral membrane protein expressed in many tissues and concentrated in the synapses of neurons. Its primary function is not known, though it has been implicated as a regulator of synapse formation[2] and neural plasticity.[3] |
| BACH1 | transcription factor (Alzheimer's) |
| Presenilin1 | the sub-component of gamma secretase that is responsible for cutting APP (mutations observed in Alzheimer's) |
| Glutamic Acid Decarboxylase 65 | neurotransmitter production (Schizophrenia) |
| Glutamic Acid Decarboxylase GAD65 67 | neurotransmitter production (Schizophrenia) |
| Neurofilament 200 | nerve cell related structural protein |
| Nitric Oxide Synthase bNOS | inducible, cell signaling, immune systems |
| Substance P Receptor | a neuropeptide receptor |
| Synaptopodin | actin binding protein, involved in spine apparatus formation in neurons |
| Connexin 32 | major component of peripheral myelin |
| Tumor Necrosis Factor a | regulation of immune cells |
| Ubiquitin Cterminal Hydrolase L1 | neuron specificity (Alzheimer's and Parkinson) |

### Example 4. Identification of Key Proteins involved in Epidermal Cell Culture Response to Calcitriol - Proteomic Analysis

A series of HEKa cultures were treated with calcitriol, and cell pellets were harvested at 3, 6, and 24 hours after calcitriol3 exposure. The cell pellets were then analyzed using proteomic methods, such as 2-D gel and Western blot analysis. In the experiment described below, HEKa cells were treated with 0.1 µg/mL calcitriol, and samples obtained at 3-, 6-, and 24-hour were processed by 2-D gel electrophoresis and the associated comparative analysis (results not shown).

In all, analysis of about 458 protein spots in the comparative study was performed, comparing the control sample against the 3-, 6-, and 24-hour treatment samples. Six spots showing statistically significant differential changes were identified. These spots were excised, and their protein contents subjected to sequence identification by trypsin digestion and mass spectrometry characterization.

Results (Table 4-1) showed that the set of six spots from the HEKa keritinocyte samples contained pure endogenous keratins, as opposed to keratin often observed as a common contaminant. Two S100 proteins were identified as being strongly regulated, along with Glutathione S-transferase and Galectin 1. There was evidence that Galectin 1 was glycosylated.

**Table 4-1 Proteins identified as being strongly modulated by Calcitriol based on 2-D gel electrophoresis study**

| **Spot** | **Identified Protein** | **Name** | **Response** | **Function** | **Cellular Location** |
|---|---|---|---|---|---|
| 4 | Glutathione S-transferase | GST | up at 3, 6, and 24 hours | GST transfer | cytoplasm |
| 2 | Keratin 1 | KRT1 | up at 6 hours and down at 24 hours | intermediate filament | cytoplasm |
| 8 | Keratin 17 | KR17 | down at 24 hours | intermediate filament | cytoplasm |
| 10 | S100 A9 (Calprotectin) | S 100A9 | down at 6 and 24 hours | Calcium binding protein | cytoplasm |
| 14 | S100 A13 | S100A13 | up at 6 and 24 hours | Calcium binding protein | cytoplasm |
| 27 | Galectin 1 | LGALS1 | up at 6 and 24 hours | beta-galactoside-binding protein | Extracellular |

The two S100 proteins (A9 and A13) belong to the calprotectin family of proteins. There are 21 different types of these low molecular weight proteins in the family. These S100 proteins bind calcium (EF-hand motif), and each type is expressed in a cell-specific manner, and in a level dependent upon environmental factors. Various diseases are associated with altered S100 protein levels (cardiomyopathies, neurodegenerative and inflammatory disorders, and cancer). Note that the S100 proteins were also identified in the antibody array results as being upregulated upon contacting calcitriol.

### Example 5. Effect of Calcitriol on Keratinocyte Growth

A series of HEKa cultures were treated with different concentrations of calcitriol, and the growth behavior of the HEKa cells analyzed after a pre-determined growth period. All experiments were conducted in 96-well plate format. Each well contained the same amount of HEKa cells in about 100 µL of media (usually between 2,000 - 5,000 cell/well). Calcitriol was dissolved in ethanol to make a stock solution. The stock solution was serially diluted 1:2 in the growth media, covering a range of between 4.0 µg/mL to about 15.5 ng/mL (9 test concentrations). About 100 µL of each test concentration of calcitriol was added a corresponding test well, resulting in a final volume of about 200 µL/well. The tested calcitriol concentrations are in the range of between 2.0 - 0.008 µg/mL (*e.g.,* corresponded to columns 2 through 10 in the 96-well plate). Column 11 was used as negative control (no calcitriol). All experiments were conducted in duplicates.

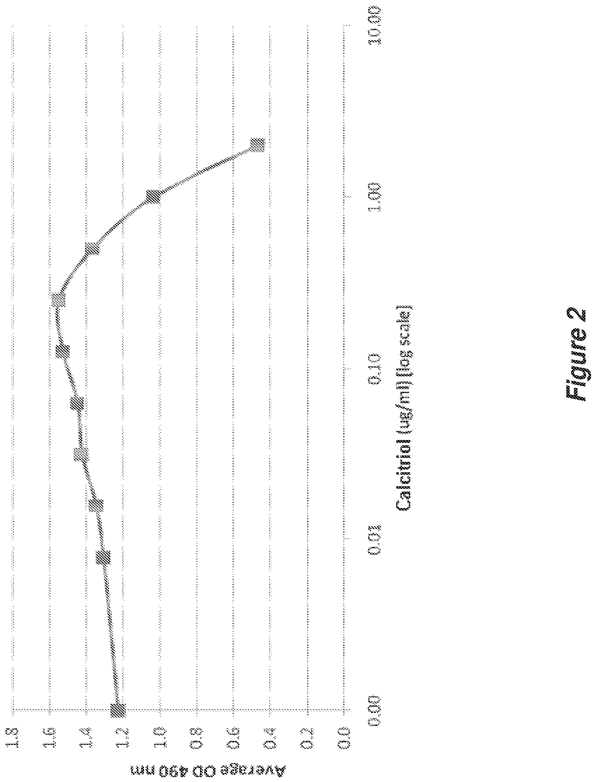

As shown in **Figure 2****,** calcitriol was titrated into HEKa cells over a concentration range from about 0.008-2.0 µg/mL. The lowest levels of calcitriol were well tolerated in the HEKa cells, and calcitriol appears to mildly stimulate HEKa cell growth (-10-20%). However, at calcitriol concentrations of about 1.0 µg/mL or greater, cell growth is inhibited. The overall dose response by the HEKa cells to calcitriol was consistent over a series of nineteen independent experiments over a period of about six weeks (data not shown).

### Example 6. Effect of Calcitriol on Cancer Cell Growth

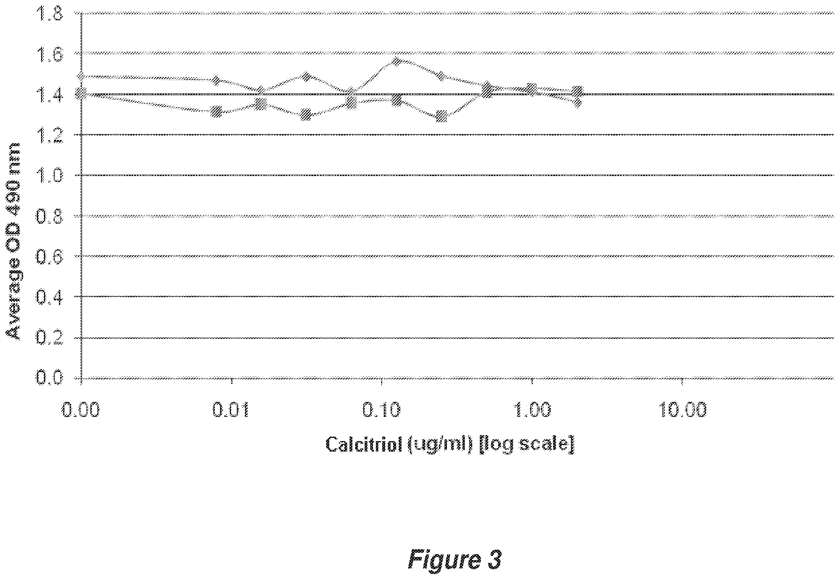

Unlike what was observed in the normal keratinocytes HEKa, no significant growth promoting or growth inhibiting effects were observed for most cancer or immortalized cell lines tested, including SkBr-3 (breast adenocarcinoma cancer, Her2 overexpressed), SKMEL-28 (melanoma), PaCa2 (pancreatic carcinoma), NCI-ES-0808, and NIH-3T3 (immortalized fibroblast). One exemplary growth curve exhibited by such cancer / immortal cell lines is shown in **Figure 3** for the pancreatic carcinoma cell line PaCa2. Note that the growth of PaCa2 was not affected over a wide range of calcitriol concentrations.

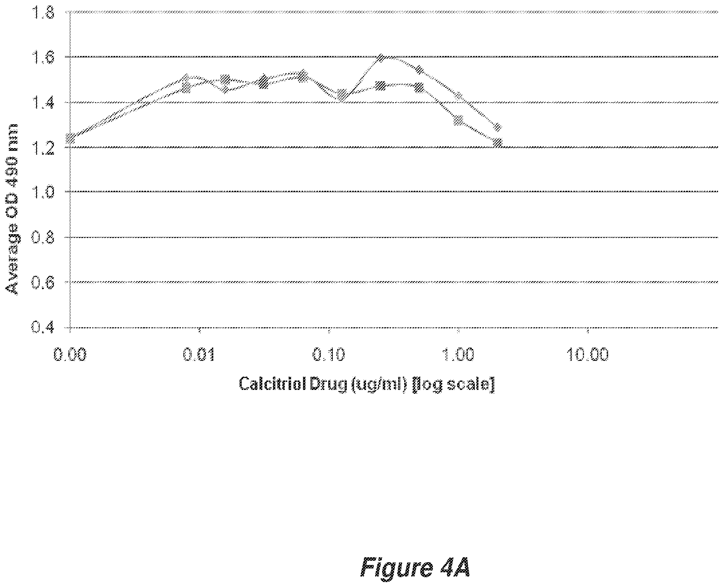

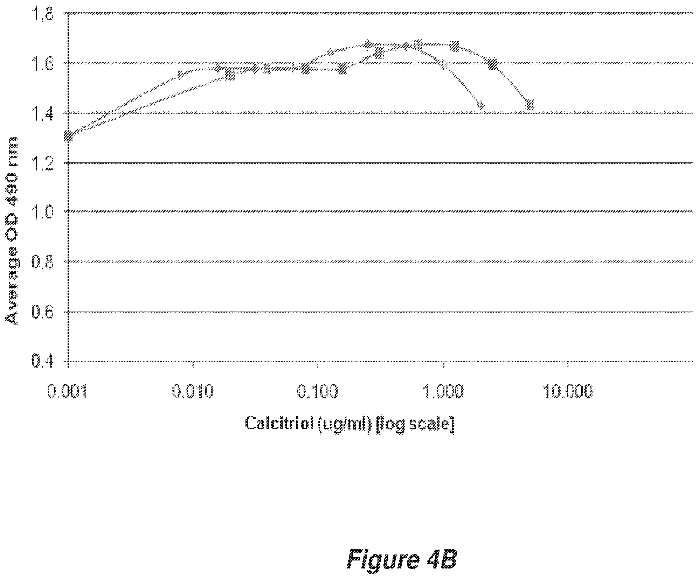

One two of the tested cancer cell lines, MCF-7 (breast cancer with p53 mutation) and HepG2 (liver cancer), similarly responded to calcitriol stimulation at low vitamin D3 concentrations (0.05-0.25 µg/mL), and calcitriol inhibition at high calcitriol concentrations (> 0.5 µg/mL). See **Figure 4****.**

These data suggest that the subject vitamin D compounds, when applied to normal keratinocytes (such as HEKa) up to a certain concentration limit, may be able to promote the growth of these normal keratinocytes, without simultaneously promoting cancer cell growth. Exceeding the concentration limit, the vitamin D compounds may in fact inhibit the growth of normal keratinocytes.

### Example 7. Protective Effect of Calcitriol on HEKa cells Against Various Chemotherapeutic Drugs

This example demonstrates that, with few exceptions, the vitamin D compounds of the invention can protect the normal keratinocytes (such as HEKa) against the cytotoxic effects of most types of front-line chemotherapeutic drugs. Specifically, seventeen anti-cancer drugs were tested to evaluate the impact of calcitriol on the cytotoxic effect of these drugs. The drug names and their respective mechanisms of actions are listed in the table below.

**Table 7-1 Drugs tested for chemoprotective activity of calcitriol in HEKa cells**

| **Drug Tested** | **Mechanism** |
|---|---|
| Doxorubicin | cytotoxic |
| 5-FU | pyrimidine antimetabolite |
| Tamoxifen | binds to estrogen receptors |
| Irinotecan | topoisomerase 1 inhibitor |
| Paclitaxel | mitotic inhibitor |
| Carboplatin | DNA alkylating agent |
| Etoposide | topoisomerase 2 inhibitor |
| Cyclophosphamide | alkylating agent |
| Cisplatin | DNA alkylating agent |
| Erlotinib (Tarceva) | EGFR tyrosine kinase inhibitor |
| Gemcitabine | pyrimidine antimetabolite |
| Staurosporin | nonspecific kinase inhibitor |
| Vincristine | microtubial inhibitor |
| Imatinib (Gleevec) | tyrosine kinase inhibitor (abl, c-kit, PDGF-R) |
| Gefitinib (Iressa) | EGFR tyrosine kinase inhibitor |
| Sorafenib | tyrosine kinase inhibitor (Raf, VEGF-R2, c-kit, PDGF-R) |
| Dasatinib | tyrosine kinase inhibitor (BCR/ABL) |

In the first series of experiments, a number of kinase inhibitor based drugs were used in assays designed to assess the ability of 0.1 µg/mL calcitriol to provide a protective effect on HEKa cells. These include: erlotinib (Tarceva), an EGFR Tyr kinase inhibitor; gefutubib (Iressa), an EGFR Tyr kinase inhibitor; sorafenib, inhibitor of several Tyr kinases (Raf, VEGF-R2, c-kit, PDGR-R); Dasatinib, a BCR/ABL Tyr kinase inhibitor; and staurosporin, a relatively nonspecific kinase inhibitor.

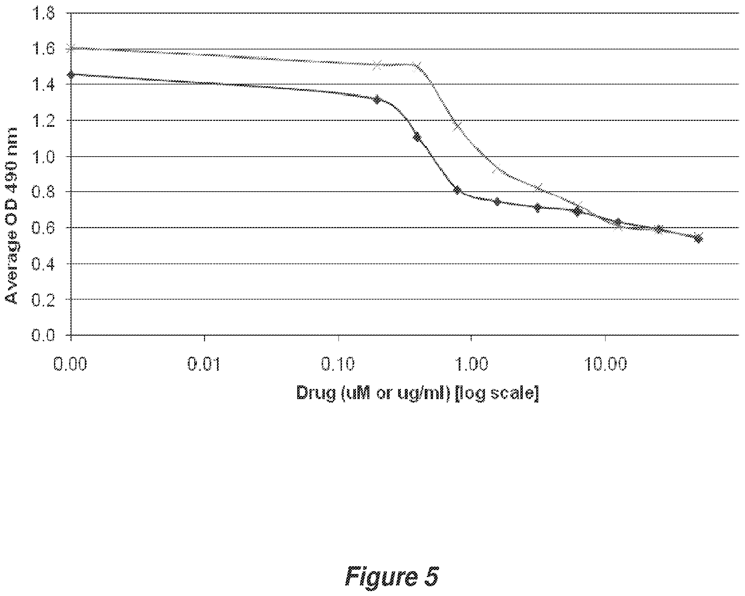

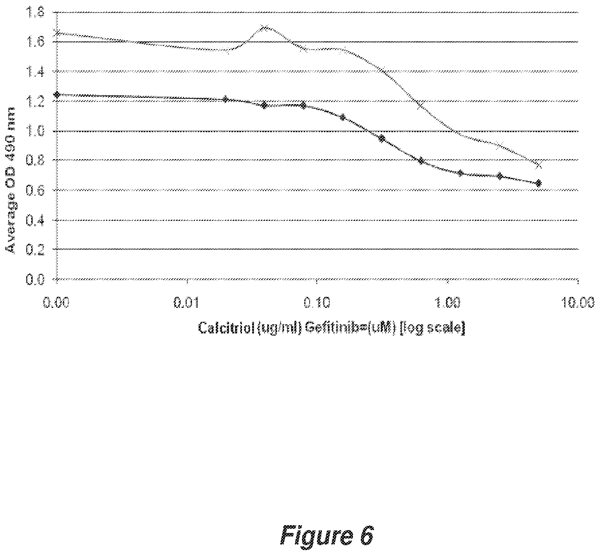

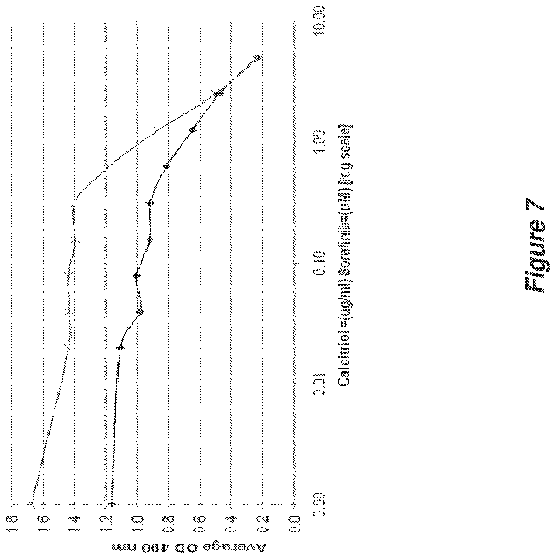

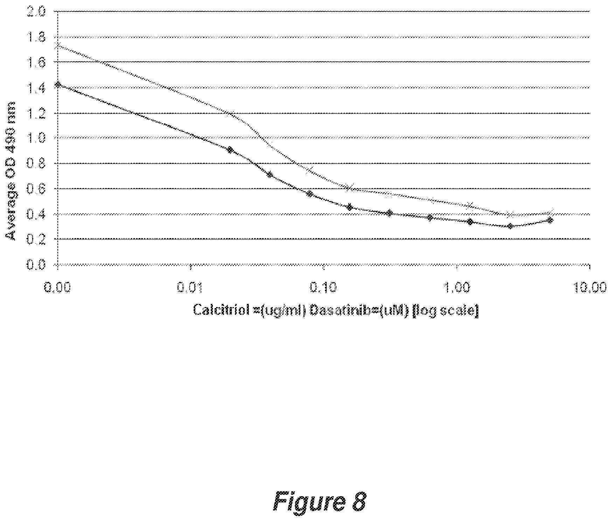

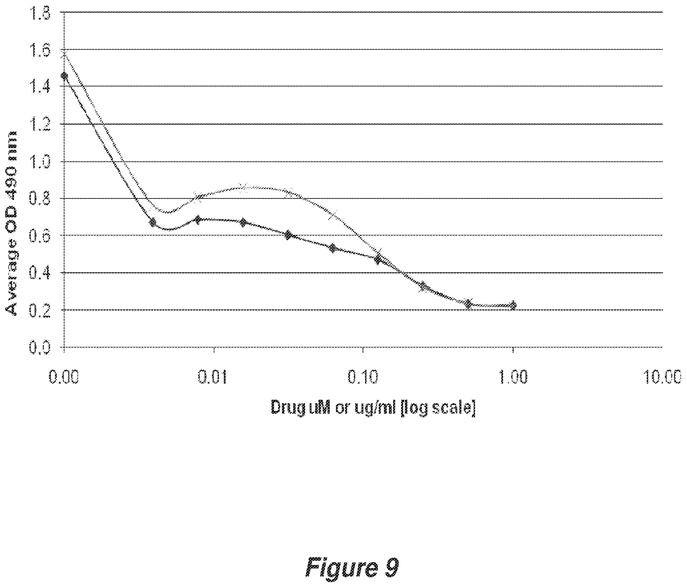

The dosing curves obtained in these experiments show a general trend that, at low drug dosage levels (not unlike those affecting the skin of patients undergoing systemically delivered chemotherapy), calcitriol provided certain growth stimulation and protected the HEKa cells *(see* **Figures 5-9****).** In addition, it appears that calcitriol has a more pronounced protective effect against more specific kinase inhibitors as compared to more non-specific kinase inhibitors.

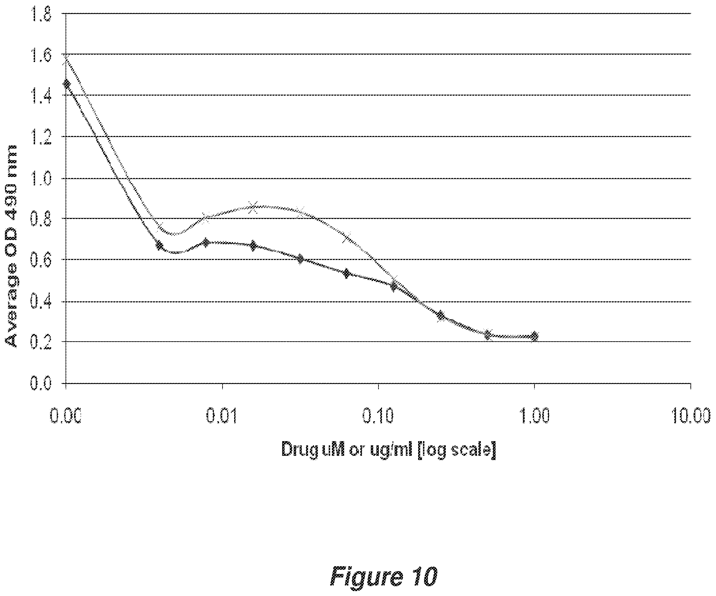

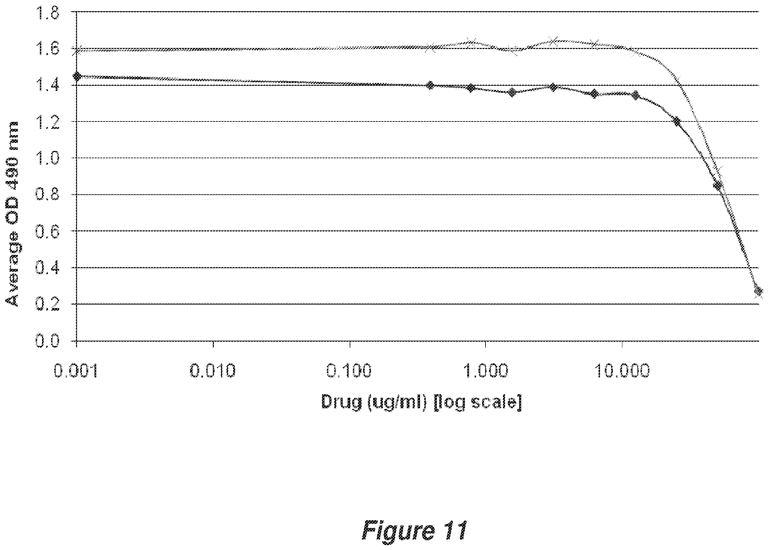

Similarly, calcitriol also exhibited a moderate level of protection against low dosage levels of alkylating agents, such as cisplatin and carboplatin (see **Figures 10** and **11****).**

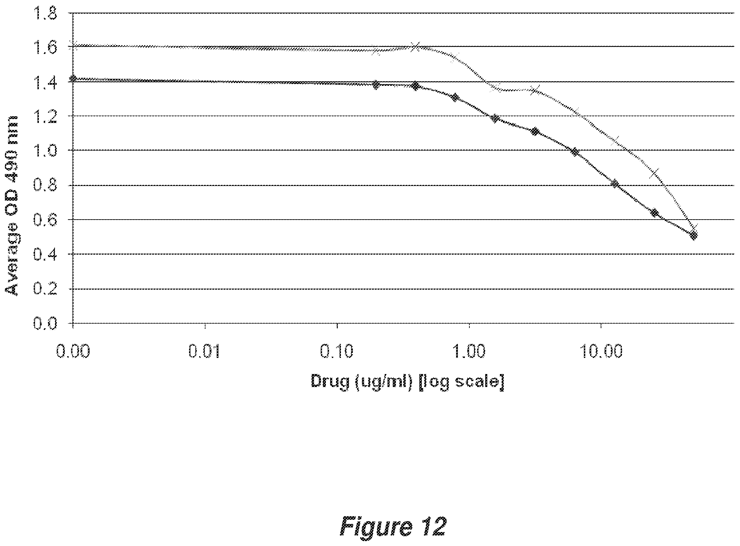

Irinotecan presumably inhibits cell growth through interaction with topoisomerase I. A positive protective effect against irinotecan was also observed in the presence of calcitriol **(****Figure 12****).**

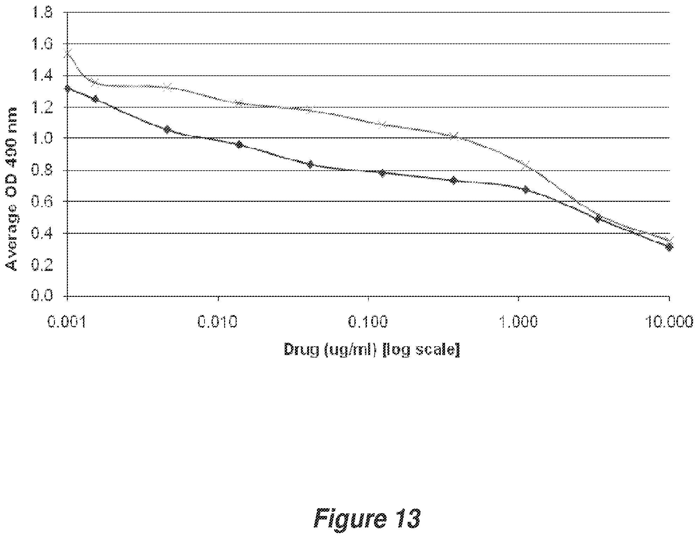

Paxlitaxol is a mitotic inhibitor. The presence of 0.1 µg/mL of calcitriol did provide some protective effects against Paxlitaxol **(****Figure 13****).**

Pyrimidine antimetabolite based drugs, such as 5-Fluorouricil (5-FU), act in several ways, but principally as a thymidylate synthase inhibitor. 5-FU blocks the synthesis of thymidine, which is required for DNA replication. Thus 5-Fluorouracil has been used topically for treating actinic (solar) keratoses and some types of basal cell carcinomas of the skin. At least a mild protective effect against 5-FU is seen when 0.1 µg/mL of calcitriol was present **(****Figure .14****).**

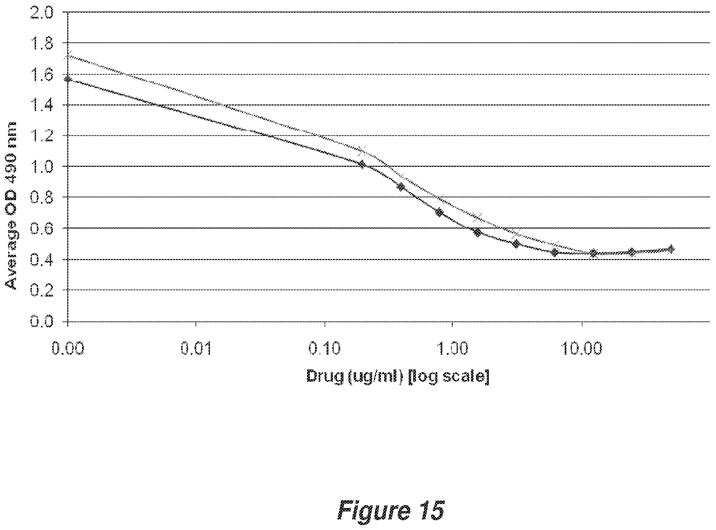

Gemcitabine is a nucleoside analog in which the hydrogen atoms on the 2' carbons of deoxycytidine are replaced by fluorine atoms. Similar to fluorouracil and other analogues of pyrimidines, gemcitabine replaces one of the building blocks of nucleic acids (which in this case is cytidine) during DNA replication. Gemcitabine is used in the treatment of various carcinomas: non-small cell lung cancer, pancreatic cancer, bladder cancer, and breast cancer. **Figure 15** shows that at least a mild protective effect against gemcitabine is seen when 0.1 µg/mL of calcitriol was present.

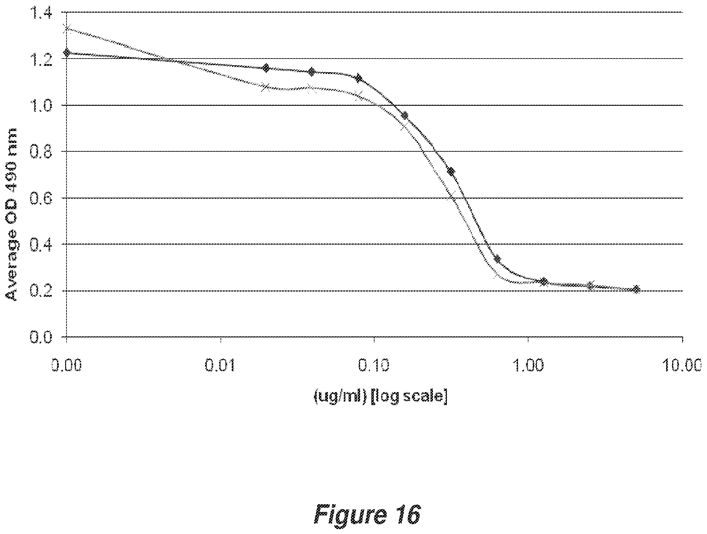

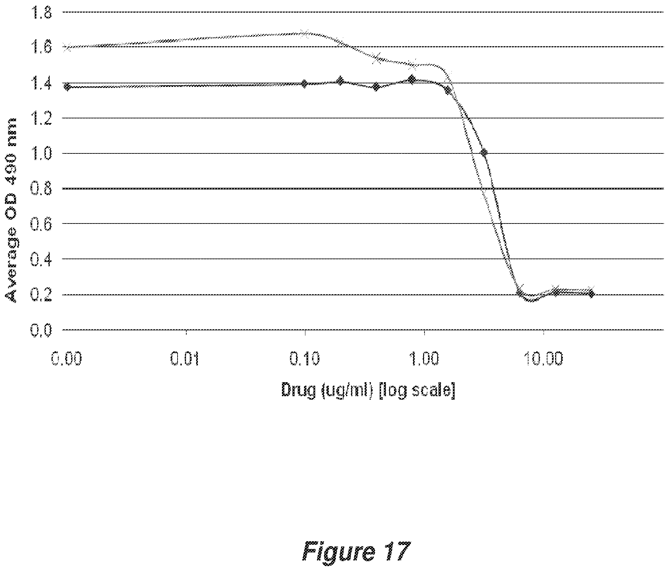

On the other hand, calcitriol did not appear to provide a significant protective effect against the cytotoxic effect of doxorubicin **(****Figure 16****).** In addition, any protective effect against tamoxifen is weak **(****Figure 17****).** Tamoxifen binds competitively to estrogen receptors on tumors and other tissue targets, producing a nuclear complex that decreases DNA synthesis and inhibits estrogen effects.

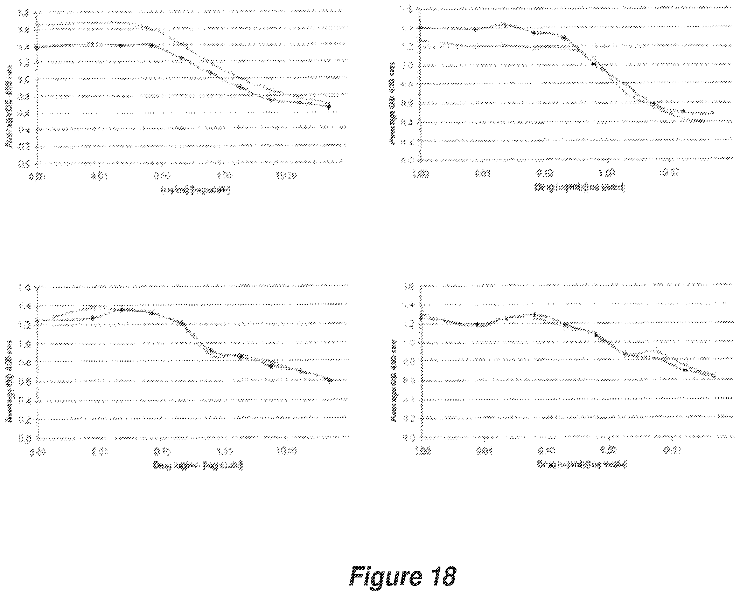

Consistent with the data above, data in **Figure 18** show that HEKa kerotinocytes were growth stimulated by calcitriol, and some levels of protection against 5-FU was observed in the HEKa cells. Interestingly, in three tested cancer cell lines, Hep-G2, PaCa-2, and SKMEL-28, the ED₅₀ curves for 5-FU treatments were not significantly different from those also having 0.1 µg/mL calcitriol supplement. Note that the Hep-G2 cells were mildly stimulated by calcitriol treatment, yet its 5-FU ED₅₀ curve did not substantially change even in the presence of calcitriol.

Similarly, exposure of the following 4 tested cancer cell lines: Hep-G2, MCF-7, PC-3 and PaCa; 2 - to 0.1 µg/mL of calcitriol for two passages did not alter the respond of these cells to other drugs (*e.g.,* doxorubucin, cisplatin, and erlotinib).

These results above suggest that calcitriol may protect the normal keratinocytes (such as HEKa) during chemotherapy (using 5-FU, for example) without antagonizing the effectiveness of the chemotherapy against cancer cells.

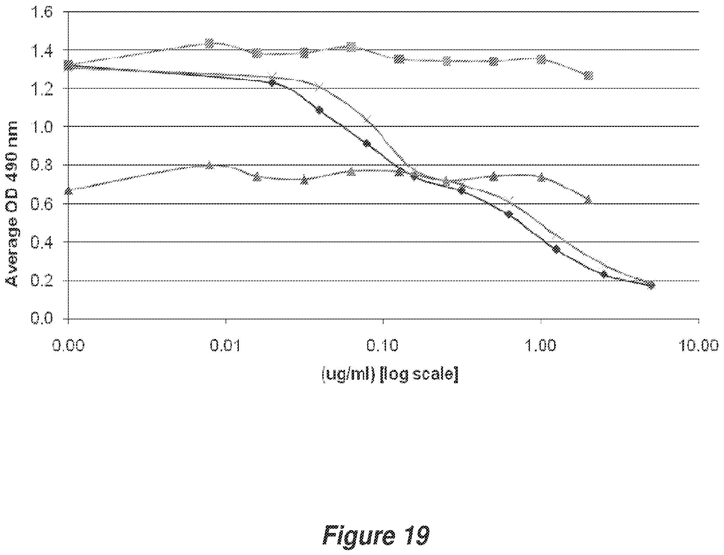

Much like what was observed in HEKa cells, calcitriol did not appear to appreciably alter the cytotoxic effect of Doxorubicin against cancer / immortal cells such as SkBr-3, SKMEL-28, PaCa-2, MCF-7, NCI-ES-0808, Hep-G2, and NIH-3T3 (see **Figure 19****).**

In addition, possible synergistic effects of the commercial drugs with calcitriol were also explored. In these experiments, a selected commercial drug was serially diluted, starting at a concentration 4-times higher than the final desired concentration for cell incubation. Meanwhile, a stock of 0.4 µg/mL calcitriol was prepared, and then mixed with the serially diluted drug (at a ratio of 1:1). The drug/calcitriol mixture was then incubated for at least 15 minutes, and was added to the cell media (at a ratio of 100 µL to 100 µL). Thus, the final calcitriol concentration was 0.1 µg/mL.

The drug treatment period was usually three days. At the end of the three days, the background OD of the 96-well plate was read at 280 nm, before 20 µL of the "Substrate Cell Titer 96 Aqueous One Solution Reagent" (Promega) was added to each well. The plate was returned to the 37°C incubator, and its OD at 490 nm was read each hour until an OD of approximately 1.5 was reached. The net OD increase was calculated by subtracting the pre-substrate OD reading.

The impact of the drug on the cells was calculated by comparing the OD at different concentrations in relation to the OD of the control wells (without the drug). The results of the Net OD as a function of drug concentration was plotted and used to determine ED₅₀ values.

Analysis of the HEKa cell results indicates that there is no interaction between calcitriol and most drugs tested, including 5-FU, doxorubicin, tamoxifen, irinotecan, paclitaxel, carboplatin, staurosporin, vincristine, cisplatin, erlotinib, gencitabine, imatinib, gefitinib, sorafinib and dasatinib. The same results also were obtained when drug combination was tested on other cells. Thus, while not wishing to be bound by any particular theory, it appears that the mechanisms of action of calcitriol and the above drugs are different.

### Example 8. Pretreatment of Cells with Calcitriol: Cell based Assay Testing of Calcitriol in the Presence and Absence of Chemotherapy Drugs

The above cell based assays to evaluate cell viability were used in the example to assess the potential protective effect of calcitriol against the action of selected chemotherapy drugs. Each cell line was allowed to grow in the presence of 0.1 µg/mL calcitriol for two cell passages. Then these pretreated cells were utilized to set up the cell based assay. In addition, untreated cells were used to establish a parallel experiment under duplicate drug/calcitriol concentrations. This allowed side-by-side comparison of the potential effects of prolonged calcitriol exposure prior to the administration of the chemotherapy drug.

After each of the five cell lines were grown for two cell passages in the presence of 0.1 µg/mL calcitriol, only the HEKa cells were significantly affected in their overall growth and morphology. The four cancer cell lines continued to grow and were not altered in their general morphological appearances. However, the HEKa cells stopped growing after prolonged calcitriol exposure, and their morphology changed into one that is elongated in one direction, as opposed to a more branched appearance prior to calcitriol treatment. For this cell line, a new batch of cells were started and were exposed to only a single passage in the presence of calcitriol, prior to testing in the presence of the chemotherapy drugs.

Three commonly used chemotherapy drugs (doxorubicin, cisplatin and erlotinib) were selected to evaluate calcitriol treated cells. The possible synergistic or protective effects of the commercial drugs with calcitriol were explored. In these experiments, the commercial drugs were serially diluted, starting at a concentration 4-times higher than the final desired concentration for cell incubation. A stock of 0.4 µg/mL of calcitriol was prepared and added to the serially diluted drug (at a 1:1 ratio). The mixture of drug and calcitriol was incubated for at least 15 minutes, and was added to the cells (at a ratio of 100 µL to 100 µL). Thus, the final calcitriol concentration was 0.1 µg/mL.

The assay was carried out according to the previously described method in order to provide consistency and allow direct comparison. The result was based upon measurement of the total number of viable cells. The results (not shown) indicate that calcitriol pretreatment was not necessary for the chemoprotective effect on the cell cultures. The results were nearly identical between the pre-treatment group and the simultaneous treatment group. Thus, a topical application of calcitriol could be applied at the same time as the systemic delivery of the chemotherapy. A staged application is not required.

### Example 9, Protection from Chemotherapy-Induced Alopecia (CIA) by a Novel Calcitriol Formulation

Alopecia is one of the most distressing side-effects of chemotherapy, for which there is no current therapeutic intervention. The neonatal rat has been demonstrated to be an excellent model in which to study Chemotherapy-Induced Alopecia (CIA), since the anagen hair follicle pattern is similar to that of humans.

In the present study, the secosteroid calcitriol (USP grade) was delivered in a topical formulation (40% (w/w) propylene glycol, USP; and 60% (w/w) dehydrated alcohol, 200 proof, undenatured USP) to treat / prevent CIA, in a dose and time-dependent manner.

Specifically, Long Evans and Sprague Dawley rats with pups were purchased from Harlan Laboratories, Inc. They were housed and fed according to applicable animal handling rules and regulations. Pups were allowed to acclimate for 48 hours prior to the start of experiments. The secosteroid calcitriol formulation (supra) or vehicle control (no calcitriol) was applied topically over the head and neck area daily, starting on day 5 for 6 consecutive days. Rats were isolated from their littermates and mother for 6-hour periods of time. Subsequently, the treated area was cleaned with soap and water and pups were returned to their litters. On day 13, rats either received etoposide (1.5 mg/kg daily for 3 days) or cyclophosphamide (CTX) (37.5 mg/kg once) or combination cyclophosphamide (35 mg/kg once) and doxorubicin (2.5 mg/kg daily for 3 days). All chemotherapies were purchased from Sigma and were given intraperitoneally (i.p.) in a total volume of 0.1 mL. Alopecia was recorded 10 days after the last dose of chemotherapy.

For experiments in which rats were transplanted with chloroleukemia, on Day 5 after birth, rats were randomly divided into three groups of 45 each. All rats received 1 × 105 chloroleukemic cell line MIAC51 (i.p.) in 0.1 mL of serum free (SF) RPMI. MIAC51 were cultured in RPMI 1640 supplemented with L-glutamine and 10% fetal bovine serum at 37°C in a 5% CO₂, 100% humidity incubator. Cells were grown to 50% confluency (1.5 × 106 mL) collected in 50 mL conical tubes, centrifuged at 600g × 10 min. at room temperature and resuspended in SF-RPMI at a concentration of 1 × 106 /mL. Group 1 rats received no further treatment. Group 2 rats received topical vehicle and CTX on day 13. Group 3 rats received the topical calcitriol formulation (0.1µg) and CTX on day 13. Topical applications were performed as described above.

On day 23 after birth, a sample of blood was taken from all rats and differentials performed. Rats with leukemia were sacrificed, rats without leukemia were kept and a second differential performed on day 31, at any point if leukemia was detected, animals were sacrificed by CO₂ asphyxiation.

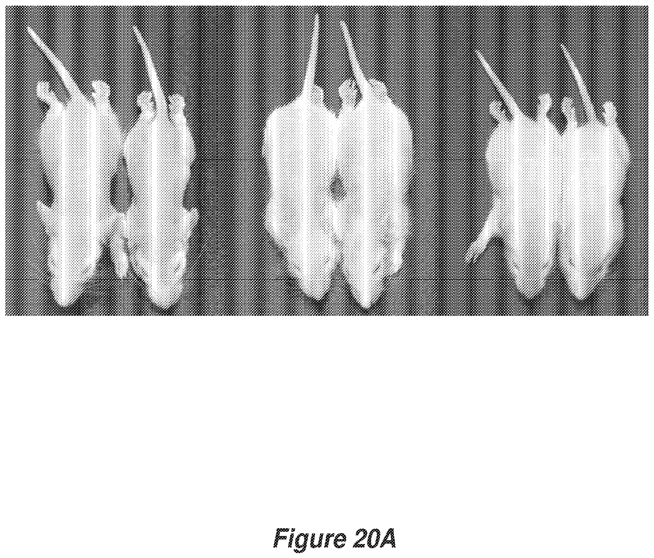

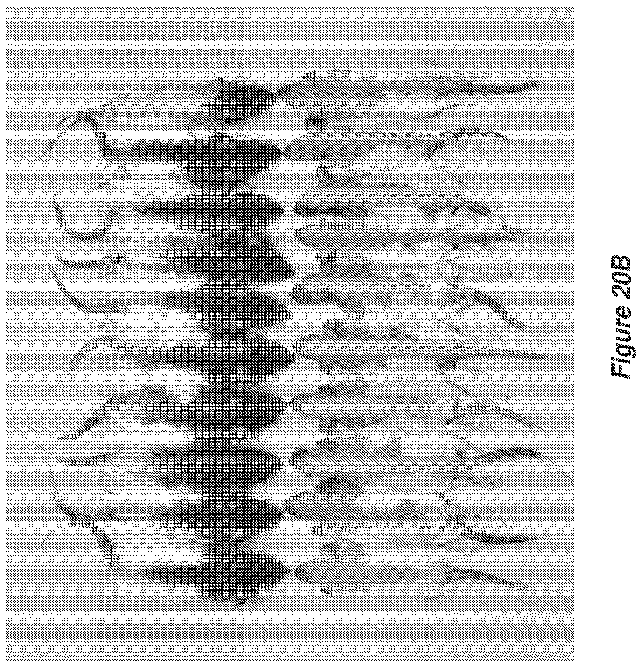

Results demonstrated that full body alopecia was observed in the group that received etoposide. In contrast, in the rats treated with 0.1 µg of calcitriol for 6 hours, partial localized protection was observed in all the animals. In the group receiving 0.3 µg calcitriol, total body protection was achieved. See **Figures 20A** and **20B****.**

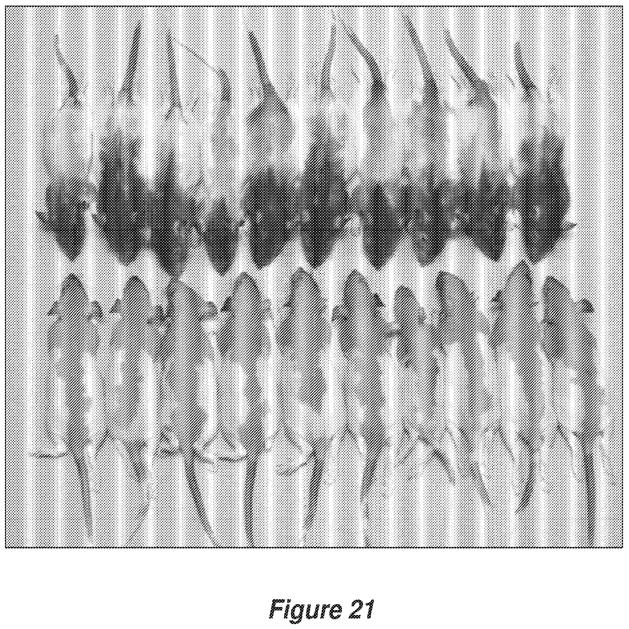

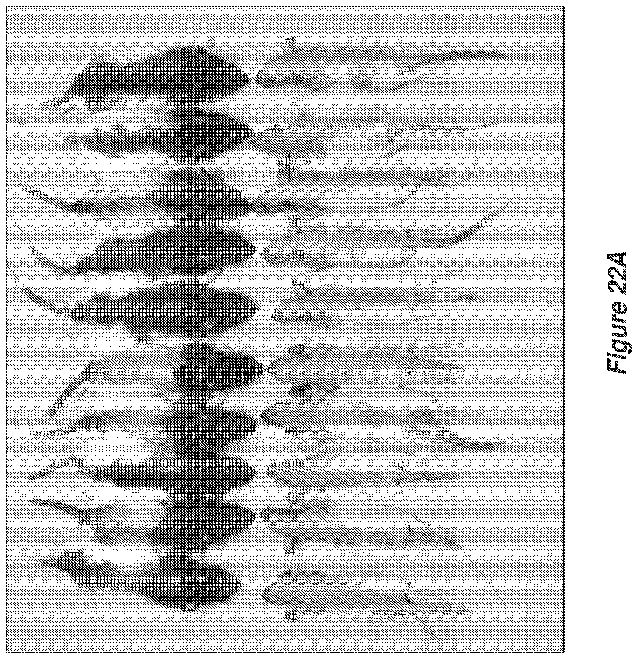

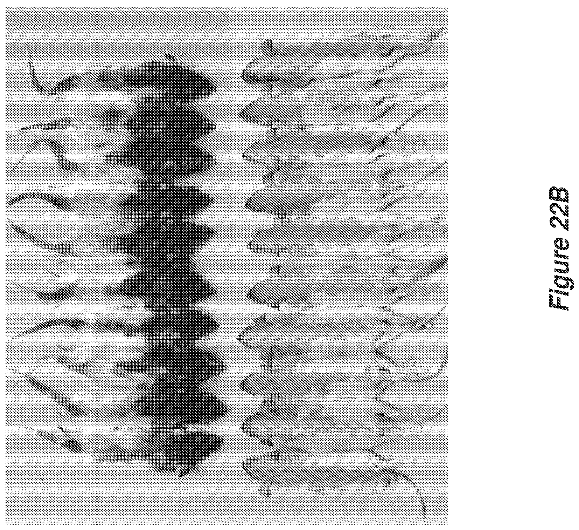

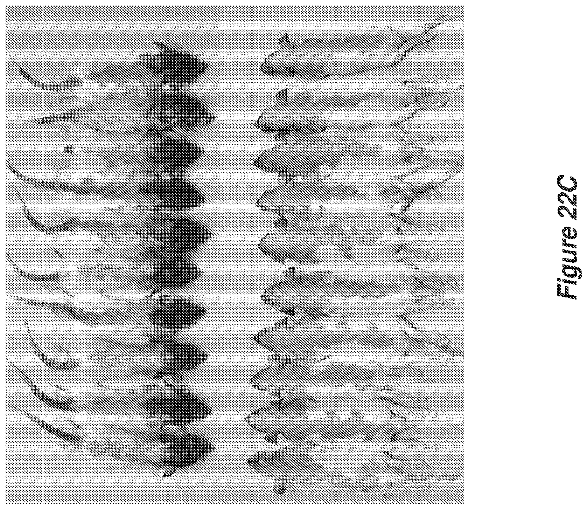

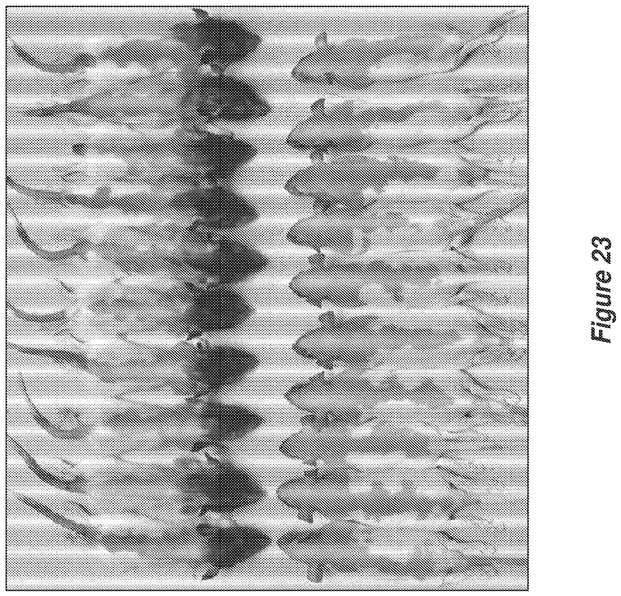

In the group that received cyclophosphamide, control rats became totally alopecic, while the rats that received 0.1 µg calcitriol achieved similar protection as observed with etoposide. Likewise, administration of 0.3 µg calcitriol resulted in full body protection in cyclophosphamide-treated rats. See **Figure 21****.** Similar results using other chemotherapy or combination chemotherapy regimens are shown in **Figures 22A****,** **22B****,** **22C** and **23****.**

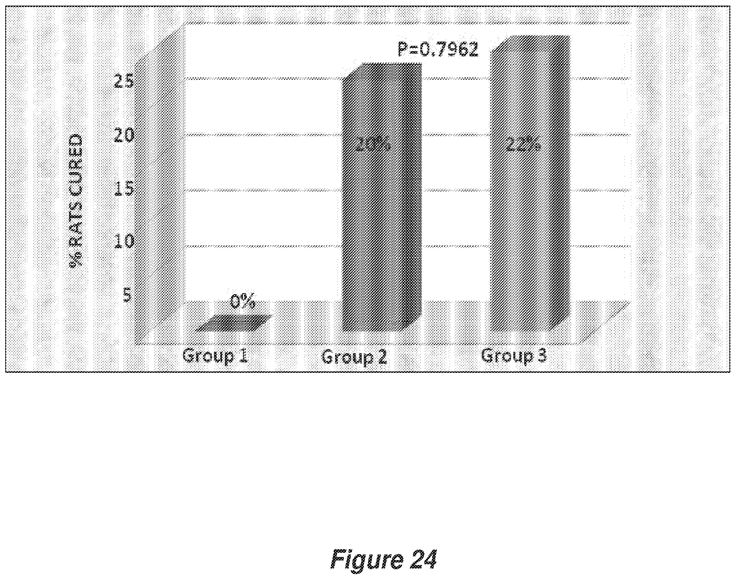

In a separate experiment in which rats were transplanted with chloroleukemia, preliminary results have not shown protection of the cancer cells from cyclophophamide by the topical application of calcitriol. See **Figure 24****.**

In conclusion, pretreatment with calcitriol in the subject formulation offered protection against CIA without protecting cancer cells. Topical calcitriol prevented CIA, in a dose dependent manner, from CIA induced by single as well as combination chemotherapy. In addition, topical calcitriol prevented CIA while not protecting the cancer cells from the cytotoxic effects of chemotherapy.

### Example 10. Protection of CIA by Topical Calcitriol in Chloroleukemic Rats Receiving Multi-Chemotherapy Regimens

This study verifies the protective effect of the topical calcitriol solution in an animal model of multi-course chemotherapy-induced alopecia. The rats used in the study bear MIAC51, a rat chloroleukemia cell line developed by gastric instillation of 20-methylcolanthrene and subsequent injection of the chloroleukemic cells into rat neonates. The MIAC51 cell line causes malignant myelogenous leukemia with features of human chloroleukemia (leukemia, leukemic ascites and chloroma formation). See Jimenez et al., Science 238: 1278-1280 (1987).

To date, there is no effective *in vitro* or non-vertebrate model to test chemotherapy-induced alopecia (CIA). Amongst the most used models, the neonatal rat developed by Jimenez *et al.* has demonstrated a direct correlation with human (Int J Cancer 1996; 65: 97-103). Subsequently, a rat model was developed in which a second anagen stage can be induced by clipping hair and thereby allow for testing multiple courses of chemotherapy. This model can be used to test frequently used alopecic chemotherapies, including cyclophosphamide, doxorubicin, paclitaxel, etoposide, and cytarabine, and combinations thereof.

When testing protective agents for chemotherapy-induced alopecia, it is paramount to determine whether the test article will protect the hair follicles and also the cancer cells from the chemotherapy and/or interfere with therapy. The neonatal rat model of leukemia, developed by Jimenez *et al.,* provides an opportunity to simultaneously test any effect of the vitamin D compound on the development of leukemia, the treatment of leukemia, potential interaction with chemotherapeutic agents, and the effect of the vitamin D compound on prevention of chemo-induced alopecia. This model also answers the question of whether multiple cycles of the test agent in the same animals will result in the protection of hair follicles multiple times. In addition, by using the pigmented Long Evans rat, the study also allows the determination of whether the test agent protects hair color.

The calcitriol formulation is a clear, anhydrous liquid containing USP-grade calcitriol in a vehicle containing USP-grade propylene glycol (40% w/w) and anhydrous absolute ethanol, 200 proof (60%w/w). The concentration of calcitriol in these studies is ~ 0.2 µg/100 µL (2 µg/mL). The test article is received on ice, and is immediately stored at 4-5 °C upon arrival. The lot will then be subdivided into 4.5 mL tubes while being maintained on ice. Since animal groups will be no smaller than 40 per variable, each 4.5 mL units of the test article will be packaged in a polypropylene tube at 4-5°C with the lot number. The 4.5 mL tubes of test article will be kept in dark boxes and only the amount needed per experiment will be taken out of the refrigerator. A sample of test article packaged in 4.5 mL tube will be assayed at a regular interval to determine calcitriol levels. At the time of the experiments, tubes will be kept on ice while rats are treated.

The vehicle is comprised of USP-grade propylene glycol (40% w/w) and USP-grade anhydrous and undenatured absolute ethanol, 200 proof (60% w/w). At the time of the experiments, the control vehicle is handled exactly as the test article.

Both the test article as well as the vehicle itself are tested. Each test group consists of 40 animals, which is statistically significant for this study. This number includes model attrition, and accounts for any eventuality which reduces the number of animals. All animals are injected with MIAC51 when they are 5 days of age. Five (5) chemotherapy regimens are tested: cyclophosphamide, cyclophosphamide/doxorubicin, cyclophosphamide/doxorubicin/cytarabine, cyclophosphamide/paclitaxel/etoposide and doxorubicin/paclitaxel/etoposide. Test groups are: no chemotherapy, chemotherapy alone, chemotherapy + vehicle, chemotherapy + test article = 160 animals per chemotherapy regimen. Therefore, the final estimated number of animals used are as follows: 5 combination chemotherapy regimens × 160 animals = 800 pups/rats. For experiments using the second anagen phase adult rat model, only animals that are cancer-free (*e.g.,* those who have survived chemotherapy) are used, while animals evidencing early signs of leukemia are euthanized.

*Culture of the Shay's Chloroleukemia MIAC51 cell line:* MIAC51 is cultured in a 5% CO₂ incubator with 100% humidity at 37 °C as previously described (Science 1987; 238:1278-80). Cells are grown in non-tissue culture-treated flasks (Falcon) in RPMI 1640 medium (Gibco Invitrogen, Carlsbad, CA) supplemented with L-glutamine and 10% fetal bovine serum (Gibco Invitrogen, Carlsbad, CA). Prior to the injection of cells into the animals, they are grown to 50% confluency and collected in conical tubes. Cells are then centrifuged at 600 g for 10 minutes at room temperature, and resuspended at a concentration 1 × 106 in RPMI 1640 without fetal bovine serum. The cell suspension is then transferred to 29 gauge (ga). 1/2 cc insulin syringes under sterile conditions.

*Injection of MIAC51:* All pups are five days old upon injection of MIAC51 and are manually restrained. The right leg is gently pulled and the area is cleaned with an alcohol swab. MIAC51 is then injected intraperitoneally. The needle, path and cells in the syringe are sterile and a fresh syringe is used for each injection. Development of early signs of leukemia are usually observed during Days 21-33. Therefore, blood smears are performed on Days 23 and 31. Only animals that are cancer-free are shaven on day 31, while the rest are euthanized.

Test and control article administration in the first anagen stage in the neonatal rat: Each litter is administered either vehicle or test article topically on the head and the neck area of approximately 2 cm². For 5- and 6-day old rats, 100 µL is applied in 4 aliquots of 25 µL 4 times to account for their smaller size. Test article or vehicle is applied with a calibrated micropipette using 200 µL sterile tips. Once test article or vehicle is on the surface of the head, it is rubbed in with gloved finger until fully absorbed. Immediately after, another aliquot is applied to the head and the process is repeated until 100 µL total test article or vehicle is applied. On 7-, 8- 9- and 10-day-old animals, 50 µL aliquots are applied twice. In older animals, 100 µL can be applied in one dose. Application of the testing article is applied to the head and neck, and rubbed in with a solvent-resistant nitrile glove for 10 seconds with the right index finger. The rationale behind this application regime is that at different ages, the saturation rate may differ, and the delivery of the test article or vehicle may also differ. Once the solution has completely penetrated the skin, pups will be maintained isolated in cages with specially designed isolated compartments for 6 hours. Pups are then washed with mild laboratory hand soap (Soft-Cide EC, VWR international) and carefully dried with paper towels.

*Administration of chemotherapy in the first anagen stage in the neonatal rat:* Forty pups receive each chemotherapy regime, 40 receive each chemotherapy regime and test article, and 40 receive each chemotherapy regime and vehicle. As a control, 40 animals do not receive chemotherapy. An average of the weights of each litter is obtained and is used to prepare a suitable concentration of chemotherapy. Chemotherapies are injected intraperitoneally in a volume of approximately 100 µL according to the weight of the animals using 29 ga. 1/2cc insulin syringes. When injecting, the right leg of each pup is gently pulled and the area is cleaned with an alcohol swab.

*Test and control article administration in the second anagen stage of the adult rat:* Survivors that have been demonstrated to be cancer-free on day 31 according to the hematological analysis of blood smears are manually restrained and shaven in the head and neck area (2-3 cm²). Nine days later, when rats are 40 days old to 45 days old inclusive, either vehicle or test article is applied to the head and the neck area. An amount of 100 µL is applied in one dose to the head and neck, and rubbed in with a solvent-resistant nitrile glove for 10 seconds with the right index finger. Once the solution has completely penetrated the skin, single rats are maintained isolated in cages. Rats are then washed with mild laboratory hand soap (Soft-Cide EC, VWR international) and carefully dried with paper towels.

*Administration of chemotherapy in the second anagen stage adult rat:* Each group receives 1 of 5 different chemotherapy regimens, starting on day 47 and ending on day 53 for those receiving combination cytarabine. An average of the weights is obtained and is used to prepare a suitable concentration of chemotherapy. Chemotherapies are injected intraperitoneally in a volume of approximately 100 µL according to the weight of the animals using 29 ga. 1/2cc insulin syringes. For administering chemotherapy, rats are manually restrained using no anesthesia. The injection area is cleaned with an alcohol swab.

*Route of administration:* Test article and vehicle are applied dermally. Chemotherapies are injected intraperitoneally.

*Frequency and duration of administration and dose levels and volumes:* The test article and vehicle are administered daily for 6 days for both the first and second anagen cycle. Test article contains a concentration of 2 µg/mL calcitriol in the propylene glycol/ethanol, and the vehicle contains only the propylene glycol/ethanol vehicle. Chemotherapies are given based on weight in a volume of approximately 100 µL intraperitoneally.

*Visual Observation and Grading of alopecia:* Total (head and neck) or complete body alopecia is graded using the following scale: 0 = No Alopecia; 1+ = 0-25% Alopecia; 2+ = 25-50% Alopecia; 3+ = 50-75% Alopecia; 4+ = 75-100% Alopecia. The visual observation scale is used daily to grade alopecia while performing routine cage observations. In addition, this scale complements the photographic documentation once the entire litter or the adult rats have lost the hair.

### Example 11. A Dermal Absorption Study: Topical Application of Calcitriol Solution in Gottingen Minipigs^{®} and Quantification of Calcitriol in ex vivo Porcine Skin

Pigs are frequently used in toxicity studies involving the dermal route of delivery because the skin of the pig is very similar to that of humans. Therefore, pigs were used in this study to evaluate the dermal tolerability and dermal penetration of the calcitriol topical formulation in Gottingen minipigs^{®}, following 7 days of dermal administration.

One treatment group of three male and three female Gottingen minipigs^{®} was administered the test or placebo article dermally to five separate administration sites at dose concentrations of 0 (placebo), 1, 3, 10, and 30 µg/mL. An additional treatment group of one male minipig was administered the test or placebo article dermally to two separate administration sites, at dose concentrations of 0 (placebo) and 100 µg/mL, respectively. The placebo or test article was administered at an application rate of 4 mg/cm² (equivalent to 144 mg in a 6 cm × 6 cm test area, or 166 µL of test solution, which contains the active ingredient at various concentrations and vehicle, per application site to both groups twice daily approximately 6 hours apart, for 7 days during the study.

Observations for morbidity, mortality, injury, and the availability of food and water were conducted twice daily for all animals. Clinical observations were conducted daily. Evaluation of skin reaction was conducted pretest and daily prior to dosing. Body weights were measured and recorded pretest and terminal (Day 7). Physical examinations were conducted at pretest. At study termination, necropsy examinations were performed and sections of treated and untreated skin were collected and preserved. Microscopic examination of each of the skin sites, as well as an untreated skin site near the treated sites, was conducted.

Results show that dermal administration of the calcitriol topical formulation at concentrations of 0, 1, 3, 10, 30, and 100 µg/mL to Gottingen minipigs^{®} was well tolerated. No effect of treatment was seen on survival, clinical findings, dermal irritation, body weights, macroscopic or microscopic examination of the skin at any of the treatment sites (data not shown). The data from the tissue distribution study indicate that calcitriol was measurable in most stratum corneum and other parts of the epidermal samples, but not in the dermal sample (with the single exception of the 100 µg/mL dose application to a single male minipig). In this set of experiments, males appeared to demonstrate greater calcitriol tissue levels than females. The clearest applied dose correlation to tissue level was observed in the epidermis, with a near linear increase with increasing calcitriol concentrations from 3 to 100 µg/mL.

Specifically, the placebo (a 40/60 mixture (w/w) of propylene glycol (USP) and ethanol (undenatured) anhydrous, 200 proof - U.S., USP), and the calcitriol topical formulation, were used at the pre-formulated concentrations of 1, 3, 10, 30, and 100 µg/g. The test article was administered neat (undiluted). Formulations of the placebo and test articles were dispensed for each required concentration once for daily use, and were stored at room temperature.

A total of three male and three female experimentally naive Gottingen minipigs^{®} (approximately 4 to 5 months of age) were received from Marshall BioResources, North Rose, New York. An additional male (approximately 4.5 months of age at receipt), was later transferred in from the stock colony. Using a simple randomization procedure, four male and three female animals (weighing 11.75 to 15.55 kg and 14.50 to 16.65 kg, respectively, at randomization) were assigned to the placebo and treatment groups. The placebo and test articles were administered dermally twice daily approximately 6 hours apart for 7 days during the study. The dose concentrations were 0, 1, 3, 10, 30, and 100 µg/mL, and administered at an application rate of 4 mg/cm² (equivalent to 144 mg or 166 µL of test solution). Prior to initiation of administration (Days -4 and -5 for Groups 1 and 2, respectively), the hair was clipped from the application sites using an electric clipper. Care was taken to avoid abrading the skin. The dorsal surface of each animal was divided into five application sites for Group 1 and two application sites for Group 2. Each application site was approximately 6 × 6 cm with at least a 2 cm space between each site. The placebo and test article formulations were uniformly applied over the specified application site with a glass stirring rod or appropriate instrument. Prior to dosing, the residual test article from the previous dose was gently removed using a soft paper towel (*i.e.*, WyPall^{®}) moistened with tap water.

At the end of the study, the skin was reflected from a ventral midline incision, and sections of treated and untreated skin were collected and preserved. Sections of each 6 × 6 cm dosing site were first thoroughly surface washed with a mild soap and water mixture (*e.g.,* 1% Ivory Soap in water or equivalent) to remove any residual topical test formulation. The washed skin sections were then wiped clean with ethanol, and were excised down to and including the adipose layer. If the area to be excised is larger than the dosed area, the dosed area was demarked with indelible ink to delineate the skin area that was dosed. The 1.5 cm × 1.5 cm sections were laid flat, wrapped in two layers of Saran wrap (or equivalent) and flash frozen in liquid nitrogen. The samples were stored at -70°C and shipped on dry ice *via* overnight courier for analysis. Each skin section was identified as appropriate (*e.g.,* animal identification, study number, date, etc.).

Upon arrival at the analysis site, skin sections were placed in water tight plastic bag and thawed by emersion in warm water (~30°C - 35°C). Each skin section was gently rinsed with distilled de-ionized water to remove any residual test article and blood. All subcutaneous tissue (*e.g.,* adipose) was removed by manual scalpel ablation. Within the central region of the dosed area, four individual 1 cm² circles (replicates) were demarked, and each site was subsequently identified and the actual area recorded. The replicate test sites were then excised from the skin sheet using a 1 cm² punch. The skin sections were weighed and the weight recorded. Each replicate demarcated area was tape stripped (Transpore^{™}, 3M) sufficient times (~10 - ~20) until approximately 10% - 25% of the area's surface demonstrated glistening. This process removed the stratum corneum and any residual surface dose.

Following tape stripping, the skin was separated into epidermis (sans stratum corneum, simply referred to herein after as "epidermis") and dermis by heat exposure to 60°C for approximately 1-1.5 minutes. The skin layers were then teased apart using fine-tipped forceps or scalpel. The epidermis and dermis were weighed and the weight recorded.

For extraction, all skin samples were extracted in 1 mL of absolute ethanol (Sigma-Aldrich, USP/NF Grade). Tape strips were extracted in 5 mL acetonitrile (EMD, HPLC Grade). All extractions were conducted at room temperature for approximately 24 hours. An amount of 500 µL of the tape strip extract was dried by vacuum centrifugation and reconstituted in 100 µL absolute acetonitrile. The epidermal extract was also dried and reconstituted in 100 µL 80:20 ethanol:water.

Quantification of calcitriol was by reverse phase High Performance Liquid Chromatography (HPLC) with ultra-violet and mass spectroscopy detectors. Lower limit of detection is estimated at 0.4 ng/mL.

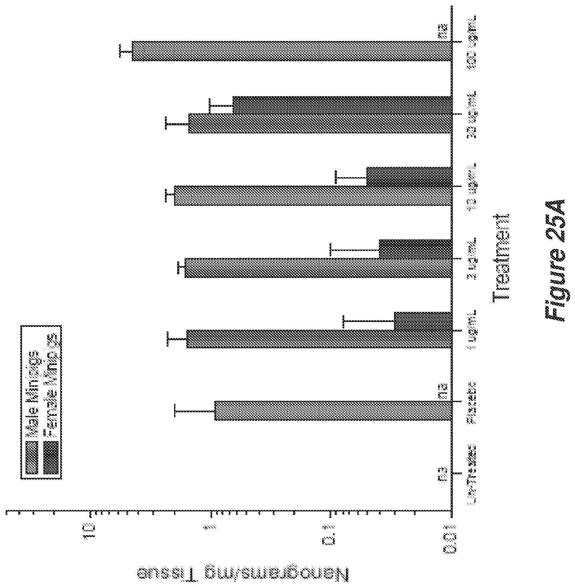

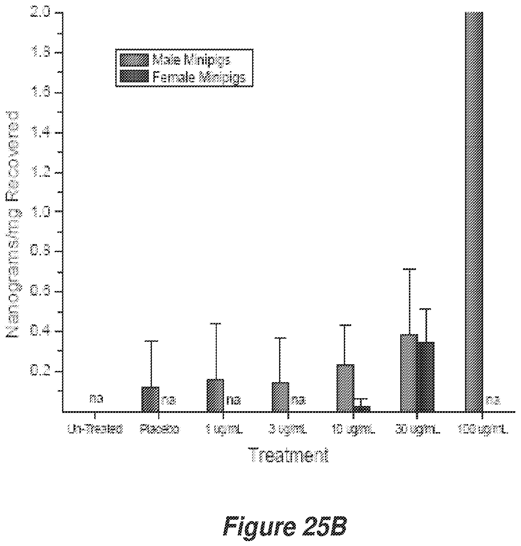

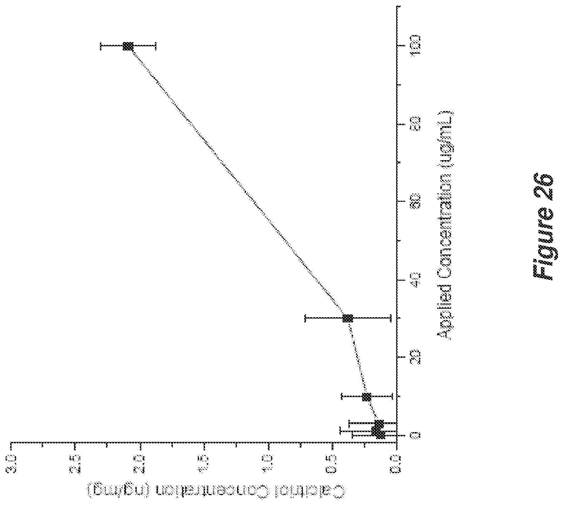

The results for the quantification of calcitriol, from stratum corneum (tape strips), epidermis and dermis are summarized in Tables 11-1 to 11-4. **Figures 25A** and **25B** illustrate the levels in the stratum corneum and epidermis, respectively, and **Figure 26** illustrates the epidermal levels in the males only. Stratum corneum data are provided in two different units, ng/cm², to reflect the amount of calcitriol recovered in the tape stripped samples as a function of the sample area, and as estimated µg/mg tissue. However, the concentration reported, as µg/mg, is determined by the differential total sample weight before layer separation minus the weights of the epidermis and dermis for that sample (rather than by actual weight due to its adherence to the tape strips). Epidermal and dermal samples are reported as tissue concentration (ng/mg) using the amount measured from the sample divided by actual wet weight of the skin layer.

**Table 11-1 Stratum Corneum (ng/cm²) Mean ± SD of Calcitriol Recovered (n = Number of Animals [4 replicates / animal])**

| **Treatment** | **Male Minipigs** | **Female Minipigs** |
|---|---|---|
| Untreated | 0 ± 0* (1) | na |
| Placebo | 30.2± 35.0 (4) | 0 ± 0 (3) |
| 1 µg/mL | 56.1 ± 11.4 (3) | 0.76 ± 1.3 (3) |
| 3 µg/mL | 62.4 ± 7.91 (3) | 1.12 ± 1.49(3) |
| 10 µg/mL | 59.6 ± 14.1 (3) | 1.65 ± 1.80 (3) |
| 30 µg/mL | 54.6 ± 32.5 (3) | 20.2 ± 11.7 (3) |
| 100 µg/mL | 118.1 ± 11.4 (1) | na |

| | | |
|---|---|---|
| • Zeros indicate results to be below the Lower Limit of Defection. • na = not applicable | | |

**Table 11-2 Estimated Stratum Corneum (ng/mg) Mean ± SD of Calcitriol Recovered (n = Number of Animals [4 replicates / animal])**

| **Treatment** | **Male Minipigs** | **Female Minipigs** |
|---|---|---|
| Untreated | 0 ± 0* (1) | na |
| Placebo | 0.92 ± 1.07 (4) | 0 ± 0 (3) |
| 1 µg/mL | 1.54 ± 0.79 (3) | 0.03 ± 0.05 (3) |
| 3 µg/mL | 1.63 ± 0.25 (3) | 0.04 ± 0.06 (3) |
| 10 µg/mL | 2.02 ± 0.39 (3) | 0.05 ± 0.04 (3) |
| 30 µg/mL | 1.51 ± 0.89 (3) | 0.64 ± 0.37 (3) |
| 100 µg/mL | 4.52 ± 1.21 (1) | na |

| | | |
|---|---|---|
| • Zeros indicate results to be below the Lower Limit of Detection. • na = not applicable | | |

**Table 11-3 Epidermis (ng/mg) Mean ± SD of Calcitriol Recovered (n = Number of Animals [4 replicates / animal])**

| **Treatment** | **Male Minipigs** | **Female Minipigs** |
|---|---|---|
| Untreated | 0 ± 0* (1) | na |
| Placebo | 0.12 ± 0.23 (4) | 0 ± 0 (3) |
| 1 µg/mL | 0.16 ± 0.28 (3) | 0 ± 0 (3) |
| 3 µg/mL | 0.14 ± 0.23 (3) | 0 ± 0 (3) |
| 10 µg/mL | 0.23 ± 0.20 (3) | 0.02 ± 0.04 (3) |
| 30 µg/mL | 0.38 ± 0.33 (3) | 0.34 ± 0.24 (3) |
| 100 µg/mL | 2.09 ± 1.0 (1) | na |

| | | |
|---|---|---|
| • Zeros indicate results to be below the Lower Limit of Detection • na = not applicable | | |

**Table 11-4 Dermis (ng/mg) Mean ± SD of Calcitriol Recovered (n = Number of Animals [4 replicates / animal])**

| **Treatment** | **Male Minipigs** | **Female Minipigs** |
|---|---|---|
| Untreated | 0.08 ± 0.01 (1) | na |
| Placebo | 0.02 ± 0.03 (4) | 0 ± 0* (3) |
| 1 µg/mL | 0 ± 0 (3) | 0 ± 0 (3) |
| 3 µg/mL | 0 ± 0 (3) | 0 ± 0 (3) |
| 10 µg/mL | 0 ± 0 (3) | a ± 0 (3) |
| 30 µg/mL | 0 ± 0 (3) | 0 ± 0 (3) |
| 100 µg/mL | 0.13 ± 0.04 (1) | na |

| | | |
|---|---|---|
| • Zeros indicate results to be below the Lower Limit of Detection. • na = not applicable | | |

The data indicate that calcitriol was measurable in most stratum corneum and epidermal samples, but not in the dermal samples (with the single exception of the 100 µg/mL dose application to a single male minipig). This is consistent with the results obtained in Franz human skin finite dose model described above in Example 1.

Across tissue samples evaluated, male minipigs appeared to demonstrate, in general, greater calcitriol tissue levels than female minipigs.

The highest concentrations of calcitriol were observed to be in the stratum corneum. Though the stratum corneum content is an estimated value, its higher concentration may reflect the presence of calcitriol deep in the pores of the skin, not removed by the surface wash process, or could be attributable to the solubility of calcitriol in the very lipophilic matrix of the stratum corneum.

The clearest applied dose correlation to tissue level, however, was observed in the epidermis with a near linear increase in calcitriol concentrations from 3 to 100 µg/mL applications.

### Example 12. A Topical Solution Study in Chloroleukemic Rats Receiving Multi-Course Chemotherapy

Long Evans Rats (Harlan Laboratories, Inc) were 3 days old upon arrival. The weight of the animals was obtained upon arrival and every day until the conclusion of the experiments using an electronic scale (American Scientific Products TL 410s). Rats were housed for two days prior to the beginning of experiments. Animals were then randomized in four groups. All rats received MIAC51 as described below.
▪ Group 1 (n=27) received no further treatment.
▪ Group 2 (n=40) received chemotherapy only.
▪ Group 3 (n=40) received chemotherapy and topical vehicle as describe below.
▪ Group 4, (n=40) received chemotherapy and topical calcitriol

Treatments were started on day 6 after birth. A 0.1mL amount of topical calcitriol was applied topically on the top of head and neck of the rats. For the first anagen cycle, on days 6 and 7, either vehicle or calcitriol was applied in a volume of 25 µL four times to avoid saturation. On days 8, 9, 10 and 11, a volume of 50 µL was applied twice. For the second anagen cycle, rats were treated with 0.1 mL of vehicle or calcitriol daily on days 40 to 45. Each application entailed rubbing an area of 2 cm² for 10 seconds with right index finger covered with a nitrile exam glove. After the completion of the treatments, each rat was individually separated for 6 hours. Subsequently each rat's head and back was washed with mild hand soap (Soft CIDE-EC from VWR International) and distilled water. Pups where then placed back with their mothers and taken back to the animal rooms. For the second anagen cycle, adult rats were placed back in their cages with their littermates and taken back to the animal rooms.

On day 5 after birth all rats received 1x105 MIAC51 intraperitoneally in 0.1 mL of serum free (SF) RPMI. MIAC51 were cultured in RPMI 1640 supplemented with L-glutamine and 10% fetal bovine serum at 370 in a 5% CO2, 100% humidity incubator. Cells were grown to 50% confluency (1.5x106 mL) collected in 50 mL conical tubes, centrifuged at 600 g for 10 minutes at room temperature and resuspended in SF-RPMI at a concentration of 1x106 /mL prior to injection.

On day 23 after birth, a blood sample was taken from all rats and differentials were performed. Rats with leukemia were sacrificed while rats without leukemia were used for further experiments. A second differential was performed on day 31, and leukemic animals were sacrificed. Surviving animals were shaved an area of 2cm² prior to the administration of the second set of vehicle or calcitriol treatment and a second course of chemotherapy was given 15 days later. In both the second and first anagen phase, alopecia was recorded ten days after chemotherapy treatment.

The extent of alopecia on each rat was determined by the following scale:
0 = no alopecia
1+ = 0-25% alopecia
2+ = 25-50% alopecia
3+ = 50-75% alopecia
4+ = 75-100% alopecia

### Experimental Compounds

The 2.3 µg/g calcitriol formulation was diluted with the vehicle (40% by weight propylene glycol and 60% by weight anhydrous 200 proof ethanol) to a final concentration of 2µg/mL. Vials of 1mL were subdivided and kept in the refrigerator at 4°C. For each experiment, one vial of 2.3 µg/g calcitriol and vehicle were taken out and placed on ice during the experimental procedure. Unused preparations were disposed of.

### A. Cyclophosphamide Alone

### Administration of Chemotherapy

Young rats: On day 13, all rats received cyclophosphamide (CTX) (Sigma Aldrich, Lot #068k1131) 37.5 mg/kg intraperitoneally using a ½ cc insulin syringe 29G ½" (B-D) in a total volume of 0.1 mL of H₂O/mannitol mixture.

Adult rats: For the second course of chemotherapy, 150 mg/kg cyclophosphamide was administered to 47-day old rats to anesthetized (50 mg/kg ketamine/5 mg/kg xylazine) intraperitoneally using a ½ cc insulin syringe 29G ½" (B-D) in a total volume of 0.1 mL of H₂O/mannitol mixture.

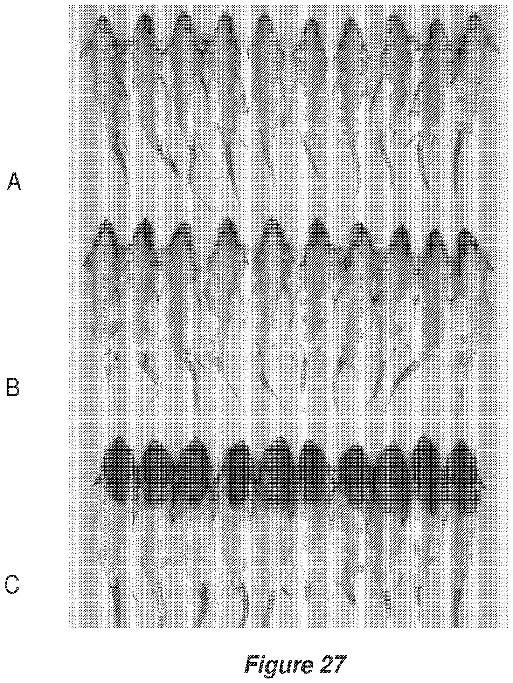

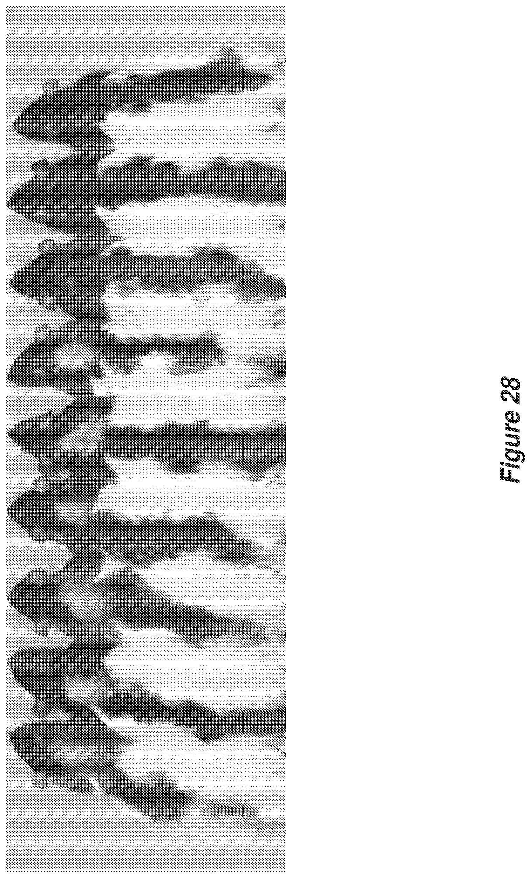

Results are seen in Tables 12-1 and 12-2. Specifically, after the first round of chemotherapy (Table 12-1 and **Figure 27****),** all rats receiving cyclophosphamide alone or cyclophosphamide in combination with the vehicle had severe alopecia (+4). In contrast, all rats that received cyclophosphamide in combination with calcitriol did not exhibit any signs of alopecia, similar to the control group. Similar results were obtained after the second round of chemotherapy, as shown in Table 12-2 (see also **Figure 28****).**

**Table 12-1. Extent of Alopecia in Rats Treated with Cyclophosphamide (CTX) after First Round of Chemotherapy**

| GROUP | ALOPECIA | | | | | | | |
|---|---|---|---|---|---|---|---|---|
| | 0 | 1+ | 2+ | 3+ | 4+ | Total | Groups / Prob. | Groups / Prob. |
| 1. Control (No chemotherapy) | 27 | | | | | 27 | 1 vs2 p<0.01 | 2 vs 3 p= 1.000 |
| 2. CTX | | | | | 40 | 40 | 1 vs 3 p<0.01 | 2 vs 4 p<0.01 |
| 3. CTX + Vehicle | | | | | 40 | 40 | 1 vs 4 p= 1.000 | 3 vs 4 p<0.01 |
| 4. CTX + Calcitriol | 40 | | | | | 40 | | |

**Table 12-2. Extent of Alopecia in Rats Treated with Cyclophosphamide (CTX) after Second Round of Chemotherapy**

| GROUP | ALOPECIA | | | | | | |
|---|---|---|---|---|---|---|---|
| | 0 | 1+ | 2+ | 3+ | 4+ | Total | Groups / Prob. |
| 2. CTX alone | | | | | 8 | 8 | 2 vs 3 p= 1.000 |
| 3. CTX + Vehicle | | | | | 9 | 9 | 2 vs 4 p<0.01 |
| 4. CTX + Calcitriol | 10 | | | | | 10 | 3 vs 4 p<0.01 |

Further, this experiment indicated that the survival rate of the rats receiving the topical formulation of calcitriol was substantially similar to those rats receiving chemotherapy alone or in combination with the vehicle. As shown in Table 12-3, the survival rate of those animals treated with cyclophosphamide and the topical formulation of calcitriol (25%) was similar to those rats treated with cyclophosphamide alone (20%) and those rats treated with cyclophosphamide and vehicle (23%).

**Table 12-3. Survival Rate of Rats Treated with Cyclophosphamide (CTX) after Two Rounds of Chemotherapy**

| GROUP | | | | | |
|---|---|---|---|---|---|
| | Cured | % | Total | Groups Prob. | Groups Prob. |
| 1. Control (No chemotherapy) | 0 | 0 | 27 | | |
| 2. CTX | 8 | 20 | 40 | 1 vs 2 p < 0.01 | 2 vs 3 p= 0.7846 |
| 3. CTX + Vehicle | 3 | 23 | 40 | 1 vs 3 p < 0.01 | 2 vs 4 p= 0.5923 |
| 4. CTX + Calcitriol | 10 | 25 | 40 | 1 vs 4 p < 0.01 | 3 vs 4 p= 0.7927 |
| TOTAL | 27 | 18 | 147 | | |

In summary, in the cyclophosphamide group, calcitriol offered 100% protection from CIA in both cycles and did not interfere with the cure rate which was in the range of 20-25%.

### B. Cyclophosphamide and Doxorubicin

### Administration of Chemotherapy

Young rats: On day 13, all rats received Cyclophosphamide (CTX) (Sigma Aldrich, Lot #068k1131) 37.5 mg/kg intraperitoneally using a ½ cc insulin syringe 29G ½" (B-D) in a total volume of 0.1 mL of H₂O/mannitol mixture. On days 13, 14, and 15 rats received doxorubicin hydrochloride (Sigma Aldrich, Lot # 038k1349) (ADM) 2.5 mg/kg I.P. in 0.1 mL distilled water.

Adult rats: For the second course of chemotherapy, 150 mg/kg cyclophosphamide to anesthetized (50 mg/kg ketamine/5 mg/kg xylazine) intraperitoneally using a ½ cc insulin syringe 29G ½" (B-D) in a total volume of 0.1 mL of H₂O/mannitol mixture on day 47. For the second course of chemotherapy, rats received 20 mg/kg ADM on days 47 to 49 as described above.

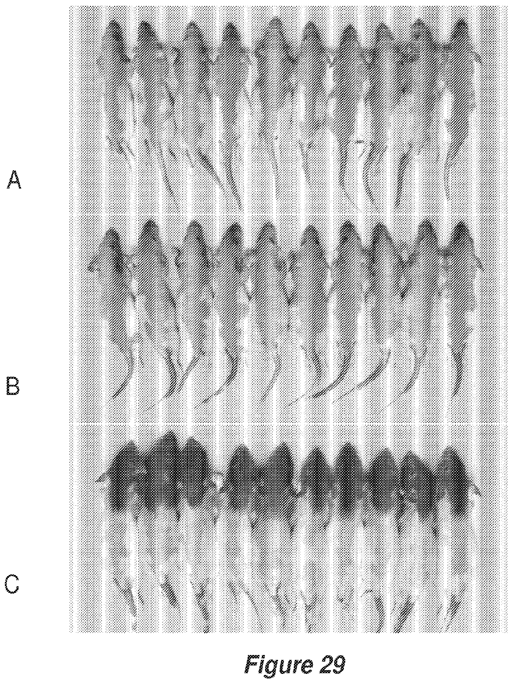

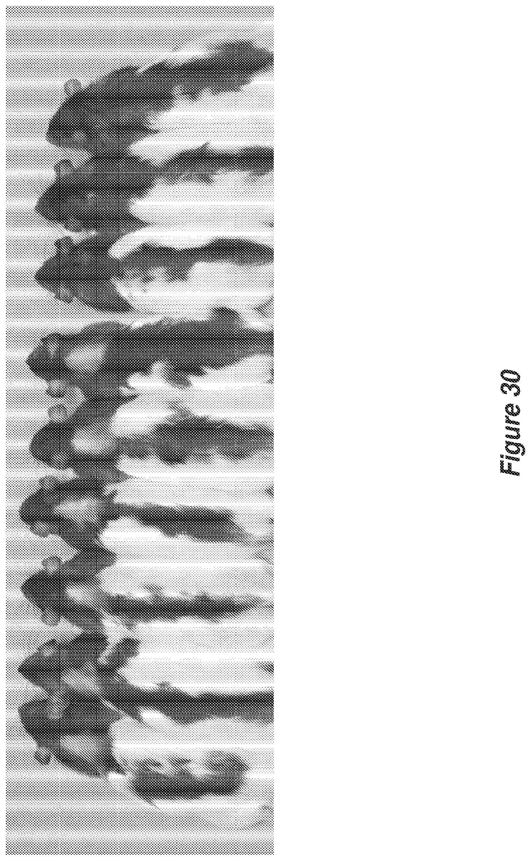

Results are seen in Tables 12-4 and 12-5. Specifically, after the first round of chemotherapy (Table 12-4 and **Figure 29****),** all rats receiving cyclophosphamide and doxorubicin alone or in combination with the vehicle had severe alopecia (+4). In contrast, all rats that received cyclophosphamide and doxorubicin in combination with calcitriol did not exhibit any signs of alopecia, similar to the control group. Similar results were obtained after the second round of chemotherapy, as shown in Table 12-5 (see also **Figure 30****).**

**Table 12-4. Extent of Alopecia in Rats Treated with Cyclophosphamide (CTX) and Doxorubicin (ADM) after First Round of Chemotherapy**

| GROUP | ALOPECIA | | | | | | | |
|---|---|---|---|---|---|---|---|---|
| | 0 | 1+ | 2+ | 3+ | 4+ | Total | Groups / Prob. | Groups / Prob. |
| 1. Control (No chemotherapy) | 40 | | | | | 40 | 1 vs 2 p<0.01 | 2 vs 3 p= 1.000 |
| 2. CTX + ADM | | | | | 40 | 40 | 1 vs 3 p<0.01 | 2 vs 4 p<0.01 |
| 3. CTX + ADM + Vehicle Car | | | | | 40 | 40 | 1 vs 4 p= 1.000 | 3 vs 4 p<0.01 |
| 4. CTX + ADM + Calcitriol | 40 | | | | | 40 | | |

**Table 12-5. Extent of Alopecia in Rats Treated with Cyclophosphamide (CTX) and Doxorubicin (ADM) after Second Round of Chemotherapy**

| GROUP | ALOPECIA | | | | | | |
|---|---|---|---|---|---|---|---|
| | 0 | 1+ | 2+ | 3+ | 4+ | Total | Groups / Prob. |
| 2. CTX + ADM | | | | | 21 | 21 | 2 vs 3 p= 1.000 |
| 3. CTX + ADM + Vehicle | | | | | 22 | 22 | 2 vs 4 p<0.01 |
| 4. CTX + ADM + Calcitriol | 20 | | | | | 20 | 3 vs 4 p<0.01 |

Further, this experiment indicated that the survival rate of the rats receiving the topical formulation of calcitriol was substantially similar to those rats receiving chemotherapy alone or in combination with the vehicle. As shown in Table 12-6, the survival rate of those animals treated with cyclophosphamide and doxorubicin in combination with the topical formulation of calcitriol (50%) was similar to those rats treated with chemotherapy alone (53%) and those rats treated with chemotherapy and vehicle (55%).

**Table 12-6. Survival Rate of Rats Treated with Cyclophosphamide (CTX) and Doxorubicin (ADM) after Two Rounds of Chemotherapy**

| GROUP | | | | | |
|---|---|---|---|---|---|
| | Cured | % | Total | Groups / Prob. | Groups / Prob. |
| 1. Control (No chemotherapy) | 0 | 0 | 40 | | |
| 2. CTX + ADM | 21 | 53 | 40 | 1 vs 2 p < 0.01 | 2 vs 3 p= 0.8225 |
| 3. CTX + ADM + Vehicle | 22 | 55 | 40 | 1 vs 3 p < 0.01 | 2 vs 4 p= 0.8230 |
| 4. CTX + ADM + Calcitriol | 20 | 50 | 40 | 1 vs 4 p < 0.01 | 3 vs 4 p= 0.9336 |
| TOTAL | 63 | 39 | 160 | | |

In summary, in the cyclophosphamide and doxorubicin group, calcitriol offered 100% protection from CIA in both cycles and did not interfere with the cure rate, which was in the range of 50-55%.

### C. Cyclophosphamide, Doxorubicin and Cytarabine

### Administration of Chemotherapy

Young Rats: On day 13, all rats received Cyclophosphamide (CTX) (Sigma Aldrich, Lot #068k1131) 30 mg/kg intraperitoneally using a ½ cc insulin syringe 29G ½" (B-D) in a total volume of 0.1 mL of H₂O/mannitol mixture. On days 13, 14, and 15 rats received 2.0 mg/kg doxorubicin hydrochloride (Sigma Aldrich, Lot # 038k1349) (ADM) intraperitoneally in 0.1 mL distilled water and on days 13-19, the ras received 50 mg/kg cytarabine.

Adult Rats: For the second course of chemotherapy, 100 mg/kg cyclophosphamide was administered to anesthetized rats (50 mg/kg ketamine/5 mg/kg xylazine) for one day, 20 mg/kg doxorubicin for three days and 100 mg/kg cytarabine for seven days.

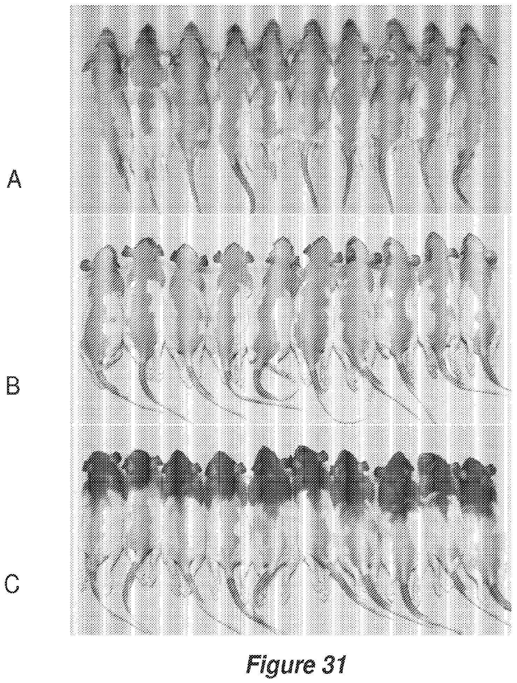

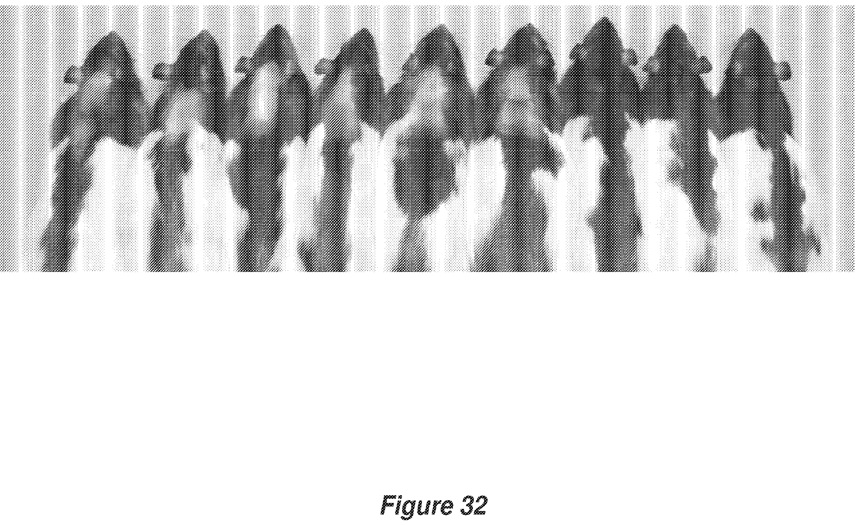

Results are seen in Tables 12-7 and 12-8. Specifically, after the first round of chemotherapy (Table 12-7 and **Figure 31****),** all rats receiving cyclophosphamide, doxorubicin and cytarabine alone or cyclophosphamide, doxorubicin and cytarabine in combination with the vehicle had severe alopecia (+4). In contrast, all rats that received cyclophosphamide, doxorubicin and cytarabine in combination with calcitriol did not exhibit any signs of alopecia, similar to the control group. Similar results were obtained after the second round of chemotherapy, as shown in Table 12-8 (see **Figure 32****).**

**Table 12-7. Extent of Alopecia in Rats Treated with Cyclophosphamide (CTX), Doxorubicin (ADM) and Cytarabine (ARA-C) after First Round of Chemotherapy**

| GROUP | ALOPECIA | | | | | | | |
|---|---|---|---|---|---|---|---|---|
| | 0 | 1+ | 2+ | 3+ | 4+ | Total | (Groups / Prob. | Groups / Prob. |
| 1. Control (No chemotherapy) | 40 | | | | | 40 | 1 vs 2 p<0.01 | 2 vs 3 p= 1.000 |
| 2. CTX + ADM + ARA-C | | | | | 40 | 40 | 1 vs 3 p<0.01 | 2 vs 4 p<0.01 |
| 3. CTX + ADM + ARA-C + Vehicle | | | | | 40 | 40 | 1 vs 4 p= 1.000 | 3 vs 4 p<0.01 |
| 4. CTX + ADM + ARA-C + Calcitriol | 40 | | | | | 40 | | |

**Table 12-8. Extent of Alopecia in Rats Treated with Cyclophosphamide (CTX), Doxorubicin (ADM) and Cytarabine (ARA-C) after Second Round of Chemotherapy**

| GROUP | ALOPECIA | | | | | | |
|---|---|---|---|---|---|---|---|
| | 0 | 1+ | 2+ | 3+ | 4+ | Total | Groups / Prob. |
| 2. CTX + ADM + ARA-C | | | | | 32 | 32 | 2 vs 3 p= 1.0000 |
| 3. CTX + ADM + ARA-C + Vehicle | | | | | 30 | 30 | 2 vs 4 p<0.01 |
| 4. CTX + ADM + ARA-C + Calcitriol | 31 | | | | | 31 | 3 vs 4 p<0.01 |

Further, this experiment indicated that the survival rate of the rats receiving the topical formulation of calcitriol was substantially similar to those rats receiving chemotherapy alone or in combination with the vehicle. As shown in Table 12-9, the survival rate of those animals treated with cyclophosphamide, doxorubicin and cytarabine in combination with the topical formulation of calcitriol (78%) was similar to those rats treated with chemotherapy alone (80%) and those rats treated with chemotherapy and vehicle (75%).

**Table 12-9. Survival Rate of Rats Treated with Cyclophosphamide (CTX), Doxorubicin (ADM) and Cytarabine (ARA-C) after Two Rounds of Chemotherapy**

| GROUP | | | | | |
|---|---|---|---|---|---|
| | Cured | % | Total | Groups / Prob. | Groups / Prob. |
| 1. Control (No chemotherapy) | 0 | 0 | 40 | | |
| 2. CTX + ADM + ARA-C | 32 | 80 | 40 | 1 vs 2 p < 0.01 | 2 vs 3 p= 0.5923 |
| 3. CTX + ADM + ARA-C + Vehicle | 30 | 75 | 40 | 1 vs 3 p < 0.01 | 2 vs 4 p= 0.5501 |
| 4. CTX + ADM + ARA-C + Calcitriol | 31 | 78 | 40 | 1 vs 4 p < 0.01 | 3 vs 4 p= 0.7927 |
| TOTAL | 93 | 58 | 160 | | |

In summary, in the cyclophosphamide, doxorubicin and cytarabine group, calcitriol offered 100% protection from CIA in both cycles and did not interfere with the cure rate, which was in the range of 75-80%.

### D. Cyclophosphamide, Paclitaxel and Etoposide

### Administration of Chemotherapy

Young rats: On day 13, all rats received Cyclophosphamide (CTX) (Sigma Aldrich, Lot #068k1131) 37.5 mg/kg intraperitoneally using a ½ cc insulin syringe 29G ½" (B-D) in a total volume of 0.1 mL of H₂O/mannitol mixture. On days 11 to 13, rats concomitantly received 2.5mg/kg paclitaxel (Taxol) in 0.1 mL dimethyl sulfoxide (Sigma Aldrich, Lot #078K1428) and 1.5 mg/kg etoposide (VP-16) (Sigma Aldrich, Lot #047K1162) diluted in special solvent (see Standard Operating Procedures) and HBSS.

Adult Rats: For the second course of chemotherapy, 150 mg/kg cyclophosphamide to anesthetized (50 mg/kg ketamine/5 mg/kg xylazine) intraperitoneally using a ½ cc insulin syringe 29G ½" (B-D) in a total volume of 0.1 mL of H₂O/mannitol mixture on day 47. For the second course of chemotherapy, rats received 10 mg/kg Taxol and 15 mg/kg VP-16 on days 45 to 48 as described above.

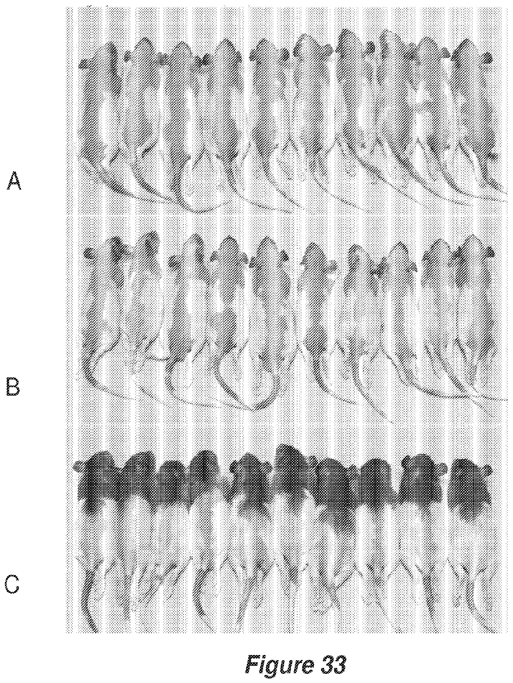

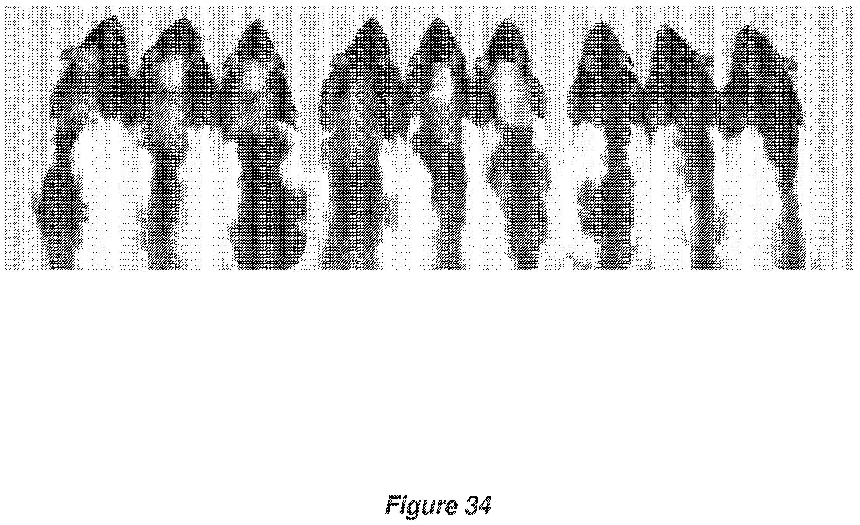

Results are seen in Tables 12-10 and 12-11. Specifically, after the first round of chemotherapy (Table 12-10 and **Figure 33****),** all rats receiving cyclophosphamide, paclitaxel and etoposide alone or cyclophosphamide, paclitaxel and etoposide in combination with the vehicle had severe alopecia (+4). In contrast, all rats that received cyclophosphamide, paclitaxel and etoposide in combination with calcitriol did not exhibit any signs of alopecia, similar to the control group. Similar results were obtained after the second round of chemotherapy, as shown in Table 12-11 (see also **Figure 34****).**

**Table 12-10. Extent of Alopecia in Rats Treated with Cyclophosphamide (CTX), Paclitaxel and Etoposide after First Round of Chemotherapy**

| GROUP | ALOPECIA | | | | | | | |
|---|---|---|---|---|---|---|---|---|
| | 0 | 1+ | 2+ | 3+ | 4+ | Total | Groups / Prob. | Groups / Prob. |
| 1. Control (No chemotherapy) | 40 | | | | | 40 | 1 vs 2 p<0.01 | 2 vs 3 p= 1.000 |
| 2. CTX + PACLITAXEL + ETOPOSIDE | | | | | 40 | 40 | 1 vs 3 p<0.01 | 2 vs 4 p<0.01 |
| 3. CTX + PACUTAXEL + ETOPOSIDE + Vehicle | | | | | 40 | 40 | 1 vs 4 p= 1.000 | 3 vs 4 p<0.01 |
| 4. CTX + PACLITAXEL + ETOPOSIDE + CALCITRIOL | 40 | | | | | 40 | | |

**Table 12-11. Extent of Alopecia in Rats Treated with Cyclophosphamide (CTX), Paclitaxel and Etoposide after Second Round of Chemotherapy**

| GROUP | ALOPECIA | | | | | | |
|---|---|---|---|---|---|---|---|
| | 0 | 1+ | 2+ | 3+ | 4+ | Total | Groups / Prob. |
| 2. CTX + PACLITAXEL + ETOPOSIDE | | | | | 33 | 33 | 2 vs 3 p= 1.0000 |
| 3. CTX + PACLITAXEL + ETOPOSIDE + Vehicle | | | | | 31 | 31 | 2 vs 4 p<0.01 |
| 4. CTX + PACLITAXEL + ETOPOSIDE +CALCITRIOL | 33 | | | | | 33 | 3 vs 4 p<0.01 |

Further, this experiment indicated that the survival rate of the rats receiving the topical formulation of calcitriol was substantially similar to those rats receiving chemotherapy alone or in combination with the vehicle. As shown in Table 12-12, the survival rate of those animals treated with cyclophosphamide, paclitaxel and etoposide in combination with the topical formulation of calcitriol (83%) was similar to those rats treated with chemotherapy alone (83%) and those rats treated with chemotherapy and vehicle (78%).

**Table 12-12. Survival Rate of Rats Treated with Cyclophosphamide (CTX), Paclitaxel and Etoposide after Two Rounds of Chemotherapy**

| GROUP | | | | | |
|---|---|---|---|---|---|
| | Cured | % | Total | Groups / Prob. | Groups 1 Prob. |
| 1. Control (No chemotherapy) | 0 | 0 | 40 | | |
| 2. CTX + PACLITAXEL + ETOPOSIDE | 33 | 83 | 40 | 1 vs 2 p < 0.01 | 2 vs 3 p= 0.5762 |
| 3. CTX + PACLITAXEL + ETOPOSIDE + Vehicle | 31 | 78 | 40 | 1 vs 3 p < 0.01 | 2 vs 4 p= 1.000 |
| 4. CTX + PACLITAXEL + ETOPOSIDE + CALCITRIOL | 33 | 83 | 40 | 1 vs 4 p < 0.01 | 3 vs 4 p= 0.5762 |

In summary, in the cyclophosphamide, paclitaxel and etoposide group, calcitriol offered 100% protection from CIA in both cycles and did not interfere with the cure rate which was in the range of 78-83%.

### E. Doxorubicin, Paclitaxel and Etoposide

### Administration of Chemotherapy

Young rats: On day 13 through 15, all rats received doxorubicin hydrochloride (Sigma Aldrich, Lot # 038k1349) (ADM) 2.5 mg/kg in 0.1 mL distilled water intraperitoneally using a ½ cc insulin syringe 29G ½" (B-D). Concomitantly, rats received 2.5 mg/kg paclitaxel (Taxol)_(Sigma Aldrich, Lot # 078k1428) and 1.5 mg/kg etoposide (VP-16) (Sigma Aldrich, Lot_# 047k1162).

Adult Rats: For the second course of chemotherapy, the above chemotherapies were started on day 47 through 49 on anesthetized rats (50 mg/kg ketamine/5 mg/kg xylazine) intraperitoneally using a ½ cc insulin syringe 29G ½" (B-D) in a total volume of 0.1 mL. Dosages for the second course were as follows: 20 mg/kg ADM, 10 mg/kg Taxol and 15 mg/kg VP-16.

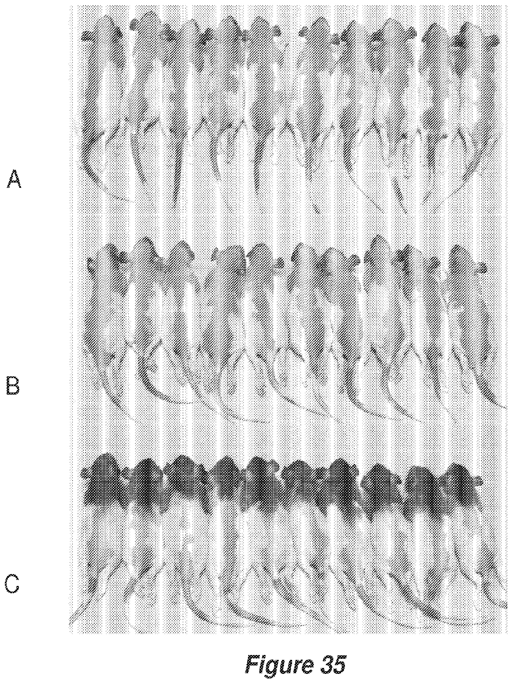

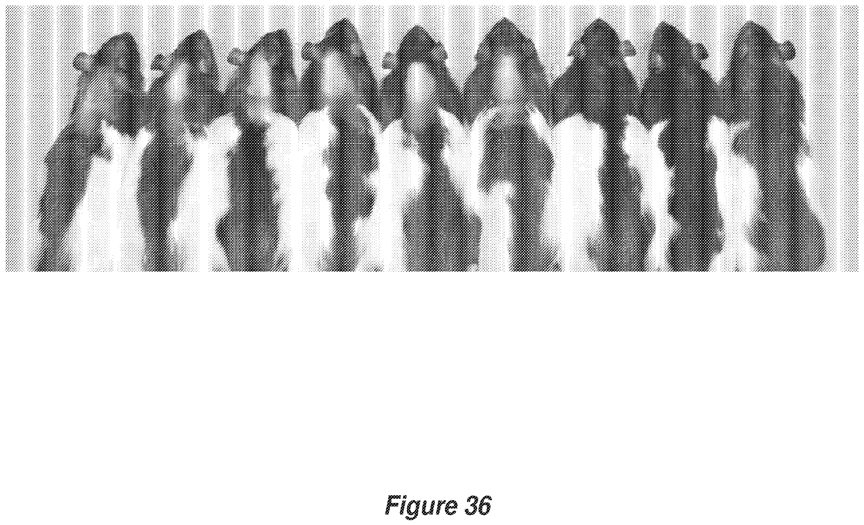

Results are seen in Tables 12-12 and 12-14. Specifically, after the first round of chemotherapy (Table 12-13 and **Figure 35****),** all rats receiving doxorubicin, paclitaxel and etoposide alone or doxorubicin, paclitaxel and etoposide in combination with the vehicle had severe alopecia (+4). In contrast, all rats that received doxorubicin, paclitaxel and etoposide in combination with calcitriol did not exhibit any signs of alopecia, similar to the control group. Similar results were obtained after the second round of chemotherapy, as shown in Table 12-14 (see also **Figure 36****).**

**Table 12-13. Extent of Alopecia in Rats Treated with Doxorubicin (ADM), Paclitaxel and Etoposide after First Round of Chemotherapy**

| GROUP | ALOPECIA | | | | | | | |
|---|---|---|---|---|---|---|---|---|
| | 0 | 1+ | 2+ | 3+ | 4+ | Total | Groups / Prob. | Groups / Prob. |
| 1. Control (No chemotherapy) | 40 | | | | | 40 | 1 vs a p<0.01 | 2 vs 3 p= 1.000 |
| 2. ADM + PACLITAXEL + ETOPOSIDE | | | | | 40 | 40 | 1 vs 3 p<0.01 | 2 vs 4 p<0.01 |
| 3. ADM + PACLITAXEL + ETOPOSIDE + Vehicle | | | | | 40 | 40 | 1 vs 4 p= 1.000 | 3 vs 4 p<0.01 |
| 4. ADM + PACLITAXEL + ETOPOSIDE + Calcitriol | 40 | | | | | 40 | | |

**Table 12-14. Extent of Alopecia in Rats Treated with Doxorubicin (ADM), Paclitaxel and Etoposide after Second Round of Chemotherapy**

| GROUP | ALOPECIA | | | | | | |
|---|---|---|---|---|---|---|---|
| | 0 | 1+ | 2+ | 3+ | 4+ | Total | Groups / Prob. |
| 3. ADM + PACLITAXEL + ETOPOSIDE | | | | | 32 | 32 | 2 vs 3 p= 1.0000 |
| 3. ADM + PACLITAXEL + ETOPOSIDE + Vehicle | | | | | 33 | 33 | Z vs 4 p<0.01 |
| 4. ADM + PACLITAXEL + ETOPOSIDE + Calcitriol | 32 | | | | | 32 | 3 vs 4 p<0.01 |

Further, this experiment indicated that the survival rate of the rats receiving the topical formulation of calcitriol was substantially similar to those rats receiving chemotherapy alone or in combination with the vehicle. As shown in Table 12-15, the survival rate of those animals treated with doxorubicin, paclitaxel and etoposide in combination with the topical formulation of calcitriol (80%) was similar to those rats treated with chemotherapy alone (80%) and those rats treated with chemotherapy and vehicle (83%).

**Table 12-15. Survival Rate of Rats Treated with Doxorubicin (ADM), Paclitaxel and Etoposide after Two Rounds of Chemotherapy**

| GROUP | | | | | |
|---|---|---|---|---|---|
| | Cured | % | Total | Groups / Prob. | Groups / Prob. |
| 1. Control (No chemotherapy) | 0 | 0 | 40 | | |
| 2. ADM + PACLITAXEL + ETOPOSIDE | 32 | 80 | 40 | 1 vs 2 p < 0.01 | 2 vs 3 p= 0.7745 |
| 3, ADM + PACLITAXEL + ETOPOSIDE + Vehicle | 33 | 83 | 40 | 1 vs 3 p < 0.01 | 2 vs 4 p= 0.1000 |
| 4. ADM + PACLITAXEL + ETOPOSIDE + Calcitriol | 32 | 80 | 40 | 1 vs 4 p < 0.01 | 3 vs 4 p= 0.7745 |

In summary, in the doxorubicin, paclitaxel and etoposide group, calcitriol offered 100% protection from CIA in both cycles and did not interfere with the cure rate which was in the range of 80-83%.

### Example 13. A 4-Week Dermal Toxicity Study of Topical Calcitriol in Gottingen Minipigs^{®}

*Control, Vehicle, and Test Article Preparation:* Fresh control article, 0.9% Sodium Chloride for Injection, USP, was dispensed for use on study weekly and was stored refrigerated. The vehicle, a 40/60 mixture by weight (w/w) of Propylene Glycol, USP and Ethanol (undenatured, anhydrous) 200 Proof USP, and the test article, containing Calcitriol, USP, with a specific gravity of 0.875, was used as received from and no adjustment was made for purity. The test article was received at concentrations of 5.07, 10.31, and 55.34 µg/mL. The test article was administered neat (undiluted). The vehicle and test article were dispensed for use on study weekly and stored refrigerated. On occasion, additional test material was dispensed as necessary during the course of the study.

*Administration:* Prior to administration, the hair was clipped from the back of the animal. The control animals had two test sites; site 1 was treated with the vehicle and site 2 with saline. Each site was 450 cm², bilaterally divided by the spine, and marked at the corners with indelible marker. The two test sites for the control group were evenly divided. Repeated clipping of the hair was done as necessary. Care was taken to avoid abrading the skin. The control article, vehicle, and test article were administered twice per day approximately 6 hours apart for 4 weeks (29 consecutive days) during the study dermally. The formulation was uniformly applied over the application site with a glass stirring rod or appropriate instrument. Any residual test material was gently removed prior to the next dose with a Wypall, wet with tap water. If necessary, sites were dried with a clean, dry Wypall. The dose administered to all animals was 1800 mg of the appropriate formulation. The dose concentrations were 5.07, 10.31, and 55.34 µg/mL and administered at a dose volume of 2.1 mL. The control article and vehicle were administered to the control group in the same manner as the treated groups. The dosing volume for the control animals was 1.0 mL of the vehicle and 0.9 mL of saline. Due to the severity of clinical signs observed, all animals at 55.34 µg/mL were not dosed on Day 23. Dosing resumed for all animals on Day 24.

*Results:* This study was conducted for to evaluate the potential sub chronic toxicity of a calcitriol topical solution, when administered twice daily *via* dermal application for 4 weeks. Three treatment groups of four animals/sex/group of Gottingen Minipig^{®} were administered the calcitriol topical solution at respective dose concentrations of 5.07, 10.31, and 55.34 µg/mL. One additional group of four animals/sex served as the control and received the vehicle, a 40/60 mixture by weight (w/w) of Propylene Glycol, USP and Ethanol (undenatured, anhydrous) 200 Proof USP, and the control article, 0.9% Sodium Chloride for Injection, USP. The calcitriol topical solution or vehicle was administered to all groups *via* dermal application, twice a day for 29 consecutive days, at a dose volume of 4 mg/cm² over a 450 cm² test site.

Observations for morbidity, mortality, injury, and the availability of food and water were conducted twice daily for all animals. Clinical observations were conducted weekly. Body weights were measured and recorded weekly. Dermal irritation scoring was done after each dose during Week 1 and then twice per week (after the second dose) during Weeks 2 through 4 for changes in the application site. Ophthalmoscopic examinations were conducted pretest and all survivors prior to terminal necropsy. Physical examinations were conducted pretest. Electrocardiographic examinations were conducted pretest, predose, and 1 to 2 hours post the first dose on Day 1 and during the last week of dosing. Blood and urine samples for clinical pathology evaluations were collected from all animals pretest and prior to the terminal necropsy. Blood samples for determination of the plasma concentrations of the test article were collected from all surviving animals at designated time points on Days 1 and 27. The toxicokinetic (TK) parameters were determined for the test article from concentration-time data in the test species. At study termination, necropsy examinations were performed, organ weights were recorded, and selected tissues were microscopically examined.

One male at the 55.34 µg/mL concentration was euthanized in extremis on Day 28 of the study. This animal was observed with decreased activity, inappetence, and tremors prior to euthanasia. The cause of the morbidity of this animal was considered to be the high calcium blood levels that were close to the lethal level. All remaining minipigs survived to their scheduled termination on Day 30 of the study. Decreased activity, inappetence, emesis, and tremors were observed in most minipigs at the 55.34 µg/mL concentration during Weeks 3 and 4 of the study. Mild irritation was observed in males and females at the 55.34 µg/mL concentration during the last week or two of the study. Mean body weights and body weight gains for the treated males and females at 5.07 and 10.31 µg/mL were comparable to controls. All males and females at the 55.34 µg/mL concentration lost a significant amount of body weight during the last 2 weeks of the study and the mean body weights were significantly lower in males and females during this time period.

No ophthalmoscopic abnormalities were observed in any of the animals at the pretest and terminal ophthalmoscopic examinations. The calcitriol topical solution did not cause qualitative electrocardiogram abnormalities, but there was a mild increase in the group mean heart rates at the terminal predose and postdose intervals. This increase in heart rate is undoubtedly related to the marked increase in calcium levels in these minipigs during the study. There were no other dose-related effects of the calcitriol topical solution on quantitative electrocardiogram parameters. No calcitriol topical solution-related hematology, coagulation or urinalysis alterations were observed in males or females at the terminal evaluation. Some clinical chemistry alterations were seen at the 55.34 µg/mL concentration, the most notable was the high calcium levels observed that were near the lethal level. The other changes seen were lower chloride values, and higher cholesterol, glucose, urea nitrogen, and triglyceride values.

Calcitriol topical solution-related macroscopic pathology findings were limited to the stomach mucosa of one female at the 55.34 µg/mL concentration consisting of a mild, irregular surface. Absolute and relative increased weight of the kidney and decreased weight of the thymus were seen in both sexes at the 55.34 µg/mL concentration compared to controls. Direct calcitriol topical solution-related microscopic findings were present in the bones, kidneys, heart, treated skin, thymus, and thyroid gland. In addition, direct calcitriol topical solution-related findings included multicentric vascular changes and multicentric mucosal mineralization. Indirect test articlerelated microscopic findings were noted in the pancreas. These microscopic changes were present in both genders and were limited to animals dosed at the 55.34 µg/mL concentration.

The microscopic changes of the femoral, sternal, and costal bones were limited to the diaphyseal cortical bone and to the bone cavity. They were characterized by osteodystrophy and by the deposition of basophilic matrix. The renal microscopic observations were characterized by mineralization, tubular degeneration/regeneration and by a subacute inflammation. The microscopic observations of the myocardium were myofiber mineralization, subacute inflammation and vascular changes. In addition, one male and one female had endocardial mineralization. Multicentric mucosal/epithelial mineralization was observed in decreasing order within the stomach mucosa, lungs, larynx, trachea, prostate gland, salivary mandibular gland, and within the urinary bladder. Calcitriol topical solution-related vascular changes were widespread and affected primarily small to medium-sized blood vessels. They were primarily observed within the heart and the bone cavity and sporadically in different organs/systems. The microscopic changes of the treated skin were characterized by epidermal hyperplasia and hyperkeratosis and perivascular mixed cell inflammation with the superficial dermis. The microscopic changes of the thymus, thyroid gland and pancreas were characterized by lymphoid depletion, follicular cell hypertrophy and hyperplasia and single cell necrosis respectively.

On the basis of the results of this study, the no-observed-adverse-effect-level (NOAEL) was considered to be 10.31 µg/mL based upon the clinical chemistry and microscopic changes seen at the 55.34 µg/mL concentration.

### Example 14. Preclinical Studies: Rat and Mouse Experiments

*Neonatal Rat model and PCA model.* Sprague Dawley rats were purchased from Charles River Laboratories (Wilmington, MA). C3H/HeJ mouse were purchased from Jackson Laboratories (Bar Harbour, ME).Rodents were housed and fed according to NIH guidelines. For experiments involving chemotherapy, cyclophosphamide was purchased from Mead Johnson (Evansville, IN) and Etoposide was obtained from Bristol-Myers (Evansville, IN). 1,25(OH)2D3 powder was a gift from Dr. Uskokovic (Hoffman-La Roche, Nutley, NJ). For experiments involving C3H/HeJ mice, 1,25(OH)2D3 was purchased from Sigma (St. Louis, MO).

*Topical application of 1,25(OH)2D3.* 1,25(OH)2D3 was dissolved in absolute ethanol and applied topically with an applicator. Control animals were similarly treated with vehicle only. Animals were then isolated for a period of 3 hours following which the treated area was washed with soap and water and dried. For CIA experiments, 1,25(OH)2D3 was given daily beginning on day 5 after birth and ending on day 10. For PCA experiments, 1,25(OH)2D3 was given daily starting on day 22, when the animals were completely alopecic, and ending on day 35. For CIA and PCA, the experimental group was treated with 0.2 µg of 1,25(OH)2D3 in 150 µL of absolute ethanol applied topically over the head and neck. In both instances, the control group received ethanol vehicle only. Cyclophosphamide (35 mg/kg) was given intraperitoneally for one day only. Etoposide (1.5 mg/kg) was given intraperitoneally for 3 days. Both chemotherapies were started at 11 days of age. Alopecia was recorded on the tenth day after the first dose of chemotherapy.

In the first experiment, protection from cyclophosphamide-induced alopecia was examined to evaluate the efficacy of the 1,25(OH)2D3. Rats were randomized into two groups often rats each. One group received vehicle control, while the other received 0.2 µg 1,25(OH)2D3. All 10 rats in the control group became totally alopecic by day 10 post-chemotherapy. In contrast, all animals pre-treated with 1,25(OH)2D3 did not develop alopecia. In the second experiment, 20, 22-day old rats with complete body alopecia (alopecia universalis) induced by cyclophosphamide previously administered were randomized into 2 groups of 10 each. In addition, 20, 22-day old rats with alopecia universalis induced by etoposide administration were also randomized into 2 groups of 10 animals each. One group of cyclophosphamide-and etoposide-induced alopecia received 1,25 (OH)2D3 whereas one group received vehicle control. Animals were observed until complete hair regrowth was obtained. In both control groups, 100% of animals exhibited full regrowth of hair by day 42. In contrast, in the rats treated with 1,25(OH)2D3 total body hair regrowth was delayed until day 50.

*C3H*/*HeJ Model of Abpecia Areata.* Retired breeders (8 months of age) were observed daily for the development of AA lesions. At 10 months of age, a group of 6 animals exhibiting localized foci of alopecia in the dorsal area were selected and were randomized into 2 groups of 3 animals each. One group received ethanol vehicle control whereas the other received 0.2 µg 1,25(OH)2D3 in the alopecic lesions for 15 days. Animals were observed for a total of 30 days. No regrowth of hair was observed in the control or experimental group.

Chloroleukemia was induced in 5-day-old Long Evans rats following a single intraperitoneal (IP) injection of MIAC51 cells (a rat chloroleukemia cell line). The effect of calcitriol (2 µg/mL) on hair growth was evaluated in the neonatal animals (exposure on Days 6 to 11) as well as adult animals (exposure on Days 40 to 45). Following topical application of calcitriol, both age groups of animals were then administered various chemotherapeutic agents (cyclophosphamide; cyclophosphamide and doxorubicin; cyclophosphamide, doxorubicin and cytosine beta-D arabinofurosamine; cyclophosphamide, paclitaxel and etoposide; and doxorubicin, paclitaxel and etoposide). In each of the experiments, calcitriol produced 100% protection from CIA after both the first and second chemotherapy cycles (*i.e.,* in neonatal as well as adult animals). CIA was unaffected in the other groups not treated with calcitriol (chloroleukemia controls, chemotherapeutic agent(s) alone, chemotherapeutic agent(s) plus vehicle). It was also observed that calcitriol did not affect the efficacy of the chemotherapy agents - the number of animals free from leukemia was similar in the chemotherapy group alone, the chemotherapy group plus vehicle and the chemotherapy group plus calcitriol topical solution.

A second study was conducted in 5-day-old Sprague-Dawley or Long Evans rats and the ability of calcitriol to protect against CIA was evaluated. CIA was administered topically at concentrations of 1, 2, or 3 µg/mL on Days 5 to 10 followed by administration of various chemotherapeutic agents (etoposide; cyclophosphamide; doxorubicin and cyclophosphamide; cytosine beta-D arabinofurosamine; cytosine beta-D arabinofurosamine and doxorubicin; and paclitaxel). In one experiment, MIAC51 cells were administered on Day 5 to produce chloroleukemia and the animals were then administered calcitriol plus cyclophosphamide. Protection from CIA was time-dependent: no protection occurred following a 1.5 hour exposure, but complete protection was observed following a 6-hour exposure. Calcitriol at all concentrations tested (*i.e.,* 1 to 3 µg/mL) provided 100% protection against CIA induced by all the chemotherapeutic agents tested when applied for 6 hours. In addition, it was noted that at 1 µg/mL hair growth was limited to the treated area (head and neck) whereas at 3 µg/mL hair growth was also observed on the dorsal region (untreated area) of the animals. Finally, administration of calcitriol did not affect the cure rate (or number of animals free from leukemia) associated with cyclophosphamide when chloroleukemia was induced.

Secondary pharmacodynamic effects of calcitriol are well understood and include changes in the bone, intestines, the immune system, and parathyroid.

*Conclusions.* The results presented herein strongly suggest that 1,25(OH)2D3 exerts a protective effect against chemotherapy-induced alopecia, but it does not treat alopecia itself. The rationale behind the preventive effect of 1,25(OH)2D3 in CIA is that healthy hair follicles can be arrested and thereby rendered resistant to the chemotherapy. However, in hair follicles that are already apoptotic, treatment with 1,25(OH)2D3 does not offer any benefit.

Findings in another animal study demonstrated a potential mechanism by which application of topical calcitriol may diminish the incidence of CIA. Topical treatment with calcitriol was demonstrated to significantly diminish the degree of follicular apoptosis induced by cyclophosphamide in C57BL/6 mice. Another experiment conducted with BALB/c mice, the animal model which allows investigating the hair follicles in the first adult anagen phase and evaluate gender differences, demonstrated that topical application of calcitriol for 5 days prior to intraperitoneal administration of cyclophosphamide significantly reduced the degree of CIA in a dose-dependent manner in male mice31. The protective effect of topical calcitriol was diminished in tumor-bearing male mice that were injected with EMT-6 murine breast tumor cells. In contrast, pre-treatment with topical calcitriol in tumor-bearing female mice injected with EMT-6 murine breast tumor cells had a more efficient protective effect against cyclophosphamide induced alopecia as compared to female mice without tumor and their tumor-bearing male counterparts. Histopathologic examination of post-mortem skin demonstrated marked reduction in morphological alterations induced by cyclophosphamide in hair follicles and a protective effect from cyclophosphamide-induced follicular damage. However, in this model, no significant differences in apoptosis staining pattern were noted between mice treated with topical calcitriol and those without exposure to the solution.

On the other hand, another animal study using the female C57BL/6 adolescent mice instead of a neonatal rat model, failed to demonstrate efficacy of topical calcitriol in prevention of cyclophosphamide-induced alopecia. Interestingly, however, the regrowth of pigmented hair shafts was significantly accelerated, enhanced, and quantitatively improved. Histopathologic investigations suggested that this may have been due to hair follicles favoring the "dystrophic catagen pathway" of response to chemical injury: follicular repair strategy allowing for the unusually fast reconstruction of a new, undamaged anagen hair bulb.

Multiple *in vivo* animal investigations failed to demonstrate the potential protective effect of calcitriol from cytotoxicity of chemotherapy on cancer cells. No statistically significant difference in the survival rate was seen between Sprague Dawley rats transplanted with chloroleukemia C51 cells pretreated with 0.2 µg of 1,25(OH)2D3 and those pretreated with an ethanol vehicle prior to cyclophosphamide treatment. More importantly, pretreatment with topical calcitriol of EMT-6 murine breast tumor-bearing male mice prior to intraperitoneal administration of cyclophosphamide resulted in the greater reduction in the rate of tumor growth as compared to mice treated with either agent alone, suggesting potential anti-tumor effects of calcitriol. Similarly, pretreatment with 2.5 µg of calcitriol 3 doses daily for 3 days, followed by administration of varying doses of paclitaxel of squamous cell carcinoma and human prostate cell carcinoma bearing mice resulted in significant tumor regression.

### Example 15, Skin Sensitization Study of Calcitriol in Guinea Pigs

*Induction phase.* A single group of 10 male and 10 female guinea pigs received an induction exposure to the test article (at the highest concentration determined in the range-finding screen that was well tolerated) *via* topical patch application for 6 hours. The induction exposure was repeated at the same skin site once a week for 2 weeks. The vehicle and positive control groups composed of 5 male and 5 female guinea pigs, each of which were exposed in the same manner as the treatment group to propylene glycol/anhydrous ethanol or hexylcinnamic aldehyde (a known mild to moderate skin-sensitizer), respectively. Skin reactions were scored and recorded at approximately 24 hours after patch removal during the induction phase. After the last induction exposure, the animals remained untreated for two weeks before the challenge exposure.

*Challenge phase.* The challenge exposure was conducted two weeks after the final topical induction. The test article was topically applied to the Vehicle Control and Test Group at the concentration determined to be non-irritating in the irritation screen. The Vehicle Control Group was also challenged with the Vehicle. The Positive Control Groups received the positive control article. The challenge application sites were scored at approximately 24 and 48 hours after patch removal. Under the conditions of this study, calcitriol at 5.7 µg/g was not determined to be a sensitizer.

### Example 16. Calcitriol Topical Solution Eye Irritation Study in Rabbits

This study was conducted to evaluate the potential ocular irritant and/or corrosive effects of calcitriol topical solution. One treatment group of three male New Zealand White Hra:(NZW)SPF rabbits were administered the test article at a respective dose level of 0.1 mL/animal once on Day 1 *via* the right eye. The left eye remained untreated and served as the control.

Observations for morbidity, mortality, injury, and the availability of food and water were conducted twice daily for all animals. Ocular observations and irritation scoring were conducted at 1, 24, 48, and 72 hours post-dose and on days 5, 8, 10, and 15. Following the 24 hours post-dose evaluation, a lukewarm water wash out was performed on each eye. Body weights were measured and recorded prior to dosing. At study termination, all animals were euthanized without further evaluation.

Irritation scores were present through Day 10. The most severe scores were generally limited to the 1 hour post-dose observation. Signs of ocular irritation were still present at 24 hours post-dose but with generally lower severity than at the 1 hour post-dose observation. By Day 3, all scoring had returned to zero with the exception of redness. Scores of 1 (some blood vessels definitely hyperemic) were observed through Day 10. All signs of ocular irritation were resolved by Day 15. Based on the results of the study, calcitriol topical solution caused moderate irritation of the eye which was mostly resolved by Day 3 and completely resolved by Day 15.

*Summary of animal studies.* The animal studies presented above, *e.g.* Examples 1, 9, 10, 11, 13, 14, 15, and 16, were conducted to characterize the effect of vitamin D compounds in alopecia and, in context of the present invention, the applicability of vitamin D compounds to preventing or mitigating CIA.

Both CIA and alopecia areata (AA) have been extensively studied due to the availability of animal models which closely mimic the diseases in the human. In this regard, it was previously demonstrated that alopecic chemotherapies (cytarabine, cyclophosphamide, doxorubicin, doxorubicin/cyclophosphamide, etoposide) induce alopecia in the neonatal rat model (Sprague Dawley rats). In this model, rats of 8 to 11 days of age are injected intraperitoneally with chemotherapy. Alopecia ensues 5-7 days later, and is graded depending on the hair loss observed. The protective compounds are given on day 5 after birth for 6 days. This model is useful to study CIA because the hair follicles at this stage are in 100% anagen, which renders them susceptible to chemotherapy toxicity and is comparable to the human setting. This is a very effective and a reproducible model and can be used to study protective formulations against CIA. One of the drawbacks of this model is that the hair follicles are in the first hair growth cycle after birth and that the rats have white fur and lack pigmentation. In contrast, the use of neonatal Long Evans rats allows studying pigmented hairs. Another model that is widely used to study the effect of chemotherapy is an adult C57BL/6 mouse model. The hair follicles in this model have gone through several postnatal growth cycles and the hair shafts are pigmented, similar to the human scalp. The anagen cycle in this particular model is induced by depilation and is observed 8 to 9 days after the procedure. Using this model and cyclophosphamide, it has been demonstrated that as a response to cytotoxicity of chemotherapy, the hair follicle utilizes two pathways: dystrophic anagen or dystrophic catagen, which determines the onset of chemotherapy-induced alopecia and the pattern of hair re-growth. A new model that has been used to investigate CIA is an adult Long Evans rat model, which allows studying hair follicles that have gone through several cycles and rats possess both pigmented and non-pigmented hairs. Additionally, the induction of anagen phase is performed by shaving fur with clippers, the method that may avoid the trauma to the hair follicles that is induced by depilation. Another excellent model to study alopecia, more specifically, AA, has been the C3H/HeJ mouse model26. In this model, 20% of the animals spontaneously develop alopecia in clusters or throughout the entire body (alopecia universalis) by 18 months of age. Alopecia patterns and histopathological analysis has shown that the pathology in this animal is almost identical to that of the human.
1,25-Dihydroxyvitamin D3 protects by modulating the differentiation of hair follicle keratinocytes, rendering them resistant to the toxic metabolites of chemotherapy.

### Example 17. A phase I dose-escalation study, to evaluate the safety, tolerability and pharmacokinetics of a topical calcitriol in adult cancer patients receiving taxane based chemotherapy regimens for the treatment of advanced or recurrent disease.

### 1.0. OVERVIEW

This will be a dose escalation study to determine the maximum tolerated dose (MTD) and the overall safety and tolerability of a topical calcitriol in patients with metastatic or recurrent cancer of the breast, cervical, endometrial, ovarian, fallopian tube, primary peritoneal carcinoma or soft tissue and bone sarcoma, who are undergoing chemotherapy with a taxane based (paclitaxel/ nanoparticle albumin-bound paclitaxel/ docetaxel) regimen. A standard 3+3 dose escalation design will be employed with 3-6 patients at each dose level. Eligible patients >18 years of age and scheduled to receive a taxane based regimen with treatment breaks as per physician's discretion, will start applying the topical solution twice a day at each cohort dose level two weeks prior to initiation of chemotherapy and then continue twice daily for 3 months or until termination of chemotherapy treatment. If topical calcitriol is found to be effective in preventing and/or diminishing taxane chemotherapy-induced alopecia as determined by the photographic assessments and patient self-assessments, patients will be allowed to continue topical application for the duration of their chemotherapy treatment, assuming no Dose Limiting Toxicities (DLTs) related to the topical agent are observed. Toxicity to the topical calcitriol will be assessed on a weekly basis during the first 28 days of topical treatment and subsequently every four weeks by a study clinician, either a physician or a nurse. For the purpose of pharmacokinetic studies (PKs), blood samples will be collected on Day 1 of topical treatment at the following time points: pre-dose, at 2 hours (+/- 30 minutes), 4 hours (+/- 30 minutes), and 8 hours (+/- 1 hour post dose) after a single application on the morning of Day 1. The second application of drug product will be applied 10-14 hours after the initial application and after the 8 hour PK sample. Thereafter, topical application frequency will be twice daily, morning and night. Subsequently, a PK sample will be taken 12 hours (+/- 2 hours) after the last dose of each 28 day treatment, before the first application of Day 1 of the next 28 day treatment cycle. This schedule will continue for three consecutive 28 day topical treatment cycles. (PKs will be drawn at weeks 1, 5, 9, 13. In addition, if patients are still on study, a PK will also be drawn at week 54.)

As a secondary objective, potential efficacy of the topical calcitriol will be evaluated by photographic assessment. Photographic assessment will be performed using a Canon Power Shot G12 camera system to ensure standardization and uniformity among all enrolled patients. The following five views will be obtained at each photographic assessment: bilateral sides of head/scalp view, front of head/face view, back of head/scalp view, and top of head/scalp view. Additionally, close-up photographs will be taken at the same times points. They will include the mid-pattern of the scalp from a superior view and a vertex view with hair parted in the center and combed away from the center part. Photographs will be standardized for lighting, camera angle, and position to the participants head. These assessments will be performed at the following time points: at baseline, weeks 7, 15, 27, and 54. Photographs for patients in each cohort representing baseline, and treatment weeks 7 and 15 will be presented blind to the study principle investigator after at least 3 patients have completed 15 weeks of treatment. Photographs will also be taken at week 27 and week 54 of the study but will be included in the final photographic assessment as secondary information. In addition, all patients will be asked to maintain an application log throughout treatment to ensure compliance. Additionally patients will maintain a self-assessment diary that will require assessment of hair thickness, hair fullness, hair breakage, and hair cosmetic qualities (ease of styling, etc.) on an analog 10 point scale to assess patient-reported efficacy. The principle investigator clinical assessment of baseline, weeks 7 and 15 photographs will be used, together with the patient diary information, for the primary assessment of alopecia. The study is expected to take place over a period of approximately 12 months, including the screening period.

### 2.0. OBJECTIVES AND SCIENTIFIC AIMS

Primary objective: to determine the maximum tolerated dose (MTD) and the overall safety and tolerability of topical calcitriol in adult cancer patients receiving taxane based chemotherapy regimens.

Secondary Objectives: to determine the single and multiple dose pharmacokinetics of calcitriol at different dose levels; and to evaluate preliminary efficacy of calcitriol topical solution for preventing chemotherapy-induced alopecia

### 3.0. OVERVIEW OF STUDY DESIGN/INTERVENTION

It is possible that a short treatment duration is not sufficient to induce catagen stage in scalp hair follicles, which subsequently makes them more susceptible to cytotoxicity of chemotherapy. To address this, topical calcitriol will be applied starting at least 5-7 days (e.g., preferably at least two weeks) prior to the initiation of chemotherapy in an attempt to induce catagen stage that is anticipated to render protection against CIA. Continued application on the daily basis will ensure the maintenance of catagen stage and extended protection throughout administration of multiple doses of a taxane-containing regimen.

*3.1 Design.* This is a single arm, dose-escalation phase I study. Eligible patients will begin applying the topical formulation of calcitriol to the scalp twice daily two weeks prior to the first dose of chemotherapy and continue the application twice daily until termination of chemotherapy treatment. Dose escalation will occur in stepwise increments of the immediate prior dose group, in the absence of grade 3 or greater toxicities attributed to the topical calcitriol, in order to determine the MTD for this agent. Dose limiting toxicity (DLT) will need to possibly, probably or definitely (defined in section 9.2) be related to topical calcitriol and not taxane based regimen as best determined by participating investigators. Similarly, appropriate dose modifications or treatment interruption of the chemotherapeutic regimen will be instituted according to the pre-defined guidelines (defined in sections 9.2.2-9.2.7) and the current standard of care. Determination of DLTs of the topical calcitriol formulation will be made during the first 28 days of topical agent application. Subjects will be managed with adequate safety monitoring and also real time PK analysis in order to determine levels of exposure. PK analysis will be done before each next cohort moves forward. For the purpose of pharmacokinetic studies (PKs), blood samples will be collected on Day 1 of topical treatment at the following time points: pre-dose, at 2 hours (+/- 30 minutes), 4 hours (+/- 30 minutes), and 8 hours (+/-1 hour post-dose) after a single application on the morning of Day 1. The second application of drug product will be applied 10-14 hours after the initial application and after the 8 hour PK sample. Thereafter, topical application frequency will be twice daily, morning and night. Subsequently, a PK sample will be taken 12 hours (+/- 2 hours) after the last dose of each 28 day treatment, before the first application of Day 1 of the next 28 day treatment cycle. This schedule will continue for three consecutive 28 day topical treatment cycles (PKs will be drawn at weeks 1, 5, 9, 13. In addition, if patients are still on study, a PK will also be drawn at week 54.).

*3.2 Intervention.* Patients diagnosed with metastatic or recurrent cancer of the breast, cervical, endometrial, ovarian, fallopian tube, primary peritoneal carcinoma or soft tissue and bone sarcoma, who are scheduled to undergo chemotherapy with a taxane based (paclitaxel/ nanoparticle albumin-bound paclitaxel/ docetaxel) regimen, will be screened for eligibility to participate. All eligible patients will start applying 0.25mL of calcitriol to each of the four quadrants of the scalp - front right, front left, back right, back left with the metered pump spray unit twice a day at least 3, 4, 5, 6, 7, 8, 9, 10, 11, 12, or 13 days (e.g., preferably at least two weeks) prior to chemotherapy and subsequently continued twice daily until termination of chemotherapy. If topical calcitriol is found to be effective in preventing and/or diminishing the taxane based chemotherapy-induced alopecia, as determined by photographic assessments and patient self-report, patients will be allowed to continue twice-daily topical application for the duration of their chemotherapy treatment, assuming no DLTs related to the topical agent are observed. Patients who elect to shave their hair prior to or during chemotherapy treatment will be excluded from the trial. The topical solution will be applied to the scalp. Hair and scalp should be dry or, if application is immediately after shampooing, the hair and scalp should be damp, not wet, to the touch by first towel drying the hair and scalp before application of the topical solution. The hair and scalp will not be washed or shampooed for at least 8 hours after each application. Patients will be advised that that they may apply no more than two consecutive doses of calcitriol before washing or shampooing. Treatment induces catagen phase of hair cycling which will last 2-3 weeks after last application. Application of the drug will ensure this catagen phase protection through multiple chemotherapeutic treatments.

### 4.0. THERAPEUTIC/DIAGNOSTIC AGENTS

### Investigational Product: (Calcitriol, USP) Topical Solution

Chemical Name: (5Z,7E)-9,10-secocholesta-5,7.10( 19Hriene-1 a,33.25-triol
IUPAC Name: (1a.3B.5Z.7E)-9.10-secocholesta-5,7.10(19)-triene-1.3,25-triol
Alternate Names: Calcitriol is also known as: 1a,25-dihydroxycholecalciferol, 1a,25-dihydroxyvitamin D3,1(S),25-dihydroxyvitamin D3, 1,25-DHCC, 1,25-(OH)2D3
Molecular Formula: C27H44O3:
CAS Number: 32222-06-3
Molecular Weight: 416.6
Melting Point: 129-131 °C
Chemical Stability: Stable at room temperature
Route of Administration: Topical
How supplied: 33 mL amber glass bottle (Type III glass) with 18 mm Black Phenolic screw cap with LDPE liner. Pfeiffer topical spray pump (0.25 mL)(white) packaged separately (for In-Use study only).
Clinical Trial Formulation: The proposed clinical trial formulation for calcitriol topical solution contains Calcitriol, USP in a vehicle of Propylene Glycol, USP and Anhydrous 200 proof, Undenatured Alcohol, USP at a ratio of 40/60 by weight propylene glycol/alcohol. The concentration of calcitriol in the topical solution vehicle for human studies has been determined based on the completion of the nonclinical toxicology. The proposed Phase I study will utilize calcitriol at concentrations of 5, 10 and 20 µg/mL.
Packaging: The proposed Phase I clinical drug product is intended to be packaged in a 33 mL capacity Type III amber glass bottle fitted with a black phenolic screw cap. The proposed drug product will also include a separately packaged metered dose dispensing applicator system capable of uniformly dispensing 0.25 mL per applicator compression. The total dosage of drug product is intended to be 1.0 mL, or four repeat metered unit applications. The application of a total of 1.0 mL of drug product will be twice daily, morning and night, 10-14 hours between applications.

Patients will be supplied with a single amber glass bottle of calcitriol topical solution containing approximately 31.5 mL of drug product of topical solution sufficient to dispense 28 mL of drug product (0.25 mL x 4 applications x 2 times/day x 14 days=28 mL). The glass bottle unit will be properly labeled with use instructions, use warnings, and a place for patient ID which will be assigned by the clinical site. Each unit will be secured with a plastic safety seal covering the bottle cap and top of the bottle. Patients will be provided an application log with instructions on how to apply calcitriol and record the date and time of each application. Research staff will monitor compliance by requesting patients bring their used glass bottle, application log and self-assessment diary at each visit. Research staff will review the bottles and application log to ensure compliance of topical treatment administration and document oversight by recording their comments, initials and date of each review.

At the time of use, the patient will tear the safety seal and remove the bottle cap and insert a specially designed pump spray unit into the bottle and secure it in place on the bottle. Patients will be instructed to dispense drug product by depressing the pump three times to prime the pump and then spraying 0.25 mL of drug product four separate times to each quadrant of the scalp followed by massaging the scalp to ensure even distribution of the product. This dosing regimen will be repeated twice daily, morning and night, for 14 consecutive days prior to initiation of chemotherapy and subsequently twice on a daily basis. Clinical drug product supplies will be stored at refrigeration temperature (5-8 degrees C) until given to patients for use. During usage, patients will store the drug product at room temperature.

Storage: The stability storage conditions that will be evaluated for the proposed IND Clinical Phase 1 batches are described in the following table:

### Proposed Stability Storage Conditions for Clinical Phase I

| | | |
|---|---|---|
| Storage Conditions: | Long Term Storage: | 25 °C ± 2 °C/60 |
| | %RH ± 5 %RH | |
| | Accelerated Storage: | 40 °C ± 2 °C/75 % |
| | RH ± 5 %RH | |
| | Control Storage | 2-8 °C |
| Storage Position: | Horizontal | |
| Strengths: | 5 µg/mL, 10 µg/mL, 20 µg/mL | |
| Packaging: | 1. 33 mL amber glass bottle (Type III glass) with 18 mm Black Phenolic screw cap with LDPE liner. | |
| | 2. Pfeiffer topical spray pump (0.25 mL)(white) packaged separately (for In-Use study only) | |
| Intended Dose: | 5 µg/mL - 1.25 µg per actuation | |
| | 10 µg/mL - 2.5 µg per actuation | |
| | 20 µg/mL - 5 µg per actuation | |

Dosing Rationale: The NOAEL (no-observed-adverse-effect-level) in the nonclinical animal studies was 10.31 µg/mL. To compare the animal and human doses of calcitriol, a margin of safety (MOS) was calculated using the NOAEL dose in the 4-week minipig study and the initial dose in the upcoming clinical trial. In minipigs, the NOAEL, 10.31 µg/mL is equivalent to a dose of 3.33 µg/kg (10.31 µg/mL x 2.1 mL (dose volume) x 2X/day + 13 kg [average weight of minipigs in Week 4]). In humans, 5 µg/mL x 1 mL x 2X/day * 60kg). Based on these doses, the MOS is 20 which is an adequate margin over the initial clinical dose. If the initial dose causes toxicity, the patient will be removed from the study.

### 5.0. CRITERIA FOR SUBJECT ELIGIBILITY

The study will be conducted in patients with a diagnosis of locally advanced unresectable and/or metastatic cancer of the breast, cervical, endometrial, ovarian, fallopian tube, primary peritoneal carcinoma or soft tissue and bone sarcoma, who are scheduled to receive treatment with a taxane based (paclitaxel/ nanoparticle albumin-bound paclitaxel/ docetaxel) chemotherapy regimen, as per physician's discretion.

### 5.1 Subject Inclusion Criteria:

- Adult patients at least 18 years of age.
- Able to fully understand and participate in the informed consent process.
- History of locally advanced unresectable and/or metastatic cancer of the breast, cervical, endometrial, ovarian, fallopian tube, primary peritoneal carcinoma or soft tissue and bone sarcoma, with pathology confirmed.
- Scheduled to receive a taxane based (paclitaxel/ nanoparticle albumin-bound paclitaxel/ docetaxel) regimen, as per physician's discretion.
- Have no evidence of alopecia or mild alopecia (NCI CTCAE grade 1 alopecia defined as hair loss of < 50% of normal for that individual that is not obvious from a distance but only on close inspection; a different hairstyle may be required to cover the hair loss but it does not require a wig or hair piece to camouflage.) Female/male-pattern baldness or age-related hair loss are allowed if not greater than grade 1, per NCI-CTCAE v.4.0. Subjects that have previously lost their hair may enroll if they currently have Grade 0 or 1 alopecia.
- ECOG Performance Score of 0 or 1 within 14 days prior to registration.
- Has baseline neutrophil counts of > 1500 cells/mm³ within 72 hours prior to registration.
- Has serum calcium ≤ ULN (for patients with an albumin lower than 3.0, a corrected calcium serum calcium = serum calcium +[0.8][3.5- serum albumin]) within 72 hours prior to registration.

### 5.2 Subject Exclusion Criteria:

- Patients receiving calcium-lowering therapy or drugs that may affect calcium levels (*e.g*., calcitonin, mithramycin, phosphate, denosumab) within 4 weeks of initiation of topical calcitriol. Patients who have been managed with bisphosphonates or calcium-lowering therapy for 3 months or greater prior to the start of the trial and have demonstrated evidence for stability of calcium metabolism would be considered eligible for participation in the trial.
- Has a history of drug or alcohol abuse within 1 year of study enrollment as determined by the investigator.
- Patients who elect to shave the scalp hair prior to the initiation of chemotherapy or who plan to do so during the chemotherapy treatment.
- Any dermatological condition that in the opinion of the investigator will affect the absorption of the study medication, *e.g*., Atopic Dermatitis, etc.
- Has been treated with an investigational agent within 30 days or six half-lives of its biologic activity whichever is longer, before the start of study (Patients may not be concurrently enrolled on another trial or concurrently treated with another investigational agent).
- Patients with a history of hypercalcemia or vitamin D toxicity, or hospitalization for treatment of angina, myocardial infarction, or congestive heart failure or psychiatric illness currently or within 30 days of study entry as determined by the investigator.
- Has a history of significant allergy to calcitriol as determined by the investigator.
- Has any condition that interferes with the ability of the subject to understand or comply with the requirements of the study.
- Patients taking Vitamin D supplements during the study, unless they have been taking Vitamin D supplements for 30 days or more prior to the start of the study and that the dose of the Vitamin D supplement remain the same throughout the study.
- Patients treated with medications that are known to affect calcium levels within 4 weeks of initiation of topical therapy (> 500 IU vitamin A, calcium supplements, fluoride, antiepileptics), with the exception of subjects on stable therapy for more than six months.
- Patients receiving thiazides or furosemide diuretics, with the exception of subjects who have stable doses and have been on therapy for over six months.
- Patients with hypercalcemia or kidney stones.
- Patients that indicate they have significant hair breakage or hair damage and associated hair loss from hair over processing within the last 30 days due to peroxide applications, permanent hair coloring, bleaches, streaking, perms, relaxers and/or hair oxidative dyes.
- Alopecia grade 2 or greater as per NCI-CTCAE v.4.0, or significant hair loss or hair breakage.
- Prior radiation to the cranium.
- Pregnancy or breastfeeding.

### 6.0. RECRUITMENT PLAN

Patients will be offered the opportunity to participate if they meet the eligibility criteria. There will be no discrimination against minorities. Informed consent will be obtained from the patient. Consent will be obtained by an investigator authorized to obtain consent. Patients will not receive any payment for their participation in this study.

### 7.0. PRETREATMENT EVALUATION

The following are required at least 5-9 days (e.g., at least 4 days, 5 days, at least 6 days, at least 7 days, at least 8 days, at least 9 days, or at least 2 weeks) prior the start of calcitriol:
- Complete medical history and physical exam, including concomitant medication.
- CBC with differential, Comprehensive chemistry panel, serum phosphorus level, and Urinalysis.
- Serum 1, 25 dihydroxy Vitamin D.
- Pregnancy test (Pregnancy tests outlined here are for women of childbearing potential (WCBP) only). Please exclude all women that do not meet the criteria for WCBP and meet the criteria for women not of childbearing potential. Women of ChildBearing Potential Defined: any female who has experienced menarche and does not meet the criteria for "Women Not of Childbearing Potential." Women Not of Childbearing Potential Defined: women who are permanently sterilized (*e.g*., tubal occlusion, hysterectomy, bilateral salpingectomy, bilateral oophorectomy); women who are > 45 years of age, not using hormone replacement therapy and who have experienced total cessation of menses for at least 1 year OR who have a follicle stimulating hormone (FSH) value >40 mIU/mL and an estradiol value <40pg/mL (140 pmol/L); and women who are >45 years of age, using hormone replacement therapy and who have experienced total cessation of menses for at least 1 year OR who have had documented evidence of menopause based on FSH > 40 mIU/mL and estradiol < 40 pg/mL prior to initiation of hormone replacement therapy.)
- Photographic record of hair and scalp prior to chemotherapy. (Will serve as the baseline assessment for the study.)
- Patient self-assessment of hair condition.

### 8.0. TREATMENT/INTERVENTION PLAN

Eligible patients will be instructed to apply 0.25 mL of topical calcitriol to each of the four quadrants of the scalp - front right, front left, back right, back left with the provided metered pump spray unit twice a day at least 5-9 days (e.g., preferably at least 5 days, at least 6 days, at least 7 days, at least 8 days, at least 9 days, or at least two weeks) prior to the start of chemotherapy treatment. Subsequently, application will continue twice daily for three months or until termination of chemotherapy. The topical solution will be applied to the scalp. Hair and scalp should be dry or, if application is immediately after shampooing, the hair and scalp should be damp, not wet, to the touch by first towel drying the hair and scalp before application of the topical solution. The scalp will not be washed or shampooed for at least 8 hours after each application. Patients will be advised that that they may apply no more than two consecutive doses of calcitriol before washing or shampooing. Meaning, they must wash their hair after every other application of calcitriol. Patients will self-administer the topical solution throughout the study; except for first dose and days when pharmacokinetic studies will be performed, in which case application of topical calcitriol will be done by the study personnel.

### 8.0.1 Taxane based (Paclitaxel/Nanoparticle albumin-bound paclitaxel/ Docetaxel) Chemotherapy Administration.

All patients should be pre-medicated prior to administration of the chemotherapeutic agents in order to prevent severe hypersensitivity reactions, per institutional guidelines.

Examples of recommended regimens for patients with carcinoma of the breast:
1. For the treatment of metastatic breast cancer, recommended regimens are paclitaxel/ nab-paclitaxel/ docetaxel based or in combination with carboplatin.

Examples of recommended regimens for patients with gynecological cancer:
1. For treatment of cancers of the ovary, recommended regimens are paclitaxel/ docetaxel based or in combination with carboplatin.
2. For treatment of endometrial cancers, recommended regimens are paclitaxel based or in combination with carboplatin.
3. For treatment of uterine sarcoma, recommended regimens are docetaxel based or in combination with gemcitabine.
4. For treatment of cervical cancer, recommended regimens are paclitaxel based or in combination with cisplatin/ topotecan.

Examples of recommended regimens for patients with sarcoma:
1. For treatment of soft tissue or bone sarcoma, recommended regimens are paclitaxel/ docetaxel based or in combination with gemcitabine.

*8.1 Dose Escalation.* Patients will be treated with the topical calcitriol in cohorts of size three to six starting at dose level 1 (5 µg/mL). The dosage will be escalated if the clinical toxicity is declared acceptable (see below). Two dose levels will be considered for escalation (10 and 20 µg/mL). Determination of dose limiting toxicity (DLT, defined in section 8.2) will be made during the first 28 days of topical treatment. No intrapatient dose escalation will be performed. DLT will need to possibly, probably or definitely (defined in section 8.2) be related to topical calcitriol and not the chemotherapeutic regimen as best determined by participating investigators. The dose escalation scheme (Table 8.1) will occur as follows:
One 28 day cycle of treatment will be performed and evaluation of PK Data will occur before escalation to the next dose level.

If none of the initial three patients in a cohort experience dose limiting toxicity (DLT), then a new cohort of three patients will be treated at the next higher dose level.

If one of the three patients in a cohort experiences DLT, then up to three additional patients will be treated at the same dose level. Escalation will continue if only one of the six patients experiences DLT.

If two or more patients in a cohort experience DLT, then the maximum tolerated dose (MTD) will have been exceeded, and no further dose escalation will occur. The previous dose level will be considered as the MTD.

If only three patients were treated at a dose level under consideration as the MTD, then up to three additional patients will be accrued to that dose level. If no more than one of six patients at that dose level experience a DLT, then that dose level will be confirmed as the MTD. If two or more patients in that cohort experience DLT, then the previous dose level will be studied in the same fashion.

The MTD is defined as the dose level at which 0/3 or 1/6 subjects experiences DLT during the first 28 days treatment cycle below the dose at which 2/3 or 2/6 subjects experienced DLT. Thus, the MTD will have been exceeded when > 30% (2/3 or 2/6) of subjects at any dose level develop DLT. If the MTD is not established after completing three cohorts of escalating doses, the study Sponsor will review the study findings with FDA Division of Dermatology to seek guidance as to amending the study protocol to add additional dose escalating cohorts.

The Cohort Review Committee (CRC) will be fully aware of clinical and laboratory data, and must agree if dose escalation to the next cohort is appropriate. Pharmacokinetic data from each treatment cohort will also be reviewed by the CRC and considered in any decision to dose-escalate. Adverse event data from the treatment extension period will be presented, when available (at least monthly), to the CRC. These data will be considered for dose escalation decisions. In the event that MTD has not been reached after three cohorts have been treated, any further dose escalations will require a protocol amendment.

The CRC will review all available data from previous cohorts to assure that the actual dose escalation determined in this fashion does not expose subjects to unreasonable risk. The CRC may reduce or halt dose escalations for any reason (*e.g.*, observation of non-linear PK, AEs in subjects who receive more than one dose of calcitriol topical solution). The decision to proceed with the next cohort will require unanimous agreement of the members of the CRC.

The nonclinical toxicology has been reviewed and the cohort 1 drug concentration is approximately 1/20 or less of the expected MTD based on the nonclinical studies. As there is no presupposed risk of dose limiting toxicities at the cohort 1 dosing detailed in the Phase I protocol, it has been determined that there will be no minus 1 dosing cohort. If two or more of the initial patients experience unacceptable toxicities at the initial Cabitriol dose, the CRC will review all available data and may elect to discontinue the study.

**Table 8.1**

| Number of Subj ects per cohort with DLT during Treatment Period: | Dose Escalation Decision Rule: | |
|---|---|---|
| 0 out of 3 | Enter 3 subjects at the next dose level. | |
| 1 out of 3 | Enter at least 3 more subjects at this dose level. | |
| | | • If none of the 3 additional subjects has |
| | DLT, proceed to the next dose level. | |
| | | • If 1 or more of the 3 additional subjects |
| | has DLT, then dose escalation is stopped, and this dose is declared the maximum administered dose (MAD). Three additional subjects will be entered at the next lowest dose level if only 3 subjects were treated previously at that dose. | |
| 2 | Dose escalation will be stopped. This dose level will be declared the MAD (highest dose administered). Three additional subjects will be entered at the next lowest dose level if only 3 subjects were treated previously at that dose. | |
| 1 out of 6 at highest dose level below the maximally administered dose | This is generally the recommended phase 2 dose. | |

*Pharmacokinetics (all patients).* For the purpose of pharmacokinetic studies (PKs), blood samples will be collected on Day 1 of topical treatment at the following time points: pre-dose, at 2 hours (+/- 30 minutes), 4 hours (+/- 30 minutes), and 8 hours (+/-1 hour post dose) after a single application on the morning of Day 1. The second application of drug product will be applied 10-14 hours after the initial application and after the 8 hour PK sample. Thereafter, topical application frequency will be twice daily, morning and night. Subsequently, a PK sample will be taken 12 hours (+/- 2 hours) after the last dose of each 28 day treatment, before the first application of Day 1 of the next 28 day treatment cycle. This schedule will continue for three consecutive 28 day topical treatment cycles. (PKs will be drawn at weeks 1, 5, 9, and 13. In addition if patients are still on study, a PK will also be drawn at week 54.) Vital signs will be obtained approximately 5 minutes before each blood collection, and the actual collection times will be recorded.

At each collection, collect blood by venipuncture in a serum separator tube(s). Centrifuge at 1200 RCF for 10 ± 5 minutes, and aliquot 1.5 mL of serum each into 2 labeled externally threaded cryogenic vials and immediately freeze. Samples collected from each cohort will be shipped frozen on dry ice to a laboratory for analytical determination of calcitriol concentrations in serum. Serum samples will be transported with a sufficient amount of dry ice to keep the samples frozen until arrival.

The serum PK of calcitriol will be calculated from serum collected from all subjects who receive calcitriol. The following serum PK parameters will be calculated using non-compartmental analysis:
UCₒ₋ₜ: Area under the concentration - time curve up to the last measurable concentration calculated by the trapezoidal rule and expressed in units of concentration - time.
AUC_{o-inf}: Area under the serum concentration-time curve from time of dosing to infinity calculated by dividing the last quantifiable concentration by Kel and adding the result to UCₒ₋ₜ, expressed in units of concentration-time.
Cmax: The observed peak drug concentration obtained directly from the experimental data without interpolation, expressed in concentration units.
Tmax: The observed time to reach peak drug concentration obtained directly from the experimental data without interpolation, expressed in time units (hour).
Kel: The apparent elimination rate constant, determined by regression analysis of the log-linear segment of the serum concentration-time curve, expressed in time-1 units (1/hour).
T_{1/2}: The terminal half-life, calculated as -In 2/Kel, expressed in time units(hour)
CL: Clearance calculated as the drug dose/AUC_{o-inf}, expressed in units of flow (L/hour).
Vd: Volume of distribution calculated as CL divided by Kei and expressed in units of volume (L).

Descriptive statistics (including number, mean, median, standard deviation, and range) for PK parameters will be tabulated by dose level. Estimated renal elimination will be tabulated by dose level.

### 8.2 Definition of Dose-Limiting Toxicity (DLT) and treatment modifications.

Toxicities will be graded according to the Common Terminology Criteria for Adverse Events (CTCAE v 4.0).

*8.2.1 Dose-Limiting Toxicity related to topical calcitriol.* Dose-limiting toxicity is defined as a clinically significant grade 3 or 4 non-hematologic toxicity occurring during the first 28-day treatment cycle of the topical agent application, and needs to be possibly, probably, definitely related to calcitriol (and not the chemotherapeutic regimen) as best determined by investigators. Excessive dosage of calcitriol induces hypercalcemia and in some instances hypercalciuria. If the patient presents with symptoms of hypercalcemia, serum calcium should be determined and treatment should be stopped immediately. Hypercalcemia will be defined using CTCAE version 4.0 grade 3 (or higher), which is defined as serum calcium > 12.5 -13.5 mg/dL; > 3.1 - 3.4 mmol/L or ionized calcium > 1.6 -1.8 mmol/L and hospitalization is indicated. For patients with asymptomatic hypercalcemia determined by elevated serum calcium, their ionized calcium levels will first be checked. If the ionized calcium level is in fact elevated, the study drug will be stopped. If the ionized calcium level is normal, the patient will remain on the study and ionized calcium will be followed rather than serum calcium. If the calcitriol has to be discontinued for this reason, serum calcium and phosphate levels will be checked with daily blood draws at the testing center until they are normal for two consecutive days.

*8.2.2 Dose-Limiting Toxicity.* If patients develop toxicities related to their chemotherapeutic regimen, then the dose reductions will be followed, per institutional guidelines. During modifications in the chemotherapeutic regimen, calcitriol doses will remain stable unless modifications are deemed necessary by the investigator.

### 9.0. EVALUATION DURING TREATMENT/INTERVENTION

*Clinical.* History and physical examination will be performed as detailed in the table below. Following the start of application of topical calcitriol at least 5-7 days (*e.g*., at least 2 weeks) prior to initiation of chemotherapy, patients will be seen by an investigator for interim medical history, concomitant medication, physical exam including weight, vital signs (blood pressure, temperature, respiration rate, heart rate), and adverse events at Weeks 1, 2, 3, 5, 7, 11, 15, 27, and 54.

*Laboratory.* Laboratory evaluation will be performed as described in the table below.

### STUDY EVALUATION SCHEDULE PART A - THE BASIC INFORMATION

**All study assessments will be performed +/- 72 hours from the scheduled date unless otherwise noted.**

| Basic Information | *Chemo Week* | Sign informed consent | Demographics | Medical history | Concomitant meds | Physical examination | Pregnancy test | Weight | Vital signs | Adverse Events Evaluation |
|---|---|---|---|---|---|---|---|---|---|---|
| Pre-study | | + | + | + | + | + | + | | + | + |
| Week 1 (topical application prior to chemo) | | | | | | + | | + | | + |
| Week 2 ft (topical application prior to chemo) | | | | | | + | | + | + | + |
| Week 3 (start of chemo) | *1* | | | | + | | | + | + | + |
| Week 4 | *2* | | | | | | | | | |
| Week 5 | *3* | | | + | + | + | | + | + | + |
| Week 6†††† | *4* | | | | | | | | | |
| Week 7 | *5* | | | + | + | + | | + | + | + |
| Week 8 | *6* | | | | | | | | | |
| Week 9 | *7* | | | | | | | | | |
| Week 10†††† | *8* | | | | | | | | | |
| Week 11 | *9* | | | | + | | | + | + | + |
| Week 12 | *10* | | | | | | | | | |
| Week 13 | *11* | | | | | | | | | |

| | | | | | | | | | | |
|---|---|---|---|---|---|---|---|---|---|---|
| Week 14 †††† | *12* | | | | | | | | | |
| Week 15 | *13* | | | + | + | + | | + | + | + |
| Week 27††††††† | | | | + | + | + | | + | + | + |
| Week 54††††††† | | | | + | + | + | | + | + | + |

### STUDY EVALUATION SCHEDULE PART B - THE TESTS AND THE STUDY DRUG APPLICATION

| Tests and Study Drug Application | *Chemo Week* | PK blood samples† | Application of C31543 (Calcitriol) | CBC with diff | Comprehensive chemistry panel | Photographics record of hair and scalp | Serum phosphorus and Vitamin D | Patient self-assessment diary ttt |
|---|---|---|---|---|---|---|---|---|
| Pre-study | | | | + | + | | + | + |
| Week 1 (topical application prior to chemo) | + | + | + | + | + | | + | + |
| Week 2 tt (topical application prior to chemo) | - | | + | | | | | + |
| Week 3 [start of chemo] | *1* | | + | + | + | | + | + |
| Week 4 | *2* | | + | | | | | + |
| Week 5 | *3* | + | + | + | + | | + | + |
| Week 6†††† | *4* | | + | | | | | + |
| Week 7 | *5* | | + | + | + | + | + | + |
| Week 8 | *6* | | + | | | | | + |
| Week 9 | *7* | + | + | | | | | + |
| Week 10†††† | *8* | | + | | | | | + |
| Week 11 | *9* | | | + | + | | + | + |
| Week 12 | *10* | | + | | | | | + |
| Week 13 | *11* | + | + | | | | | + |
| Week 14†††† | *12* | | + | | | | | + |
| Week 15††††† | *13* | | | + | + | + | + | + |
| Week 19†††††† | | | | | | | | + |
| Week 27††††††† | | | + | + | + | + | + | + |
| Week 54††††††† | | + | + | + | + | + | + | |

| | | | | | | | | |
|---|---|---|---|---|---|---|---|---|
| † PK will be collected in the following manner: On Day 1 of topical treatment: PK will be collected pre-dose (before the first dose), and 2 hours (+/- 30 minutes), 4 hours (+/- 30 minutes) and 8 hours (+/- 1 hour post-dose). For study weeks 5, 9, 13, and 54 of calcitriol, PK will be collected on Day 1 predose. This should be 12 hours (+/- 2 hours) after the last application of topical calcitriol. †† For the week 2 visit, patients will be seen by a study Dermatologist in the Dermatology clinic on 8 Monday, Wednesday, or Thursday during the week. ††† All patients will be asked to maintain the self-assessment diary for up to approximately 6 months from initial application of study drug. The diary will be filled out weekly for the first 15 weeks after initiating calcitriol and at weeks 19, 23, and 27. †††† Note that some patients may be placed on a 3-week-on one-week-off regimen. The indicated weeks will be off-chemo weeks. ††††† Photographs for patients in each cohort representing baseline, and treatment weeks 7 and 15 will be presented blind to the study PI after at least 3 patients have completed 15 weeks of treatment. Photographs will also be taken at week 27 and week 54 of the study but will be included in the final photographic assessment as secondary information. The PI clinical assessment of the baseline, and weeks 7 and 15 photographs will be used, together with the patient self-assessment diary information, for the primary assessment of alopecia. †††††† A patient self-assessment is required this week if treatment continues beyond week 15. ††††††† Study assessments will be performed +/- 7 days from the scheduled date. | | | | | | | | |

*Photographic record of hair and scalp.* Alopecia is defined as any hair loss. In this study Global photographic review will be conducted using the Canfield Clinical Photography assessment images that will be acquired by the research nurse to ensure standardization and uniformity among all enrolled patients. The following five views will be obtained at each photographic assessment: bilateral sides of head/scalp view, front of head/face view, back of head/scalp view, and top of head/scalp view. Additionally, close-up photographs will be taken at the same times points. They will include the mid-pattern of the scalp from a superior view and a vertex view with hair parted in the center and combed away from the center part. Photographs will be standardized for lighting, camera angle, and position to the participants head.
Global photographic review will be conducted by one dermatologist reviewer and photographs will be evaluated using a 7-point evaluation scale for hair volume (-3 = greatly decreased, -2 = moderately decreased, -1 = slightly decreased, 0 = no change, +1 = slightly increased, +2 = moderately increased, +3 = greatly increased). The reviewer will compare the photographs acquired at baseline chemotherapy, after 1 month of chemotherapy, and after 3 months of chemotherapy treatment. The dermatologist reviewer scoring the photographs will be blinded to time sequence of the photographs aside from the baseline photographs to which all others will be compared.

*Subjective record of hair and scalp.* Alopecia will also be subjectively recorded through the patient self-reported diaries. All patients are asked to complete this self-assessment diary weekly that will require assessment of hair thickness, hair fullness, hair breakage, and hair cosmetic qualities (ease of styling, etc.) on an analog 10 point scale throughout treatment to assess patient-reported efficacy of the study drug. These will be completed weekly and returned to the research staff at every visit during calcitriol treatment. The study is expected to take place over a period of approximately 12 months, including the screening period.

### 10.0. POST-TREATMENT EVALUATION

Post-treatment physical examinations will be performed by an investigator and will take place 12 weeks after the completion of the study treatment date. Exams will include: weight measurement, vital signs, blood sample draws (complete blood count, comprehensive chemistry panel, serum phosphorus, and serum vitamin D), and Adverse Event evaluation.

### 11.0. TOXICITIES/SIDE EFFECTS

NCI CTCAE version 4.0 will be used to grade all toxicity. Below are some side-effects that may be observed:
Common side effects (20-30%)
   - Pruritus
   - Skin discomfort
   - Skin stinging or burning
   - Eye irritation or stinging
Less common side effects (< 20%)
   - scalp xerosis and flaking
   - erythema
   - Irritant dermatitis
Rare but serious side effects (1-5%)
   - Hypercalcemia
   - Hypercalciuria
   - Renal stones
   - Increased thirst
   - Increased frequency of urination
   - Changes in pulse
   - Weakness
   - Drowsiness
   - Bone pain
   - Renal Insufficiency

### 12.0. CRITERIA FOR THERAPEUTIC RESPONSE/OUTCOME ASSESSMENT

This trial is a phase I study and thus primarily a safety study of calcitriol topical solution in patients with metastatic or recurrent cancer of the breast, cervical, endometrial, ovarian, fallopian tube, primary peritoneal carcinoma or soft tissue and bone sarcoma, who are undergoing chemotherapy with a taxane based (paclitaxel/ nanoparticle albumin-bound paclitaxel/ docetaxel) regimen. This study will focus on determining MTD and therapeutic response of calcitriol topical solution.

### 13.0. CRITERIA FOR REMO VAL FROM STUDY

All patients may continue therapy unless DLT is documented. In case of death, the cause of death should be documented. If the toxicity is not dose limiting but precludes the patient from continuing treatment, the case will be reported to IRB and, for each case, a decision will be made whether the patient's toxicity should qualify as a DLT or whether the patient should be considered unavailable and replaced by another patient enrolled at the same dose level.

The following events may be considered sufficient reason for discontinuing treatment with the study medication:
- Serious toxicity due to the study drug graded according to the NCI Common Terminology Criteria for Adverse Events v4.0.
- Conditions requiring therapeutic intervention not permitted by the protocol.
- Unacceptable toxicity in the opinion of the patient or investigator even if not specifically defined elsewhere.
- Personal preference by the patient for any reason.
- Subject non-compliance with the defined treatment plan.
- Medical or psychiatric illness Any other situation where, in the opinion of the investigator, continued participation in the study would not be in the best interest of
   the patient or further therapy is not possible.
- Pregnancy.
- Loss of all of the patient's hair after the first three cycles of topical calcitriol application.

Subjects may withdraw their consent to participate in the study at any time without prejudice. The investigator may withdraw a subject if, in his or her clinical judgment, it is in the best interest of the subject or if the subject cannot comply with the protocol. When possible, the tests and evaluations listed for the termination visit should be carried out. If a subject fails to return for the protocol defined visits, an effort must be made to determine the reason. If the subject cannot be reached by telephone, a registered letter, at the minimum, should be sent to the subject (or the subject's legal guardian) requesting contact with the clinic. This information should be recorded in the CRF.

The investigator will also withdraw a subject upon the sponsor's request or if the sponsor chooses to terminate the study. Upon occurrence of a serious or intolerable adverse effect, the principal investigator will confer with the sponsor. If a subject is discontinued due to an adverse effect, the event will be followed until it is resolved, or if not resolved within a reasonable time (approximately 30 days), until its clinical relevance and etiology can be reasonably explained. A subject may withdraw his or her consent at any time during the study.

If a subject withdraws from the study at any time either at his or her request or at the principal investigator's discretion, the reason for withdrawal will be recorded in the CRF. All subjects who withdraw from the study prematurely will undergo all end-of-study assessments, if possible.

Every effort must be made to undertake protocol-specified safety follow-up procedures.

### 14.0. BIOSTATISTICS

This is a Phase I study designed to determine the maximum tolerated dose (MTD) of topical calcitriol in patients with CIA. The three proposed doses of topical calcitriol are 5 µg/mL, 10 µg/mL, and 20 µg/mL.

Patients will be treated in cohorts of size three to six and the dosage will be escalated if the clinical toxicity is acceptable. A patient is considered toxicity-free for the purpose of the trial if s/he completes the first month of topical agent application without experiencing dose limiting toxicity (DLT). If the topical agent is discontinued during the first month for reasons other than toxicity, an additional patient may be enrolled at that dose level to ensure adequate evaluation of toxicity. No within patient dose escalation will be performed. In all cases, including those when the topical treatment continues post 4 weeks, the MTD assessment will be based only on the DLT recorded during the month. Two dose levels will be considered for escalation. DLT is defined in section 8.2 and the design is constructed to minimize the chances of escalating the dose when the probability of DLT is high, and maximize the chance of escalating the dose when the probability of DLT is low. The dose escalation scheme is as follows:
1. If none of the initial three patients at a given dose level experience DLT, the next dose level will be studied.
2. If one of the initial three patients at a given dose level experiences DLT, three additional patients will be treated at the same dose level. Escalation will continue only if there has been no additional DLT observed.
3. If two or more patients experience DLT at a given dose, the previous dose will be declared the MTD.
4. If only three patients were treated at a dose under consideration as MTD, an additional three patients will be treated at that level to confirm previous results. The probability that dose escalation will occur at any stage during MTD determination, is a function of the underlying DLT rate at the current dose level. This probability can be calculated as the sum of the binomial probabilities of the following two outcomes that would permit escalation to occur: (1) No DLT observed in the first three patients. (2) One DLT is observed in the first three patients followed by no DLT observed in three additional patients at the same dose level.

The true risk of toxicity is expected to be in the range of 10% - 50%. The following table shows the corresponding probabilities of dose escalation:

| | | | | | |
|---|---|---|---|---|---|
| True Risk of Toxicity | 0.10 | 0.20 | 0.30 | 0.40 | 0.50 |
| Probability of Escalation | 0.91 | 0.71 | 0.49 | 0.31 | 0.17 |

These numbers show the probability of escalating to the next dose level is large when the underlying true toxicity rate is small and the probability of escalating decreases appropriately as the true toxicity rate increases.

*Safety Analyses.* Selected non-hematologic and hematologic toxicities, as measured by the NCI Common Terminology Criteria for Adverse Events (CTCAE Version 4.0), will be described by frequency and grade, by cycle and over all cycles, with the maximum grade over all cycles used as the summary measure per patient.

Adverse event terms recorded on the CRF will be mapped to preferred terms using the medical dictionary for regulatory activities (MedDRA) dictionary. All Adverse Events (AEs) will be listed or tabulated for overall incidence and for incidence for each dose cohort, worst reported severity, and relationship to study treatment according to system organ class and preferred term. Serious adverse events (SAEs) will be similarly summarized. Listings of deaths, SAEs, DLTs, and AEs leading to early termination of study treatment, or premature withdrawal from trial will also be provided.

Laboratory variables will be examined using mean change in value from baseline to various time points for each dose cohort. Laboratory values will also be categorized according to the CTCAE v4.0; listings or tables will be categorized by the worst on-study toxicity grade, dose cohort, and relationship. Shift tables will be presented to show the number and percent of subjects with high, normal, and low (or normal/abnormal) laboratory results at baseline and last assessment.

Concomitant medications will be summarized for all subjects, including summary by dose cohort.

*Significance Level.* Global Photographic review and patient self-reported diaries will be used for preliminary statistical analyses. Analyses will look at the efficacy of topical calcitriol. This will be helpful for further studies looking more closely at the efficacy of topical calcitriol. While no formal statistical testing is planned for this study, ninety-five percent confidence intervals may be calculated for selected safety variables. This Phase I trial will accrue only three patients per cohort, which is not a statistically significant number of patients, nor is the trial design adequate to provide objective statistical data sufficient for proof of efficacy.

*Exploratory Analyses.* Exploratory variables will be assessed for each subject, and descriptive statistics (including number, mean, median, standard deviation, and range) will be calculated for subjects by dose level.

*Interim Analyses.* No interim analysis is planned.

*Sample Size*/*Accrual Rate.* The phase I portion of the study for 3-6 patients to be treated at each dose level. Assuming at least 3 dose levels, this trial will require a minimum of 2 and a maximum of 18 patients. This clinical trial will be conducted only at a single clinical site. With the expected accrual rate of approximately 3-6 eligible patients per treatment cohort, it is expected that the Phase I portion of the trial will take less than 1 year. This allows each 3-patient cohort to be observed for 28 days, the length of time for treatment with one cycle of therapy prior to additional patients being accrued.

### References:

Diker-Cohen T, Koren R, Liberman UA, Ravid A Vitamin D protects keratinocytes from apoptosis induced by osmotic shock, oxidative stress, and tumor necrosis factor." Ann N Y Acad Sci. 2003 Dec; 1010:350-3.
(ClinicalTrials.gov, Mosby's Drug Consult, 13th Edition).
Genever PG, MAxfield, SJ, Kennovin GD, Maltman J, Bowgen CH, Raxworthy MJ, Skerry TM. Evidence for a novel glutamate-mediated signaling pathway in keratinocytes. J Invest Dermatol. 1999 Mar; 112 (3): 337-42.
Kiryu-Seo S, Gamo K, Tachibana T, Tanaka K, Kiyama H. Unique anti apoptotic activity of EAAC1 in injured motor neurons. The EMBO Journal (2006) 25, 3411-3421.
Nollen EA, Bruntsing JF, Roelofsen H, Weber La, Kampinga HH. In vivo chaperon activity of heat shock protein 70 and thermotolerance. Mol Cell Biol 1999; 19: 2069-79.
Rocchi P, Jugpal P, SoA, Sinneman S, Ettinger S, Fazli L, Nelson C, Gleave M. Small interence RNA targeting heat shock protein 27 inhibits the growth of prostatic cell lines and induces apoptosis via caspase 3 activation in vitro BJU Int 2006.
Marenholz I, Heizmann CW, Fritz G (2004). S100 proteins in mouse and man: from evolution to function and pathology (including an update of the nomenclature). Biochem. Biophys. Res. Commun. 322 (4): 1111-22.

## Claims

1. A pharmaceutical composition comprising a therapeutically effective amount of a vitamin D compound for use in a method of preventing or mitigating chemotherapy-induced alopecia in a human subject, the method comprising the steps of:
(1) selecting a human subject having a cancer and who is scheduled to receive, or is receiving, a chemotherapy; and
(2) topically administering the pharmaceutical composition to the scalp of the subject using a metered spray unit,
wherein step (2) is performed prior to and/or concurrently with the chemotherapy,
wherein the pharmaceutical composition comprises the vitamin D compound at a concentration of 5-20 µg/mL;
wherein the pharmaceutical composition is administered in a 1.0 mL dose; and
wherein the vitamin D compound is represented by Formula (1): 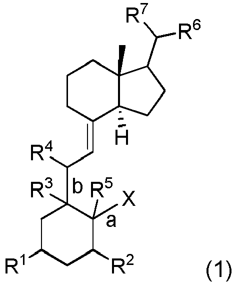
wherein
a and b are each independently a single or double bond;
X is -CH₂ when a is a double bond, or X is hydrogen or a hydroxyl substituted alkyl when a is a single bond;
R¹ is hydrogen, hydroxyl, alkoxyl, tri-alkyl silyl or alkyl, optionally substituted with one to three halogen, hydroxyl, cyano or-NR'R" moieties;
R² is hydrogen, hydroxyl, -O-trialkyl silyl, or alkyl, alkoxyl or alkenyl, optionally substituted with one to three halogen, hydroxyl, cyano or -NR'R" moieties;
R³ is absent when b is a double bond or R³ is hydrogen, hydroxyl or alkyl, or R³ and R¹ together with the carbon atoms to which they are attached may be linked to form 5-7 membered carbocyclic ring when b is a single bond;
R⁴ is absent when b is a double bond or hydrogen, halogen or hydroxyl when b is a single bond;
R⁵ is absent when a is a double bond or R⁵ is hydrogen, halogen or hydroxyl when a is a single bond;
R⁶ is alkyl, alkenyl, alkynyl, cycloalkyl, heterocyclicyl, alkyl-O-alkyl, alkyl-CO₂-alkyl optionally substituted with one to five, hydroxyl, oxo, halogen, alkoxyl, aryl, heteroaryl, cyano, nitro or-NR'R" moieties;
R⁷ is alkyl optionally substituted with one to three hydroxyl, halogen, alkoxyl, aryl, heteroaryl, cyano, nitro or-NR'R" moieties; and,
R' and R" are each, independently, hydrogen, hydroxyl, halogen, alkyl or alkoxyl, and pharmaceutically acceptable salts thereof,
or wherein the vitamin D compound is 1α-hydroxyvitamin D3; 1α,24-dihydroxyvitamin D3, calcitriol, calcipotriol or 1,25-dihydroxy-16-ene-23-yne-cholecalciferol.

2. The pharmaceutical composition for use according to claim 1, wherein step (2) is performed prior to the commencement of the chemotherapy, wherein step (2) is preferably performed at least 3, 4, 5, 6, 7, 8, 9, 10, 11, 12 or 13 days prior to the commencement of the chemotherapy, and most preferably wherein step (2) is performed at least two weeks prior to the commencement of the chemotherapy.

3. The pharmaceutical composition for use according to any one of claims 1-2, wherein the pharmaceutical composition is
(i) administered in a total daily dose of 10-40 µg of the vitamin D compound, and wherein the pharmaceutical composition is preferably administered in a total daily dose of 20 µg or 40 µg of the vitamin D compound; or
(ii) administered twice daily for a total daily dose of 10-40 µg of the vitamin D compound, wherein each of 2 individual doses per day is 5-20 µg.

4. The pharmaceutical composition for use according to any one of claims 1-3, wherein 0.25 mL is administered to each of the four quadrants of the scalp.

5. The pharmaceutical composition for use according to any one of claims 1-4, wherein step (2) is performed twice daily, wherein the two daily administrations are preferably separated by 10-14 hours.

6. The pharmaceutical composition for use according to any one of claims 1-5, wherein the subject has a solid tumor, wherein the subject optionally has an advanced or a recurrent cancer, and/or wherein the subject optionally has cervical cancer, endometrial cancer, ovarian cancer, fallopian tube cancer, primary peritoneal carcinoma, soft tissue sarcoma, bone sarcoma, or breast cancer.

7. The pharmaceutical composition for use according to any one of claims 1-6, wherein the subject is selected based on one or more of the following additional criteria:
the subject is a human of at least 18 years of age;
the subject has no evidence of alopecia or mild alopecia;
the subject has hair follicles that are not apoptotic;
the subject has an Eastern Cooperative Oncology Group (ECOG) performance score of 0 or 1 within 14 days prior to beginning topical administration;
the subject has a baseline neutrophil count greater than 1500 cells/mm³ within 72 hours prior to beginning topical administration; and
the subject has a serum calcium level less than or equal to the upper limit of normal (ULN) within 72 hours prior to beginning topical administration,
or wherein the subject is selected based on one or more of the following additional criteria:
the subject does not have a history of hypercalcemia or vitamin D toxicity within 30 days of beginning the topical administration;
the subject does not have a history of hospitalization for treatment for angina, myocardial infarction, or congestive heart failure or psychiatric illness within 30 days of beginning topical administration;
the subject does not take a vitamin D supplement during topical administration, unless the subject has been taking the vitamin D supplement for 30 days or more prior to beginning topical administration and maintains the same dose throughout topical administration;
the subject is not receiving a thiazide or furosemide diuretic, with the exception of subjects who have stable doses and have been on therapy for over 6 months; the subject does not have hypercalcemia or kidney stones; and
the subject does not have alopecia grade 2 or greater as per National Cancer Institute Common Terminology Criteria for Adverse Events (NCU-CTCAE) v4.0 or significant hair loss or hair breakage.

8. The pharmaceutical composition for use according to any one of claims 1-7, wherein step (2) is performed for at least three months after commencement of the chemotherapy, or wherein step (2) is performed for the duration of the chemotherapy.

9. The pharmaceutical composition for use according to any one of claims 1-8, wherein the pharmaceutical composition is formulated such that the vitamin D compound is delivered to epidermis while substantially avoiding dermal delivery.

10. The pharmaceutical composition for use according to any one of claims 1-9, wherein the pharmaceutical composition is anhydrous, wherein the pharmaceutical composition optionally comprises a vehicle of 40% (w/w) propylene glycol and 60% (w/w) anhydrous ethanol, or wherein the pharmaceutical composition optionally comprises a vehicle of 30% (w/w) propylene glycol, 10% (w/w) ethoxydiglycol or transcutol, and 60% (w/w) anhydrous absolute ethanol (200 proof, U.S.).

11. The pharmaceutical composition for use according to claims 1-10, wherein the vitamin D compound is 1α-hydroxyvitamin D3; 1α,24-dihydroxyvitamin D3, calcipotriol, or calcitriol.

12. The pharmaceutical composition for use according to claims 1-11, wherein the vitamin D compound is calcitriol.

13. A kit for use in a method for preventing or mitigating chemotherapy induced alopecia, the kit comprising a pharmaceutical composition as specified in any one of claims 1-12 and adapted for topical administration; and instructions for carrying out a method for preventing or mitigating chemotherapy induced alopecia.

## Patentansprüche

1. Pharmazeutische Zusammensetzung umfassend eine therapeutisch wirksame Menge einer Vitamin-D-Verbindung zur Verwendung in einem Verfahren zur Verhinderung oder Verringerung von Chemotherapie-induzierter Alopezie in einem menschlichen Probanden, das Verfahren umfassend die folgenden Schritte:
(1) Auswählen eines menschlichen Probanden, der an Krebs erkrankt ist und der eine Chemotherapie erhalten soll oder bereits erhält; und
(2) topische Verabreichung der pharmazeutischen Zusammensetzung auf die Kopfhaut des Probanden unter Verwendung einer Dosierspray-Einheit,
wobei Schritt (2) vor und/oder gleichzeitig mit der Chemotherapie durchgeführt wird,
wobei die pharmazeutische Zusammensetzung die Vitamin-D-Verbindung in einer Konzentration von 5-20 µg/mL umfasst;
wobei die pharmazeutische Zusammensetzung in einer 1,0 mL Dosis verabreicht wird; und
wobei die Vitamin-D-Verbindung durch folgende Formel (1) dargestellt ist: 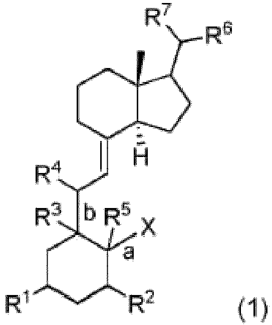
worin
a und b jeweils unabhängig voneinander eine Einfach- oder Doppelbindung sind;
X -CH₂ ist, wenn a eine Doppelbindung ist, oder X Wasserstoff oder ein Hydroxyl-substituiertes Alkyl ist, wenn a eine Einfachbindung ist;
R¹ ist Wasserstoff, Hydroxyl, Alkoxyl, Trialkylsilyl oder Alkyl, optional substituiert mit ein bis drei Halogen-, Hydroxyl-, Cyano- oder -NR'R"-Resten;
R² ist Wasserstoff, Hydroxyl, -O-Trialkylsilyl oder Alkyl, Alkoxyl oder Alkenyl, optional substituiert mit ein bis drei Halogen-, Hydroxyl-, Cyano- oder -NR'R"-Resten;
R³ fehlt, wenn b eine Doppelbindung ist, oder R³ ist Wasserstoff, Hydroxyl oder Alkyl, oder R³ und R¹ können zusammen mit den Kohlenstoffatomen, an die sie gebunden sind, verbunden sein, um einen 5-7-gliedrigen carbocyclischen Ring zu bilden, wenn b eine Einfachbindung ist;
R⁴ fehlt, wenn b eine Doppelbindung ist, oder ist Wasserstoff, Halogen oder Hydroxyl, wenn b eine Einfachbindung ist;
R⁵ fehlt, wenn a eine Doppelbindung ist, oder R⁵ ist Wasserstoff, Halogen oder Hydroxyl, wenn a eine Einfachbindung ist;
R⁶ ist Alkyl, Alkenyl, Alkinyl, Cycloalkyl, Heterocyclicyl, Alkyl-O-alkyl, Alkyl-CO₂-Alkyl, optional substituiert mit einem bis fünf Hydroxyl-, Oxo-, Halogen-, Alkoxyl-, Aryl-, Heteroaryl-, Cyano-, Nitro- oder -NR'R"-Resten;
R⁷ ist Alkyl, optional substituiert mit ein bis drei Hydroxyl-, Halogen-, Alkoxyl-, Aryl-, Heteroaryl-, Cyano-, Nitro- oder -NR'R"-Resten; und,
R' und R" sind jeweils unabhängig voneinander Wasserstoff, Hydroxyl, Halogen, Alkyl oder Alkoxyl, und pharmazeutisch akzeptable Salze davon,
oder wobei die Vitamin-D-Verbindung 1α-Hydroxyvitamin D3; 1α,24-Dihydroxyvitamin D3, Calcitriol, Calcipotriol oder 1,25-Dihydroxy-16-en-23-yne-cholecalciferol ist.

2. Pharmazeutische Zusammensetzung zur Verwendung gemäß Anspruch 1, wobei Schritt (2) vor dem Beginn der Chemotherapie durchgeführt wird, wobei Schritt (2) vorzugsweise mindestens 3, 4, 5, 6, 7, 8, 9, 10, 11, 12 oder 13 Tage vor dem Beginn der Chemotherapie durchgeführt wird, und am meisten bevorzugt, wobei Schritt (2) mindestens zwei Wochen vor dem Beginn der Chemotherapie durchgeführt wird.

3. Pharmazeutische Zusammensetzung zur Verwendung gemäß mindestens einem der Ansprüche 1-2, wobei die pharmazeutische Zusammensetzung
(i) in einer täglichen Gesamtdosis von 10-40 µg der Vitamin-D-Verbindung verabreicht wird, und wobei die pharmazeutische Zusammensetzung vorzugsweise in einer täglichen Gesamtdosis von 20 µg oder 40 µg der Vitamin-D-Verbindung verabreicht wird; oder
(ii) zweimal täglich in einer Gesamttagesdosis von 10-40 µg der Vitamin-D-Verbindung verabreicht wird, wobei jede der 2 Einzeldosen pro Tag 5-20 µg beträgt.

4. Pharmazeutische Zusammensetzung zur Verwendung gemäß mindestens einem der Ansprüche 1-3, wobei 0,25 mL auf jeden der vier Quadranten der Kopfhaut verabreicht werden.

5. Pharmazeutische Zusammensetzung zur Verwendung gemäß mindestens einem der Ansprüche 1-4, wobei Schritt (2) zweimal täglich durchgeführt wird, wobei die zwei täglichen Verabreichungen vorzugsweise durch 10-14 Stunden getrennt sind.

6. Pharmazeutische Zusammensetzung zur Verwendung gemäß mindestens einem der Ansprüche 1-5, wobei der Proband einen soliden Tumor hat, wobei der Proband optional einen fortgeschrittenen oder wiederkehrenden Krebs hat und/oder wobei der Proband optional Gebärmutterhalskrebs, Endometriumkrebs, Eierstockkrebs, Eileiterkrebs, primäres Peritonealkarzinom, Weichteilsarkom, Knochensarkom oder Brustkrebs hat.

7. Pharmazeutische Zusammensetzung zur Verwendung gemäß mindestens einem der Ansprüche 1-6, wobei der Proband auf der Grundlage eines oder mehrerer der folgenden zusätzlichen Kriterien ausgewählt wird:
der Proband ist ein Mensch im Alter von mindestens 18 Jahren;
der Proband hat keine Anzeichen von Alopezie oder leichter Alopezie;
der Proband hat Haarfollikel, die nicht apoptotisch sind;
der Proband hat einen Eastern Cooperative Oncology Group (ECOG) Performance Score von 0 oder 1 innerhalb von 14 Tagen vor Beginn der topischen Verabreichung;
der Proband weist innerhalb von 72 Stunden vor Beginn der topischen Verabreichung eine
Ausgangsneutrophilenzahl von mehr als 1500 Zellen/mm³ auf; und
der Proband weist innerhalb von 72 Stunden vor Beginn der topischen Verabreichung einen Serumkalziumspiegel auf, der kleiner oder gleich der oberen Grenze des Normalwerts (ULN) ist,
oder wobei der Proband auf der Grundlage eines oder mehrerer der folgenden zusätzlichen Kriterien ausgewählt wird:
der Proband hat keine Vorgeschichte von Hyperkalzämie oder Vitamin-D-Toxizität innerhalb von 30 Tagen vor Beginn der topischen Verabreichen;
der Proband hat keine Vorgeschichte von Krankenhausaufenthalten zur Behandlung von Angina, Myokardinfarkt, oder kongestiver Herzinsuffizienz oder einer psychiatrischen Erkrankung innerhalb vor 30 Tagen vom Beginn der topischen Verabreichung;
der Proband nimmt während der topischen Verabreichung kein Vitamin-D-Präparat ein, es sei denn, der Proband hat das Vitamin-D-Präparat 30 Tage oder länger vor Beginn der topischen Verabreichung eingenommen und behält während der topischen Verabreichung die gleiche Dosis bei;
der Proband erhält kein Thiazid- oder Furosemid-Diuretikum, mit Ausnahme von Probanden, die stabile Dosen einnehmen und die Therapie seit mehr als 6 Monaten durchführen;
der Proband leidet nicht an Hyperkalzämie oder Nierensteinen; und
der Proband hat keine Alopezie Grad 2 oder höher gemäß National Cancer Institute Common Terminology Criteria for Adverse Events (NCU-CTCAE) v4.0 oder signifikanten Haarausfall oder Haarbruch.

8. Pharmazeutische Zusammensetzung zur Verwendung gemäß mindestens einem der Ansprüche 1-7, wobei Schritt (2) für mindestens drei Monate nach Beginn der Chemotherapie durchgeführt wird, oder wobei Schritt (2) für die Dauer der Chemotherapie durchgeführt wird.

9. Pharmazeutische Zusammensetzung zur Verwendung gemäß mindestens einem der Ansprüche 1-8, wobei die pharmazeutische Zusammensetzung so formuliert ist, dass die Vitamin-D-Verbindung an die Epidermis abgegeben wird, wobei eine dermale Abgabe im Wesentlichen vermieden wird.

10. Pharmazeutische Zusammensetzung zur Verwendung gemäß mindestens einem der Ansprüche 1-9, wobei die pharmazeutische Zusammensetzung wasserfrei ist, wobei die pharmazeutische Zusammensetzung optional umfasst ein Vehikel aus 40% (w/w) Propylenglykol und 60 % (w/w) wasserfreiem Ethanol, oder wobei die pharmazeutische Zusammensetzung optional umfasst ein Vehikel aus 30% (w/w) Propylenglykol, 10 % (w/w) Ethoxydiglykol oder Transcutol und 60 % (w/w) wasserfreiem absolutem Ethanol (200 proof, U.S.).

11. Pharmazeutische Zusammensetzung zur Verwendung gemäß mindestens einem der Ansprüche 1-10, wobei die Vitamin-D-Verbindung 1α-Hydroxyvitamin D3, 1α,24-Dihydroxyvitamin D3, Calcipotriol oder Calcitriol ist.

12. Pharmazeutische Zusammensetzung zur Verwendung gemäß mindestens einem der Ansprüche 1-11, wobei die Vitamin-D-Verbindung Calcitriol ist.

13. Kit zur Verwendung in einem Verfahren zur Verhinderung oder Verringerung von Chemotherapie-induzierter Alopezie, wobei das Kit umfasst eine pharmazeutische Zusammensetzung gemäß mindestens einem der Ansprüche 1-12, die zur topischen Verabreichung angepasst ist; und Anweisungen zur Durchführung eines Verfahrens zur Verhinderung oder Verringerung von Chemotherapie-induzierter Alopezie.

## Revendications

1. Composition pharmaceutique comprenant une quantité thérapeutiquement efficace d'un composé à base de vitamine D pour une utilisation dans un procédé de prévention ou d'atténuation d'une alopécie induite par la chimiothérapie chez un sujet humain, le procédé comprenant les étapes de :
(1) sélection d'un sujet humain présentant un cancer et qui est programmé pour recevoir, ou qui reçoit, une chimiothérapie ; et
(2) administration topique de la composition pharmaceutique sur le cuir chevelu du sujet en utilisant une unité de pulvérisation dosée,
dans laquelle l'étape (2) est mise en oeuvre avant et/ou simultanément avec la chimiothérapie,
dans laquelle la composition pharmaceutique comprend le composé à base de vitamine D à une concentration de 5 à 20 µg/mL ;
dans laquelle la composition pharmaceutique est administrée en une dose de 1,0 mL ; et
dans laquelle le composé à base de vitamine D est représenté par la Formule (1) : 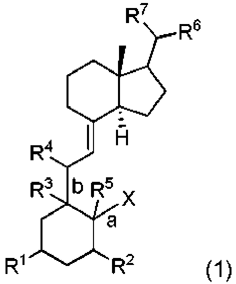
dans laquelle
a et b sont chacun indépendamment une liaison simple ou double ;
X est -CH₂ lorsque a est une liaison double, ou X est un hydrogène ou un alkyle substitué par un hydroxyle lorsque a est une liaison simple ;
R¹ est un hydrogène, un hydroxyle, un alcoxyle, un tri-alkyl silyle ou un alkyle, facultativement substitué par un à trois groupements halogène, hydroxyle, cyano ou - NR'R" ;
R² est un hydrogène, un hydroxyle, un -O-trialkyl silyle, ou un alkyle, un alcoxyle ou un alcényle, facultativement substitué par un à trois groupements halogène, hydroxyle, cyano ou -NR'R" ;
R³ est absent lorsque b est une liaison double ou R³ est un hydrogène, un hydroxyle ou un alkyle, ou R³ et R¹ conjointement avec les atomes de carbone auxquels ils sont fixés peuvent être liés pour former un noyau carbocyclique de 5 à 7 chaînons lorsque b est une liaison simple ;
R⁴ est absent lorsque b est une liaison double ou un hydrogène, un halogène ou un hydroxyle lorsque b est une liaison simple ;
R⁵ est absent lorsque a est une liaison double ou R⁵ est un hydrogène, un halogène ou un hydroxyle lorsque a est une liaison simple ;
R⁶ est un alkyle, un alcényle, un alcynyle, un cycloalkyle, un hétérocyclicyle, un alkyl-O-alkyle, un alkyl-CO₂-alkyle facultativement substitué par un à cinq groupements hydroxyle, oxo, halogène, alcoxyle, aryle, hétéroaryle, cyano, nitro ou -NR'R" ;
R⁷ est un alkyle facultativement substitué par un à trois groupements hydroxyle, halogène, alcoxyle, aryle, hétéroaryle, cyano, nitro ou -NR'R" ; et,
R' et R" sont chacun, indépendamment, un hydrogène, un hydroxyle, un halogène, un alkyle ou un alcoxyle, et des sels pharmaceutiquement acceptables de ceux-ci,
ou dans laquelle le composé à base de vitamine D est de la 1-hydroxyvitamine D3 ;
de la 1,24-dihydroxyvitamine D3, du calcitriol, du calcipotriol ou du 1,25-dihydroxy-16-én-23-yne-cholécalciférol. < <

2. Composition pharmaceutique pour utilisation selon la revendication 1, dans laquelle l'étape (2) est mise en oeuvre avant le commencement de la chimiothérapie, dans laquelle l'étape (2) est de préférence mise en oeuvre au moins 3, 4, 5, 6, 7, 8, 9, 10, 11, 12 ou 13 jours avant le commencement de la chimiothérapie, et le plus préférentiellement dans laquelle l'étape (2) est mise en oeuvre au moins deux semaines avant le commencement de la chimiothérapie.

3. Composition pharmaceutique pour utilisation selon l'une quelconque des revendications 1 à 2, dans laquelle la composition pharmaceutique est
(i) administrée en une dose quotidienne totale de 10 à 40 µg du composé à base de vitamine D, et dans laquelle la composition pharmaceutique est de préférence administrée en une dose quotidienne totale de 20 µg ou 40 µg du composé à base de vitamine D ; ou
(ii) administrée deux fois par jour pour une dose quotidienne totale de 10 à 40 µg du composé à base de vitamine D, dans laquelle chacune de 2 doses individuelles par jour est de 5 à 20 µg.

4. Composition pharmaceutique pour utilisation selon l'une quelconque des revendications 1 à 3, dans laquelle 0,25 mL est administré à chacun des quatre quadrants du cuir chevelu.

5. Composition pharmaceutique pour utilisation selon l'une quelconque des revendications 1 à 4, dans laquelle l'étape (2) est mise en oeuvre deux fois par jour, dans laquelle les deux administrations quotidiennes sont de préférence séparées de 10 à 14 heures.

6. Composition pharmaceutique pour utilisation selon l'une quelconque des revendications 1 à 5, dans laquelle le sujet présente une tumeur solide, dans laquelle le sujet présente facultativement un cancer avancé ou récurrent, et/ou dans laquelle le sujet présente facultativement un cancer du col de l'utérus, un cancer de l'endomètre, un cancer de l'ovaire, un cancer des trompes de Fallope, un carcinome péritonéal primaire, un sarcome des tissus mous, un sarcome osseux ou un cancer du sein.

7. Composition pharmaceutique pour utilisation selon l'une quelconque des revendications 1 à 6, dans laquelle le sujet est sélectionné sur la base d'un ou plusieurs des critères additionnels suivants :
le sujet est un être humain âgé d'au moins 18 ans ;
le sujet ne présente pas de signe d'alopécie ou d'alopécie légère ;
le sujet présente des follicules capillaires qui ne sont pas apoptotiques ;
le sujet présente un score de performance Eastern Cooperative Oncology Group (ECOG) de 0 ou 1 dans les 14 jours avant le début de l'administration topique ;
le sujet présente un nombre de neutrophiles de base supérieur à 1500 cellules/mm³ dans les 72 heures avant le début de l'administration topique ; et
le sujet présente un taux de calcium sérique inférieur ou égal à la limite supérieure de la normale (ULN) dans les 72 heures avant le début de l'administration topique,
ou dans laquelle le sujet est sélectionné sur la base d'un ou plusieurs des critères additionnels suivants :
le sujet ne présente pas d'antécédents d'hypercalcémie ou de toxicité à la vitamine D dans les 30 jours du début de l'administration topique ;
le sujet ne présente pas d'antécédents d'hospitalisation pour le traitement de l'angine, de l'infarctus du myocarde ou de l'insuffisance cardiaque congestive ou d'une maladie psychiatrique dans les 30 jours du début de l'administration topique ;
le sujet ne prend pas de complément de vitamine D pendant l'administration topique, sauf si le sujet a pris le complément de vitamine D pendant 30 jours ou plus avant le début de l'administration topique et maintient la même dose tout au long de l'administration topique ; le sujet ne reçoit pas de diurétique de thiazide ou de furosémide, à l'exception des sujets qui présentent des doses stables et ont été en thérapie pendant plus de 6 mois ;
le sujet ne présente pas d'hypercalcémie ou de calculs rénaux ; et
le sujet ne présente pas d'alopécie de grade 2 ou supérieur selon l'échelle du National Cancer Institute Common Terminology Criteria for Adverse Events (NCU-CTCAE) v4.0 ou une perte de cheveux ou une cassure de cheveux significative.

8. Composition pharmaceutique pour utilisation selon l'une quelconque des revendications 1 à 7, dans laquelle l'étape (2) est mise en oeuvre pendant au moins trois mois après le commencement de la chimiothérapie, ou dans laquelle l'étape (2) est mise en oeuvre pendant la durée de la chimiothérapie.

9. Composition pharmaceutique pour utilisation selon l'une quelconque des revendications 1 à 8, dans laquelle la composition pharmaceutique est formulée de telle manière que le composé à base de vitamine D est apporté à l'épiderme tout en évitant sensiblement l'apport dermique.

10. Composition pharmaceutique pour utilisation selon l'une quelconque des revendications 1 à 9, dans laquelle la composition pharmaceutique est anhydre, dans laquelle la composition pharmaceutique comprend facultativement un véhicule de 40 % (p/p) de propylène glycol et de 60 % (p/p) d'éthanol anhydre, ou dans laquelle la composition pharmaceutique comprend facultativement un véhicule de 30 % (p/p) de propylène glycol, de 10 % (p/p) d'éthoxydiglycol ou de transcutol, et de 60 % (p/p) d'éthanol absolu anhydre (200 proof, États-Unis).

11. Composition pharmaceutique pour utilisation selon l'une quelconque des revendications 1 à 10, dans laquelle le composé à base de vitamine D est de la 1-hydroxyvitamine D3 ; de la 1,24-dihydroxyvitamine D3, du calcipotriol ou du calcitriol.〈〈

12. Composition pharmaceutique pour utilisation selon les revendications 1 à 11, dans laquelle le composé à base de vitamine D est du calcitriol.

13. Kit pour utilisation dans un procédé de prévention ou d'atténuation d'une alopécie induite par la chimiothérapie, le kit comprenant une composition pharmaceutique selon l'une quelconque des revendications 1 à 12 et adaptée pour une administration topique ; et des instructions pour mettre en oeuvre un procédé de prévention ou d'atténuation d'une alopécie induite par la chimiothérapie.
